(19) Europäisches Patentamt
European Patent Office
Office européen des brevets

(11) **EP 4 643 888 A1**

(12) **EUROPEAN PATENT APPLICATION**
published in accordance with Art. 153(4) EPC


(43) Date of publication:
**05.11.2025 Bulletin 2025/45**

(21) Application number: **23910764.2**

(22) Date of filing: **27.12.2023**

(51) International Patent Classification (IPC):
***A61K 47/68*** *(2017.01)* ***C07K 16/46*** *(2006.01)*
***A61K 31/4745*** *(2006.01)* ***A61P 35/00*** *(2006.01)*

(52) Cooperative Patent Classification (CPC):
**A61K 31/4745; A61K 47/68; A61P 35/00; C07K 16/46**

(86) International application number:
**PCT/CN2023/142481**

(87) International publication number:
**WO 2024/140846 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 CN 202211691970**
**11.12.2023 CN 202311696497**



(71) Applicant: **Duality Biologics (Shanghai) Co., Ltd.**
**Shanghai 201204 (CN)**

(72) Inventors:
- **LI, Chenggang**
**Suzhou, Jiangsu 215000 (CN)**
- **YANG, Junjie**
**Suzhou, Jiangsu 215000 (CN)**
- **ZHANG, Yu**
**Suzhou, Jiangsu 215000 (CN)**
- **HUA, Haiqing**
**Suzhou, Jiangsu 215000 (CN)**
- **ZHU, Zhongyuan**
**Suzhou, Jiangsu 215000 (CN)**

(74) Representative: **Greaves Brewster LLP**
**Copa House**
**Station Road**
**Cheddar, Somerset BS27 3AH (GB)**


Remarks:
The complete document including Reference Table(s) and the Sequence Listing(s) can be downloaded from the EPO website

(54) **ANTI-B7H3 AND PD-L1 BISPECIFIC ANTIBODY-DRUG CONJUGATE, PREPARATION METHOD THEREFOR, AND USE THEREOF**


(57) Disclosed are a bispecific antibody-drug conjugate, a preparation method therefor, and use thereof. The structure of the bispecific antibody-drug conjugate comprises the following fragments: an anti-B7H3 and PD-L1 bispecific antibody or an antigen-binding fragment thereof, a linker unit L, and a cytotoxic drug. The bispecific antibody-drug conjugate of the present invention has a good endocytosis effect, proliferation inhibiting activity, and tumor growth inhibiting activity.


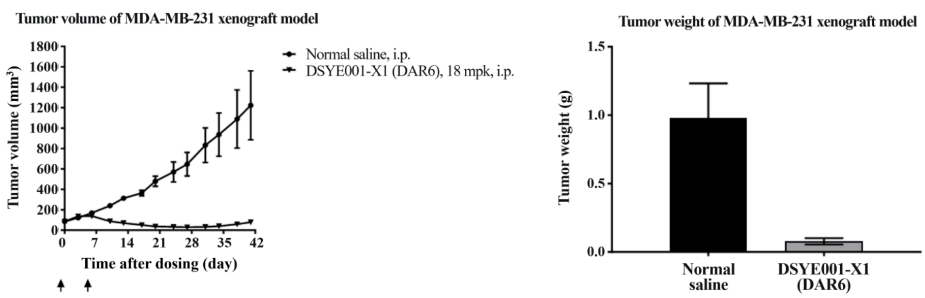


FIG. 20




EP 4 643 888 A1

## Description

The present application claims priority to Chinese patent application 2022116919701 filed on December 27, 2022 and Chinese patent application 2023116964970 filed on December 11, 2023. The contents of the Chinese patent applications are incorporated herein by reference in their entirety.

TECHNICAL FIELD

The present disclosure provides an anti-B7H3 and PD-L1 bispecific antibody-drug conjugate, a preparation method therefor, a use thereof, and a pharmaceutical composition comprising the same.

BACKGROUND

The B7-CD28 family serves as a co-stimulatory signal for T lymphocyte activation and plays a crucial role in immune responses mediated by T lymphocytes. Studies have shown that different types of B7 molecules can positively or negatively regulate immune cell responses. B7H3 (also known as CD276) is a member of the B7 family and is primarily expressed on the surface of tumor cells. Chapoval AI et al. first discovered its co-stimulatory effect on $CD4^+$ and $CD8^+$ T cells. B7H3 signaling induces cellular immunity and selectively enhances the production of interferon-γ (IFN-γ) under T cell receptor signaling. However, with the continuous in-depth research on B7H3, its inhibitory function has gradually been discovered. For example, B7H3 can inhibit the proliferation of $CD4^+$ T and $CD8^+$ T cells. Furthermore, studies have shown that abnormal expression of B7H3 is associated with the occurrence, progression, and metastasis of various cancers, with substantial evidence indicating that its high expression correlates with poor prognosis in various malignant tumors.

B7H3 is highly expressed in all tested cancer types with limited heterogeneity and is rarely expressed in normal tissues. This indicates that B7H3 can be considered a tumor antigen (TA), providing potential for targeted therapy against tumor cells with high B7H3 expression. Currently, B7H3-targeted therapeutic strategies primarily include blocking monoclonal antibodies, radioimmunotherapy, antibody-drug conjugates (ADCs), cytotoxicitymediated monoclonal antibodies, and bispecific antibodies (BsAbs).

Inhibitors targeting the immune checkpoint PD1/PD-L1 are undoubtedly the focus of tumor immunotherapy. PD-L1 is expressed on the surface of tumor cells, and cytotoxic PD-L1 inhibitory antibodies theoretically exhibit superior anti-tumor effects. However, it has been reported that among the marketed PD-L1 monoclonal antibodies, only Avelumab can mediate ADCC effects against tumors and exhibits a comparable safety profile to other PD-L1 antibodies. A key reason is that ADCC activity primarily depends on the abundance of antigen expression, and PD-L1 cannot be regarded as a typical tumor antigen, exhibiting significant heterogeneity in tumor cells. PD1/PD-L1 pathway blocking antibodies are often used in combination with cytotoxic antibodies to enhance the efficacy of immunotherapy.

An antibody-drug conjugate (ADC) consists of three components: an antibody or antigen-binding fragment thereof (targeting), a linker, and a small-molecule drug. The antibody or antigen-binding fragment thereof is conjugated to a biologically active, e.g., cytotoxic, small-molecule drug, such as a cytotoxin, via a cleavable or non-cleavable linker, which fully leverages the specificity of the antibody or antigen-binding fragment for targeting cells of interest (target cells) or binding to highly expressed antigens, as well as the high efficacy of the small-molecule drug, thereby reducing or avoiding toxic side effects on nontarget cells. This indicates that antibody-drug conjugates for tumors can precisely target tumor cells and reduce their impact on non-tumor cells compared to conventional tumor chemotherapeutic drugs.

Among the antibody-drug conjugates targeting B7H3, MGC018 from MacroGenics and DS7300 from Daiichi Sankyo are advancing most rapidly, with both drugs currently in Phase II clinical trials. MGC018 conjugates the DNA alkylating agent duocarmycin to a humanized B7H3 antibody via a cleavable linker. Early clinical trials have demonstrated that MGC018 has exhibited preliminary anti-tumor activity and manageable toxicity in patients with advanced metastatic castration-resistant prostate cancer (mCRPC) and melanoma. DS7300 conjugates an irinotecan derivative (deruxtecan) to the B7H3 antibody via a cleavable linker. Phase I clinical trials have demonstrated anti-tumor activity across various tumors, including mCRPC, small cell lung cancer, squamous cell lung carcinoma, esophageal squamous cell carcinoma, and endometrial cancer, with a favorable safety profile.

Currently, all bispecific ADCs are in the early clinical stage, with no approved drugs available. There remains a need in the art for bispecific antibody-drug conjugates with synergistic mechanisms of action, superior tumor selectivity, and excellent stability.

SUMMARY

The technical problem to be solved by the present disclosure is to overcome the deficiency of few bispecific antibody-drug conjugates in the prior art, and to provide a bispecific antibody-drug conjugate, a preparation method therefor, and a use thereof. The bispecific antibody-drug conjugate of the present disclosure has excellent endocytosis

effect, proliferation inhibiting activity, and tumor growth inhibiting activity.

The present disclosure primarily addresses the above technical problem through the following technical means.

In one aspect, the present disclosure provides a bispecific antibody-drug conjugate having a structure comprising the following fragments: an anti-B7H3 and PD-L1 bispecific antibody or an antigen-binding fragment thereof, a linker unit L, and a cytotoxic drug, wherein the bispecific antibody or the antigen-binding fragment thereof comprises:

a monoclonal antibody unit targeting PD-L1 and comprising two heavy chains and two light chains, and a nanobody unit targeting B7H3 and comprising two identical nanobodies,
wherein the N-terminus of each of the two nanobodies is connected to the C-terminus of each of the Fc fragments of the two heavy chains of the monoclonal antibody unit via a linker peptide.

In some embodiments, in the bispecific antibody-drug conjugate according to the present disclosure,
the light chain variable region of the monoclonal antibody unit comprises a CDR1 with an amino acid sequence of SEQ ID NO.: 1, a CDR2 with an amino acid sequence of SEQ ID NO.: 2, and a CDR3 with an amino acid sequence of SEQ ID NO.: 3; the heavy chain variable region of the monoclonal antibody unit comprises a CDR1 with an amino acid sequence of SEQ ID NO.: 5, a CDR2 with an amino acid sequence of SEQ ID NO.: 6, and a CDR3 with an amino acid sequence of SEQ ID NO.: 7; and the nanobody comprises a CDR1 with an amino acid sequence of SEQ ID NO.: 12, a CDR2 with an amino acid sequence of SEQ ID NO.: 13, and a CDR3 with an amino acid sequence of SEQ ID NO.: 14.

In some embodiments, in the bispecific antibody-drug conjugate according to the present disclosure,
the light chain variable region of the monoclonal antibody unit comprises an amino acid sequence as shown in SEQ ID NO.: 4; the heavy chain variable region of the monoclonal antibody unit comprises an amino acid sequence as shown in SEQ ID NO.: 8; and the nanobody comprises an amino acid sequence as shown in SEQ ID NO.: 15.

In some embodiments, in the bispecific antibody-drug conjugate according to the present disclosure,
the monoclonal antibody comprises an immunoglobulin constant region, wherein the immunoglobulin constant region is a human IgG constant region, such as a human IgG1 constant region.

In some embodiments, in the bispecific antibody-drug conjugate according to the present disclosure,

the light chain of the monoclonal antibody unit comprises an amino acid sequence as shown in SEQ ID NO.: 9; the heavy chain of the monoclonal antibody unit comprises an amino acid sequence as shown in SEQ ID NO.: 10; and the nanobody comprises an amino acid sequence as shown in SEQ ID NO.: 15; or
the full-length amino acid sequence of the light chain of the monoclonal antibody unit is as shown in SEQ ID NO.: 9; the full-length amino acid sequence of the heavy chain of the monoclonal antibody unit is as shown in SEQ ID NO.: 10; and the amino acid sequence of the nanobody is as shown in SEQ ID NO.: 15.

In some embodiments, in the bispecific antibody-drug conjugate according to the present disclosure, the linker peptide is a polypeptide comprising glycine and serine with certain elasticity and protease resistance; preferably, the amino acid sequence of the linker peptide is SEQ ID NO.: 11.

In some embodiments, in the bispecific antibody-drug conjugate according to the present disclosure, the heavy chain amino acid sequence of the bispecific antibody is as shown in SEQ ID NO.: 16, and the light chain amino acid sequence is as shown in SEQ ID NO.: 9.

In some embodiments, the amino acid sequence of the CDR may have at least 80%, 90%, 91%, 92%, 93%, 94%, 95%, 96%, 97%, 98%, or 99% identity with each of the aforementioned sequences. In some embodiments, the amino acid sequence of the variable region may have at least 90%, 91%, 92%, 93%, 94%, 95%, 96%, 97%, 98%, or 99% identity with each of the aforementioned sequences.

The Fc region of the bispecific antibody of the present disclosure may be a human Fc region. The Fc region of the bispecific antibody of the present disclosure may be of any isotype, including, but not limited to, IgG1, IgG2, IgG3, or IgG4. In some embodiments, the Fc regions are all of IgG1 isotype. In some embodiments, the Fc regions are all of IgG4 isotype.

In some embodiments, one or more amino acid modifications may be introduced into the Fc region of an antibody provided in the present disclosure to generate an Fc region variant. The Fc region variant may comprise a human Fc region sequence (e.g., a human IgG1, IgG2, IgG3, or IgG4 Fc region) containing an amino acid modification (e.g., a substitution) at one or more amino acid positions.

In some embodiments, the antibodies provided in the present disclosure may be further modified to contain other non-protein moieties that are known and readily available in the art. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethylcellulose, dextran, polyvinyl alcohol, polyvinylpyrrolidone, poly-1,3-dioxane, poly-1,3,6-trioxane, ethylene/maleic anhydride copolymers, polyamino acids (homopolymers or random copolymers), and dextran or poly(N-vinylpyrrolidone), polyethylene glycol, propylene glycol homopolymers, polypropylene oxide/ethylene oxide copolymers, polyoxyethylated polyols (e.g., glycerol), polyvinyl alcohol, and mixtures thereof.

In some embodiments, in the bispecific antibody-drug conjugate according to the present disclosure, the bispecific antibody is DSYE001.

In some embodiments, in the bispecific antibody-drug conjugate, composition, use, or method according to the present disclosure, the bispecific antibody comprises a monoclonal antibody unit and a nanobody unit, wherein the CDR sequence of the monoclonal antibody unit comprises LCDR1, LCDR2, and LCDR3 with amino acid sequences as shown in SEQ ID NO.: 1, SEQ ID NO.: 2, and SEQ ID NO.: 3, respectively, and HCDR1, HCDR2, and HCDR3 with amino acid sequences as shown in SEQ ID NO.: 12, SEQ ID NO.: 13, and SEQ ID NO.: 14, respectively; and the CDR sequence of the nanobody unit comprises HCDR1, HCDR2, and HCDR3 with amino acid sequences as shown in SEQ ID NO.: 12, SEQ ID NO.: 13, and SEQ ID NO.: 14, respectively.

In some embodiments, in the bispecific antibody-drug conjugate, composition, use, or method according to the present disclosure, the light chain variable region of the monoclonal antibody unit comprises an amino acid sequence as shown in SEQ ID NO.: 4; the heavy chain variable region of the monoclonal antibody unit comprises an amino acid sequence as shown in SEQ ID NO.: 8; and the nanobody comprises an amino acid sequence as shown in SEQ ID NO.: 15.

In some embodiments, in the bispecific antibody-drug conjugate, composition, use, or method according to the present disclosure, the full-length amino acid sequence of the light chain of the monoclonal antibody unit is as shown in SEQ ID NO.: 9; the full-length amino acid sequence of the heavy chain of the monoclonal antibody unit is as shown in SEQ ID NO.: 10; and the amino acid sequence of the nanobody is as shown in SEQ ID NO.: 15.

In some embodiments, in the bispecific antibody-drug conjugate, composition, or use according to the present disclosure, the heavy chain amino acid sequence of the bispecific antibody is as shown in SEQ ID NO.: 16, and the light chain amino acid sequence is as shown in SEQ ID NO.: 9.

In some embodiments, in the bispecific antibody-drug conjugate according to the present disclosure, the cytotoxic drug is a structure represented by formula (A-1), a tautomer, enantiomer, diastereomer, or mixture of isomers thereof, or a pharmaceutically acceptable salt or solvate thereof,

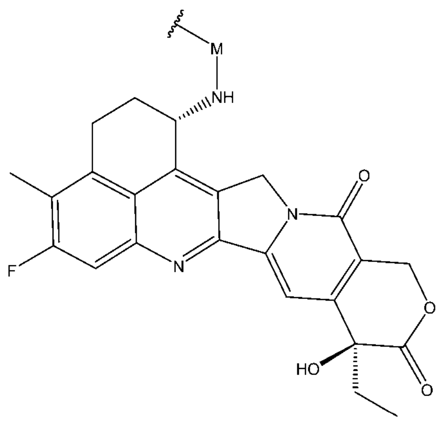

(A-1)

wherein

- M is $-L^2-L^1-C(O)-$;
- $L^2$ is -O- or -S-, and $L^2$ is connected to the linker unit L;
- $L^1$ is $-(C(R^{1a})(R^{1b}))_m-CH_2-$, $C_3$-$C_6$ saturated cycloalkyl, or 3- to 6-membered saturated heterocyclyl, wherein the $C_3$-$C_6$ saturated cycloalkyl and 3- to 6-membered saturated heterocyclyl are each independently and optionally substituted by one or more $R^{2a}$;
- m is 1, 2, 3, or 4; heteroatoms in the 3- to 6-membered saturated heterocyclyl are each independently N, O, or S, and the number of heteroatoms is 1, 2, or 3;
- $R^{1a}$ and $R^{1b}$ are each independently hydrogen, halogen, hydroxyl, amino, or $C_1$-$C_6$ alkyl, wherein the $C_1$-$C_6$ alkyl is optionally substituted by one or more halogens;
- $R^{2a}$ is halogen, hydroxyl, amino, or $C_1$-$C_6$ alkyl, wherein the $C_1$-$C_6$ alkyl is optionally substituted by one or more halogens.

In some embodiments, in the bispecific antibody-drug conjugate according to the present disclosure, $L^2$ is -O-.

In some embodiments, in the bispecific antibody-drug conjugate according to the present disclosure, $L^1$ is $-(C(R^{1a})(R^{1b}))_m-CH_2-$; $R^{1a}$ is hydrogen, halogen, or $C_1$-$C_6$ alkyl; $R^{1b}$ is hydrogen, halogen, or $C_1$-$C_6$ alkyl.

In some embodiments, in the bispecific antibody-drug conjugate according to the present disclosure, $L^1$ is

-(C($R^{1a}$)($R^{1b}$))$_m$-CH$_2$-; $R^{1a}$ is $C_1$-$C_6$ alkyl, preferably $C_1$-$C_3$ alkyl; $R^{1b}$ is hydrogen or $C_1$-$C_6$ alkyl, preferably hydrogen or $C_1$-$C_3$ alkyl.

In some embodiments, in the bispecific antibody-drug conjugate according to the present disclosure, $L^1$ is -(C($R^{1a}$)($R^{1b}$))$_m$-CH$_2$-; $R^{1a}$ is -CH$_3$; $R^{1b}$ is hydrogen or -CH$_3$.

In some embodiments, in the bispecific antibody-drug conjugate according to the present disclosure, m is 1 or 2.

In some embodiments, in the bispecific antibody-drug conjugate according to the present disclosure, $L^1$ is

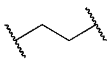, 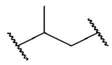, 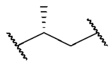, 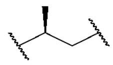, or 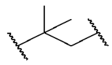.

In some embodiments, in the bispecific antibody-drug conjugate according to the present disclosure, $L^1$ is $C_3$-$C_6$ saturated cycloalkyl or 3- to 6-membered saturated heterocyclyl, wherein the $C_3$-$C_6$ saturated cycloalkyl and 3- to 6-membered saturated heterocyclyl are each independently and optionally substituted by one or more $R^{2a}$, and each $R^{2a}$ is independently halogen or $C_1$-$C_6$ alkyl.

In some embodiments, in the bispecific antibody-drug conjugate according to the present disclosure, $L^1$ is $C_3$-$C_6$ saturated cycloalkyl, wherein the $C_3$-$C_6$ saturated cycloalkyl is optionally substituted by one or more $R^{2a}$, and each $R^{2a}$ is independently halogen or $C_1$-$C_6$ alkyl.

In some embodiments, in the bispecific antibody-drug conjugate according to the present disclosure, $L^1$ is cyclopropyl, cyclobutyl, cyclopentyl, or cyclohexyl, wherein the cyclopropyl, cyclobutyl, cyclopentyl, and cyclohexyl are optionally substituted by one or more $R^{2a}$, and each $R^{2a}$ is independently halogen or $C_1$-$C_6$ alkyl.

In some embodiments, in the bispecific antibody-drug conjugate according to the present disclosure, $L^1$ is

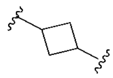, 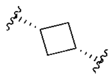, 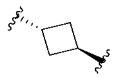, 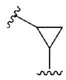, 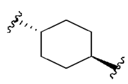, 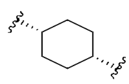,

or

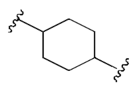.

In some embodiments, in the bispecific antibody-drug conjugate according to the present disclosure, M is

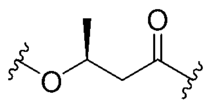 or 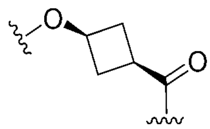.

In some embodiments, in the bispecific antibody-drug conjugate according to the present disclosure, in the structure represented by formula (A-1),

M is -$L^2$-$L^1$-C(O)-;
$L^2$ is -O-;
$L^1$ is -(C($R^{1a}$)($R^{1b}$))$_m$-CH$_2$- or $C_3$-$C_6$ saturated cycloalkylene, wherein the $C_3$-$C_6$ saturated cycloalkylene is optionally substituted by one or more $R^{2a}$;
m is 1 or 2;
$R^{1a}$ and $R^{1b}$ are each independently selected from the group consisting of hydrogen, halogen, and $C_1$-$C_6$ alkyl, wherein the $C_1$-$C_6$ alkyl is optionally substituted by one or more halogens;
$R^{2a}$ is selected from the group consisting of halogen and $C_1$-$C_6$ alkyl, wherein the $C_1$-$C_6$ alkyl is optionally substituted by one or more halogens.

In some embodiments, in the bispecific antibody-drug conjugate according to the present disclosure, the cytotoxic drug is selected from the group consisting of any one of the following structures:

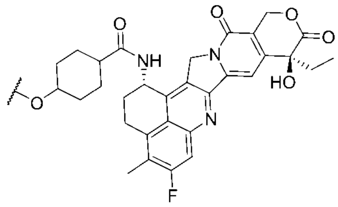,
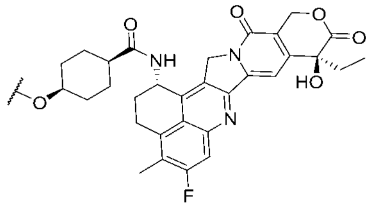,
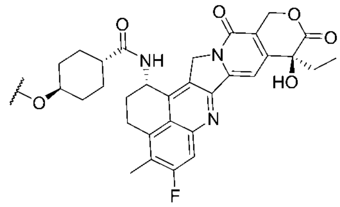,

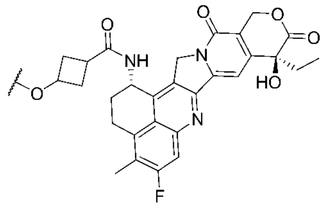,
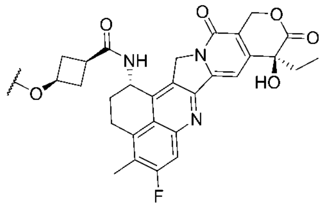,
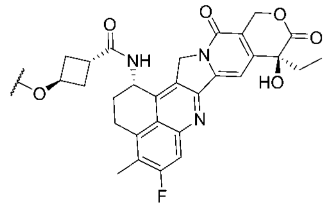,

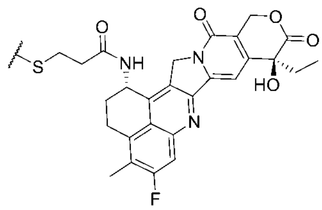,
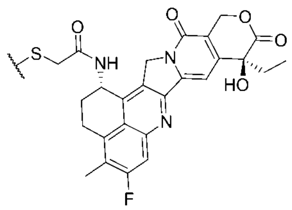,
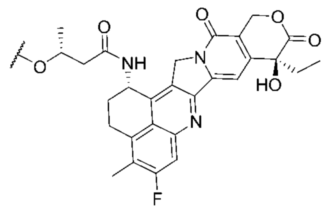,

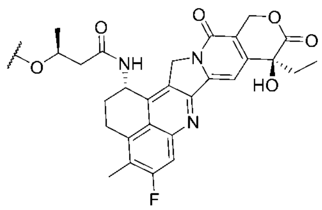,
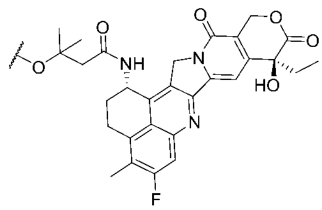, and
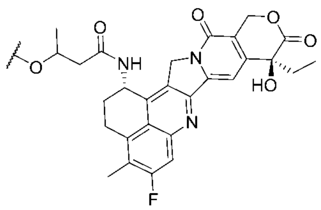.

In some embodiments, in the bispecific antibody-drug conjugate according to the present disclosure, the linker unit L is $-L_a-L_b-L_c-$; and the $L_c$ is connected to the cytotoxic drug;

$-L_a-$ is

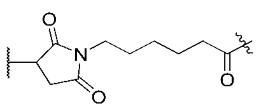 or 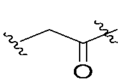;

$-L_a-$ is connected to the $L_b$;

$-L_b-$ is any one of the following structures:

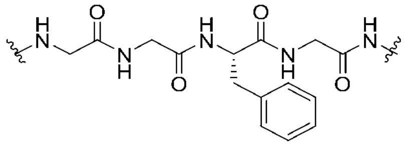,

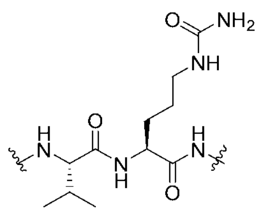, 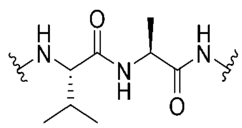, 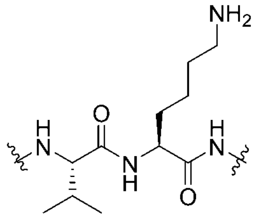, 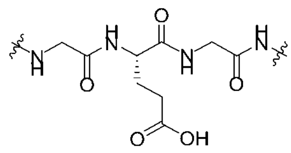,

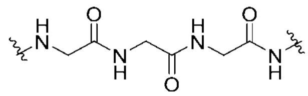, and 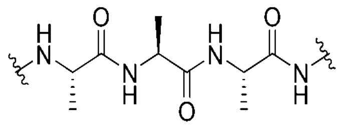;

preferably

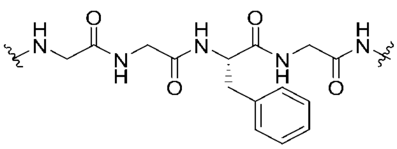 or 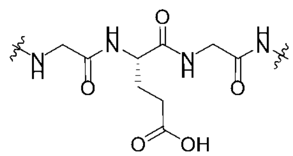;

and the right end of the aforementioned structure is preferably connected to the $L_c$;
$-L_c-$ is

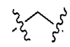.

In some embodiments, in the bispecific antibody-drug conjugate according to the present disclosure, the linker unit L is

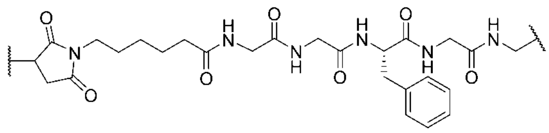,

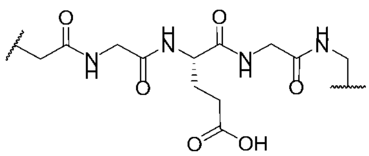, or 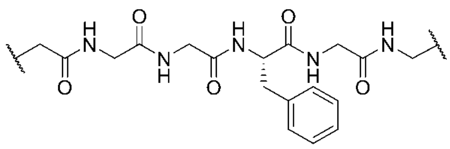;

preferably

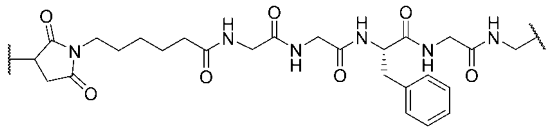.

In some embodiments, the bispecific antibody-drug conjugate according to the present disclosure has a structure represented by formula (A-2):

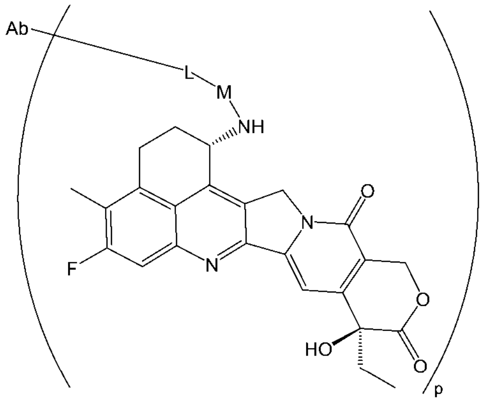

(A-2)

wherein

p represents the average number of connections, and p is any integer or decimal from 1 to 10; preferably any integer or decimal from 2 to 8; for example, 4.02 or 3.95;

Ab and M are each as defined in any one of the embodiments of the present disclosure; L is the linker unit L according to any one of the embodiments of the present disclosure.

In some embodiments, the bispecific antibody-drug conjugate according to the present disclosure has a structure represented by formula (A-2):

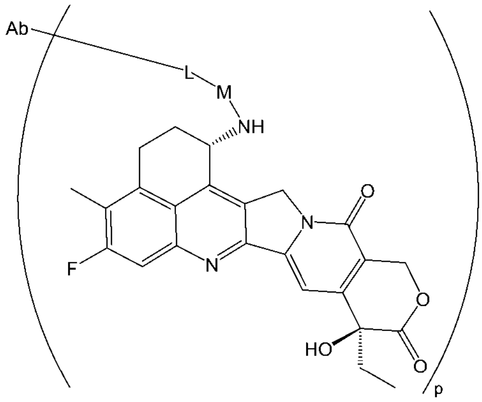

(A-2)

wherein

p represents the average number of connections, and p is any integer or decimal from 1 to 10; preferably any integer or decimal from 2 to 8; for example, 4.02 or 3.95;

M is $-L^2-L^1-C(O)-$;

$L^2$ is -O- or -S-, and $L^2$ is connected to L;

$L^1$ is $-(C(R^{1a})(R^{1b}))_mCH_2-$, $C_3$-$C_6$ saturated cycloalkyl, or 3- to 6-membered saturated heterocyclyl, wherein the $C_3$-$C_6$ saturated cycloalkyl and 3- to 6-membered saturated heterocyclyl are each independently and optionally substituted by one or more $R^{2a}$;

m is 1, 2, 3, or 4; heteroatoms in the 3- to 6-membered saturated heterocyclyl are each independently N, O, or S, and the number of heteroatoms is 1, 2, or 3;

$R^{1a}$, $R^{1b}$, and $R^{2a}$ are each independently hydrogen, halogen, hydroxyl, amino, or $C_1$-$C_6$ alkyl, wherein the $C_1$-$C_6$ alkyl is optionally substituted by one or more halogens.

In some embodiments, the bispecific antibody-drug conjugate according to the present disclosure has a structure represented by formula (A-2a) or (A-2b):

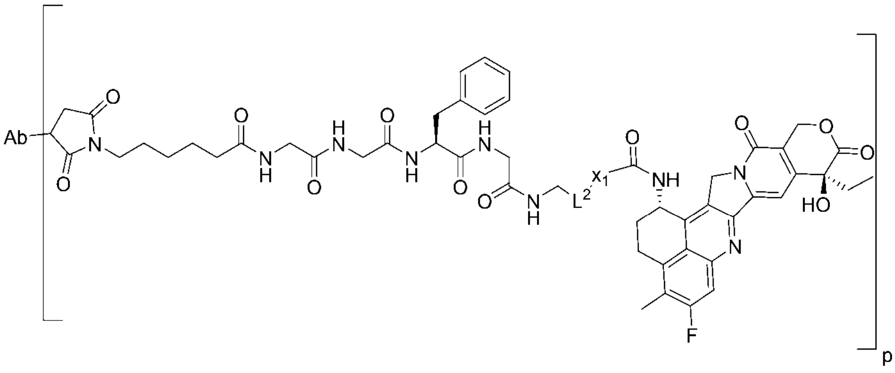

(A-2a)

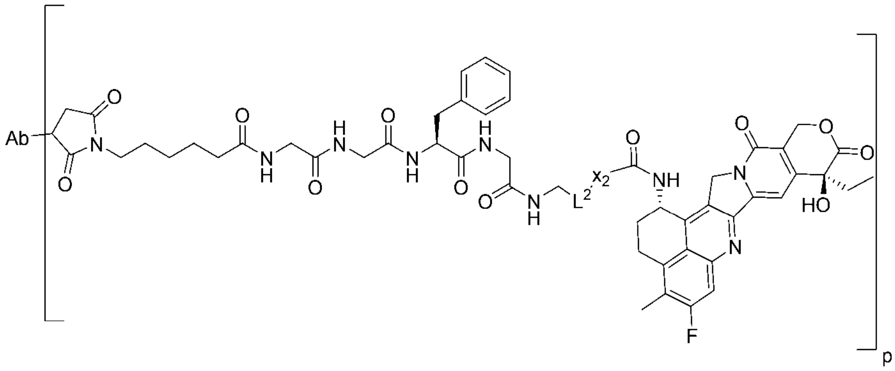

(A-2b),

wherein

p represents the average number of connections, and p is any integer or decimal from 1 to 10, preferably any integer or decimal from 2 to 8; for example, 4.02 or 3.95;
Ab is the bispecific antibody or the antigen-binding fragment thereof according to any one of the embodiments of the present disclosure;
$L^2$ is -O- or -S-; preferably -O-;
$X_1$ is $C_3$-$C_6$ saturated cycloalkyl optionally substituted by 1, 2, or 3 $R^{2a}$;
$X_2$ is $-(C(R^{1a})(R^{1b}))_m-CH_2-$;
m is 1 or 2;
$R^{1a}$, $R^{1b}$, and $R^{2a}$ are each independently hydrogen, halogen, or $C_1$-$C_6$ alkyl; the $C_1$-$C_6$ alkyl is optionally substituted by one or more halogens.

In some embodiments, in the bispecific antibody-drug conjugate according to the present disclosure, the bispecific antibody-drug conjugate is selected from the group consisting of any one of the following structures:

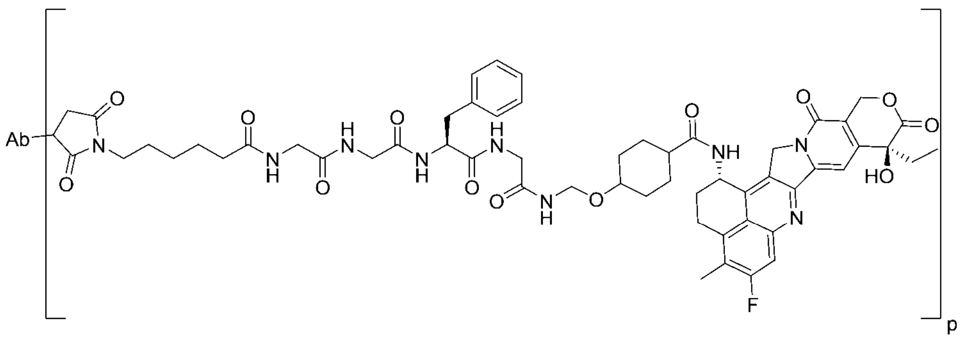

,

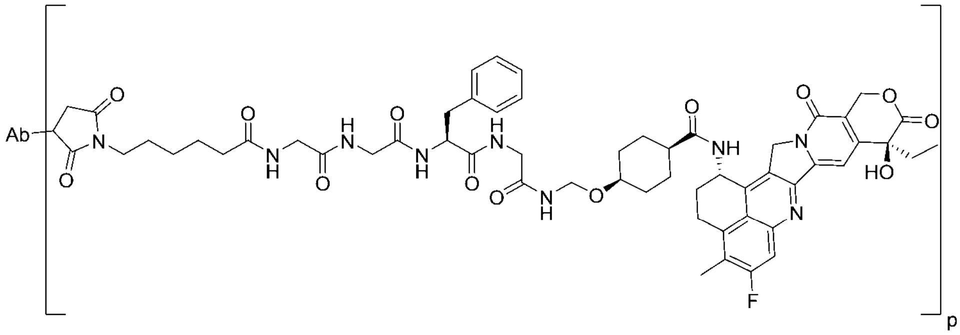
,

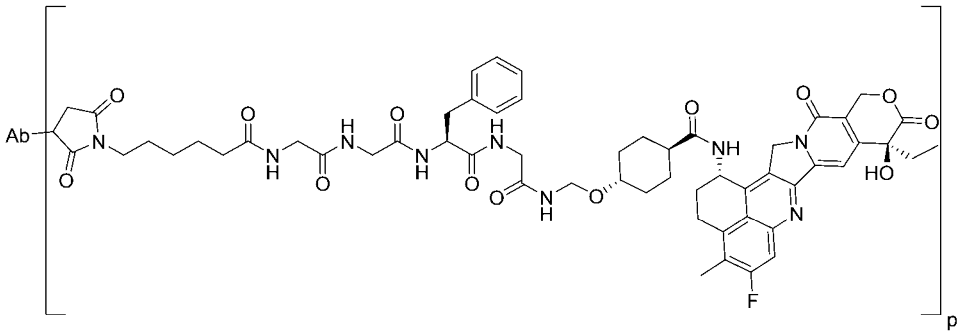
,

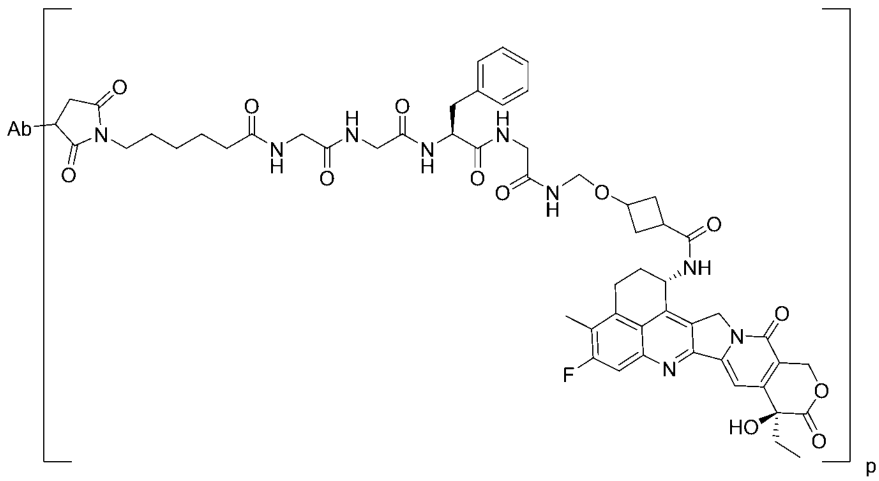
,

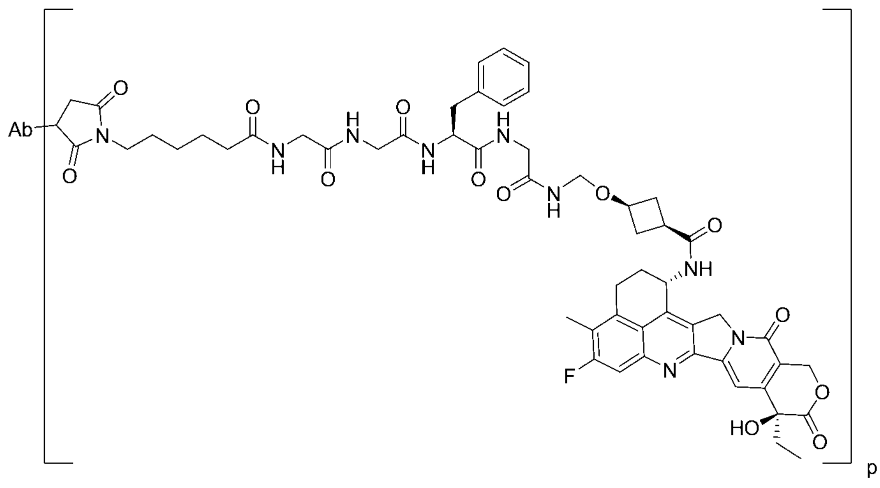
,

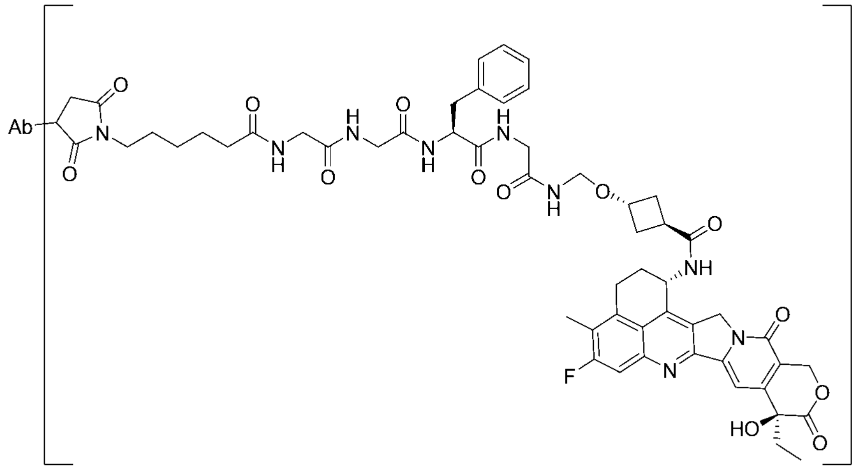

p,

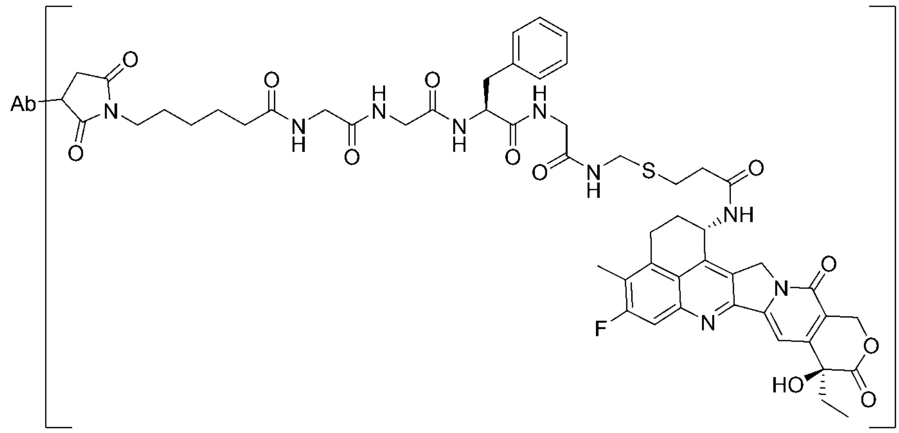

p,

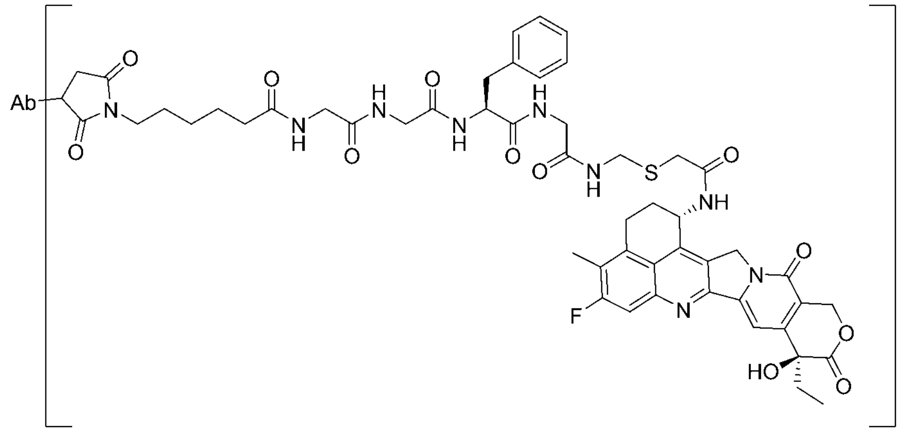

p,

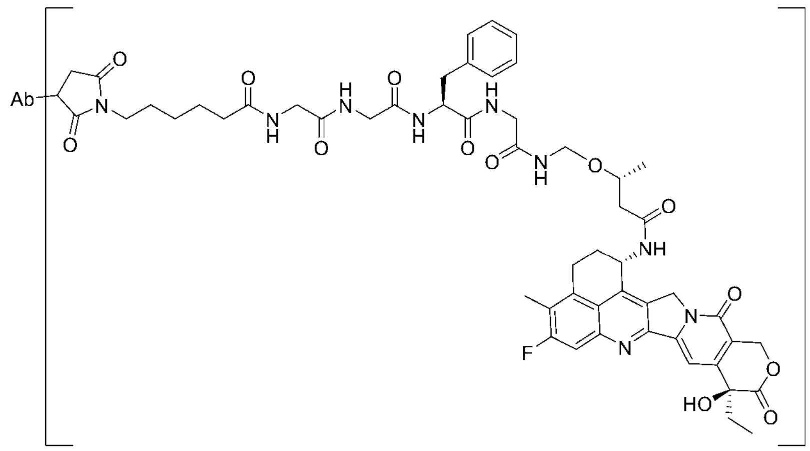

p ,

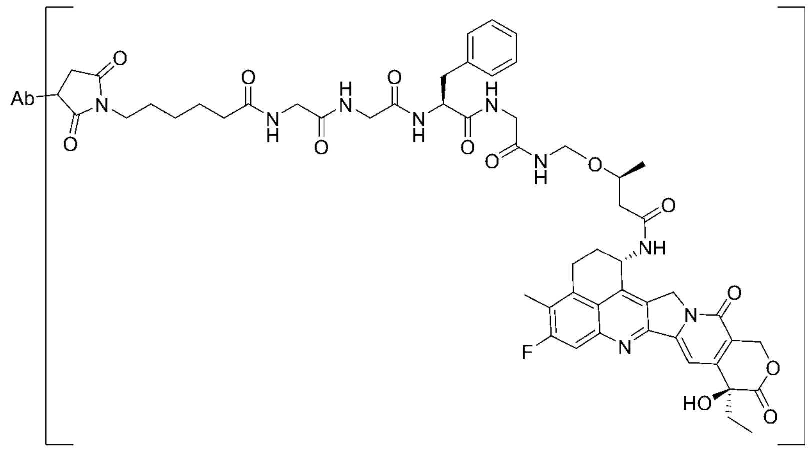

p ,

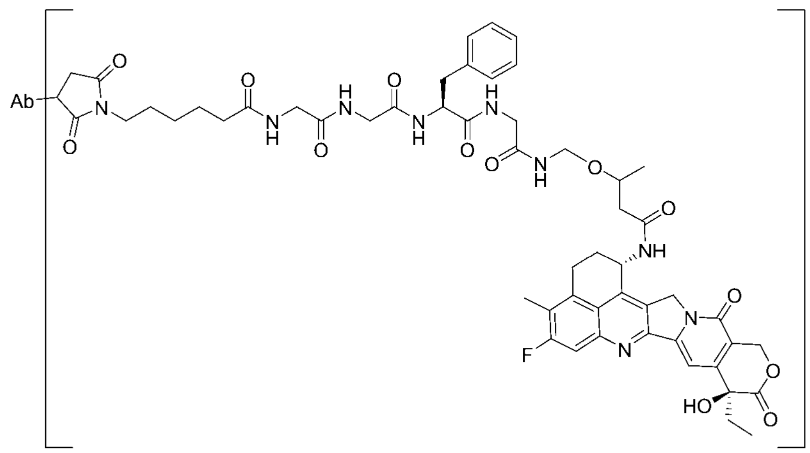

p ,

and

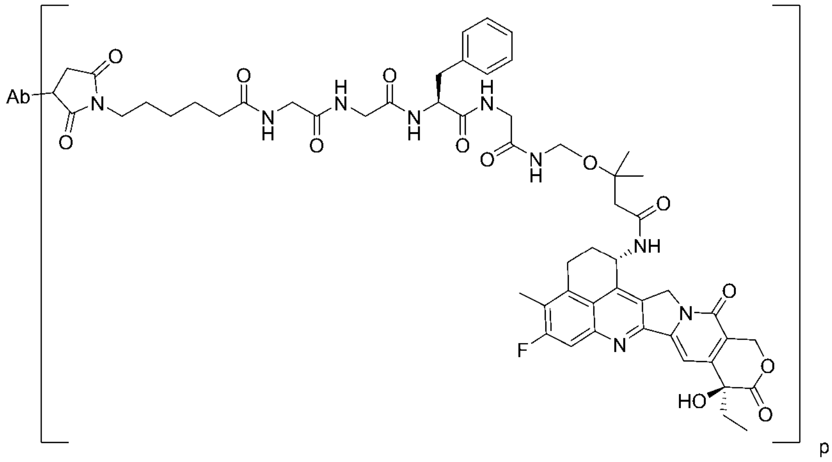;

wherein

p represents the average number of connections, and p is any integer or decimal from 1 to 10, preferably any integer or decimal from 2 to 8; for example, 4.02 or 3.95;
Ab is the bispecific antibody or the antigen-binding fragment thereof according to any one of the embodiments of the present disclosure.

In another aspect, the present disclosure provides a bispecific antibody-drug conjugate, wherein the bispecific antibody-drug conjugate is selected from the group consisting of any one of the following structures:

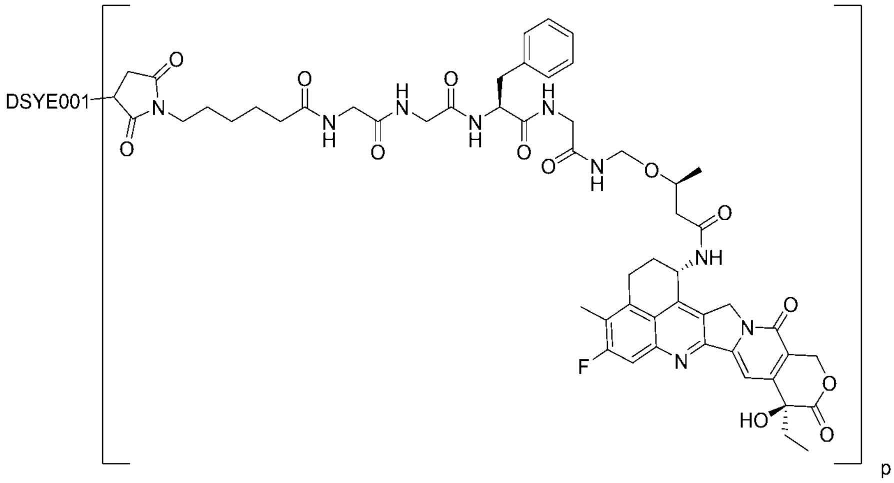,

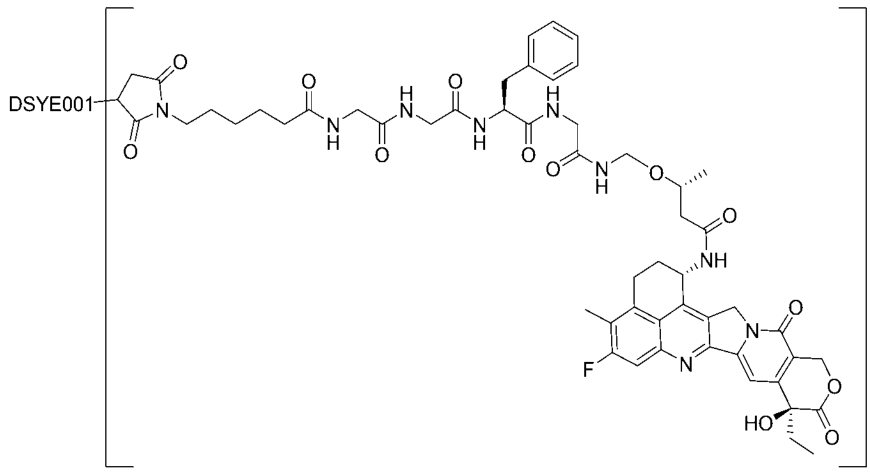

p,

and

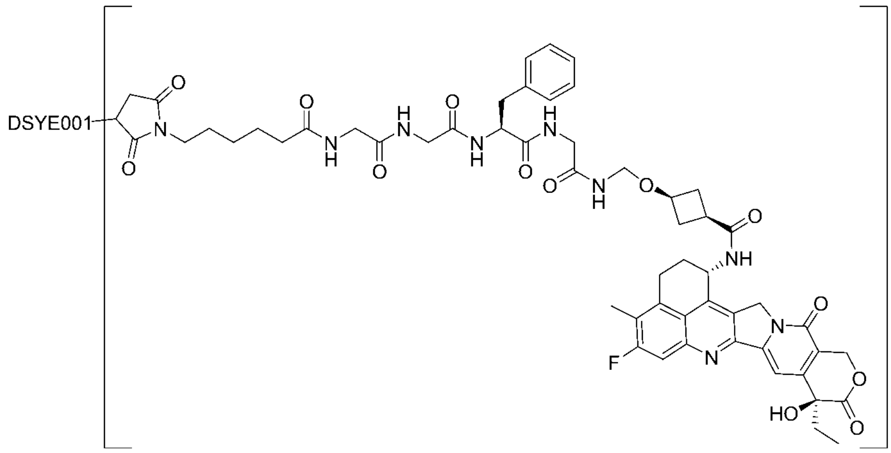

p

wherein

p represents the average number of connections, and p is any integer or decimal from 1 to 10, preferably any integer or decimal from 3 to 8, preferably any integer or decimal from 4 to 8, preferably any integer or decimal from 6 to 8; for example, 4.02, 3.95, 6.1, or 7.9;

DSYE001 is an anti-B7H3 and PD-L1 bispecific antibody, wherein the heavy chain amino acid sequence of the bispecific antibody is as shown in SEQ ID NO.: 16, and the light chain amino acid sequence is as shown in SEQ ID NO.: 9.

In some embodiments, in the bispecific antibody-drug conjugate according to the present disclosure (e.g., the bispecific antibody-drug conjugate represented by formula (A-2), (A-2a), or (A-2b) according to the present disclosure), p represents the average number of connections, and p is any integer or decimal from 1 to 10, preferably any integer or decimal from 2 to 8, preferably any integer or decimal from 4 to 8, preferably any integer or decimal from 6 to 8; for example, 4.02, 3.95, 6.1, or 7.9.

In some embodiments, the average number of connections p according to the present disclosure may be any integer or decimal from 2 to 8. For example, the average number of connections p may be any integer or decimal from 3 to 8. For example, the average number of connections p may be any integer or decimal from 1 to 2, 2 to 3, 3 to 4, 4 to 5, 5 to 6, 6 to 7, 7 to 8, 8 to 9, or 9 to 10.

In another aspect, the present disclosure provides a bispecific antibody-drug conjugate, wherein the bispecific antibody-drug conjugate is selected from the group consisting of any one of the following structures (p represents the average number of connections):

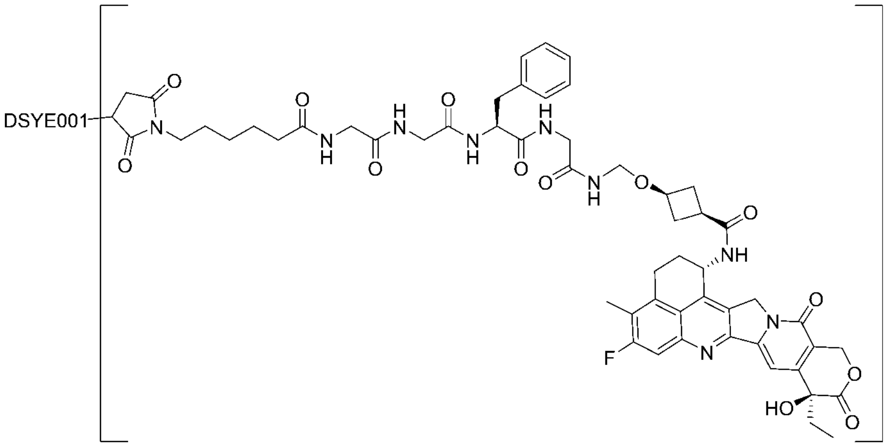

p=4.02,

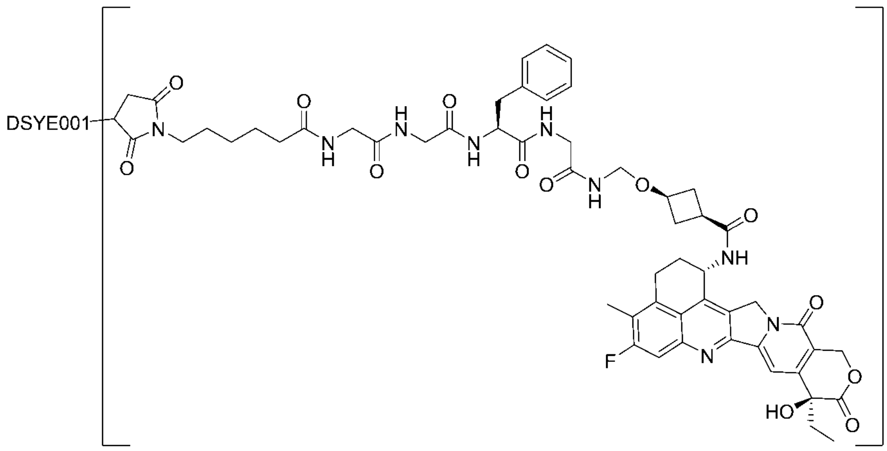

p=6.1,

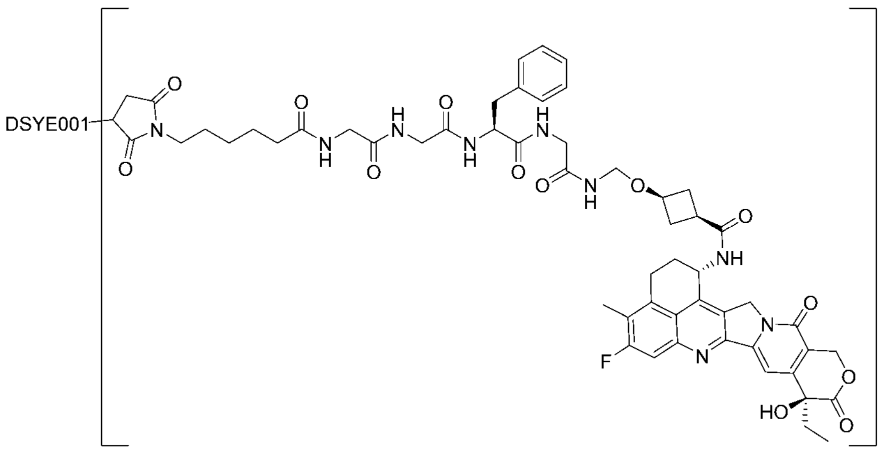

p=7.9,

and

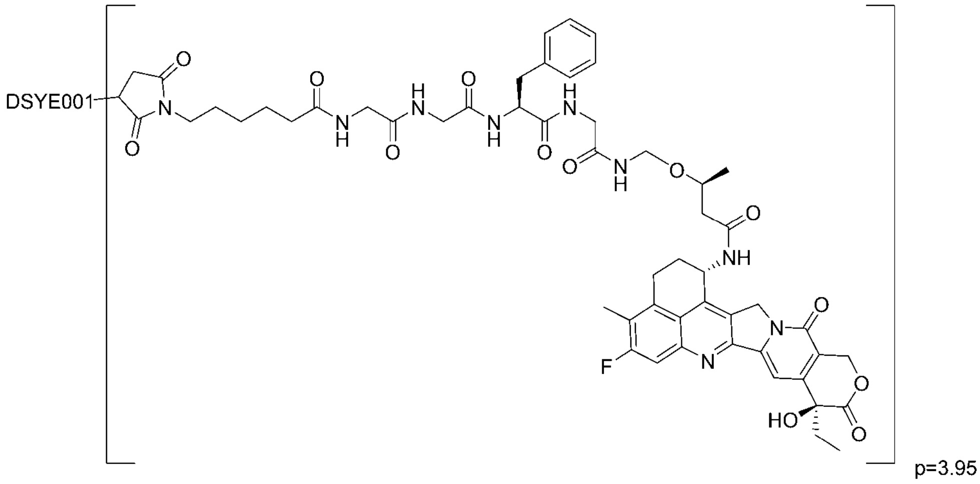.

The amino acid sequence of the anti-B7H3 and PD-L1 bispecific antibody DSYE001 according to the present disclosure is shown in the sequence listing. The numbering method for the antibody CDR in the present disclosure is: Kabat numbering.

In another aspect, the present disclosure provides a bispecific antibody-drug conjugate, wherein the bispecific antibody-drug conjugate is selected from the group consisting of any one of the following structures:

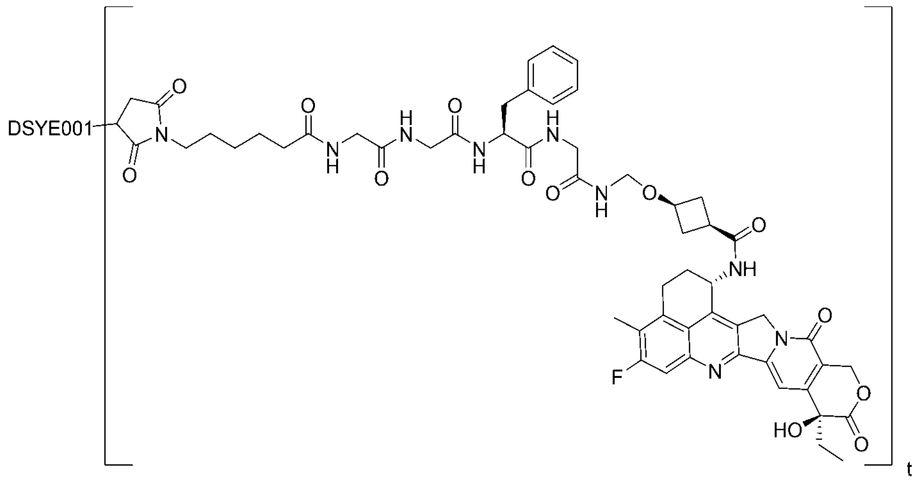

and

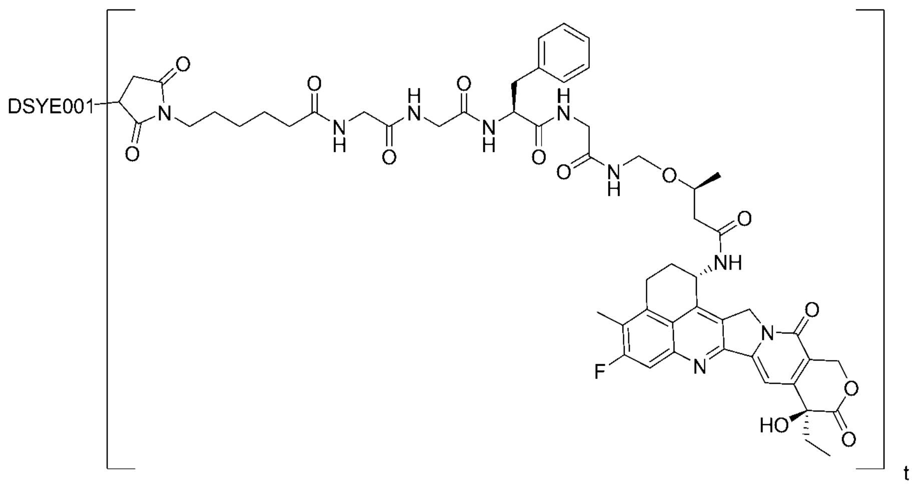,

wherein
t represents the number of connections, and t is any integer from 1 to 10, preferably any integer from 2 to 8, preferably any integer from 4 to 8; for example, 4, 6, or 8.

The amino acid sequence of the anti-B7H3 and PD-L1 bispecific antibody DSYE001 according to the present disclosure is shown in the sequence listing. The numbering method for the antibody CDR in the present disclosure is: Kabat numbering.

In some embodiments, the number of connections t according to the present disclosure is any integer from 1 to 10, preferably any integer from 2 to 8. For example, the number of connections t may be any integer from 3 to 8. For example, the number of connections t is any integer selected from 1, 2, 3, 4, 5, 6, 7, 8, 9, or 10.

In another aspect, the present disclosure provides a method for preparing a bispecific antibody-drug conjugate, comprising the following steps: mixing the bispecific antibody dissolved in a buffer with the linker-payload dissolved in a solvent in the presence of a reducing agent to obtain the bispecific antibody-drug conjugate.

In some embodiments, the reducing agent is a conventional reducing agent for such reactions in the art, such as tris(2-carboxyethyl)phosphine hydrochloride.

In some embodiments, the buffer is a conventional buffer for such reactions in the art.

In some embodiments, the solvent is a conventional solvent for such reactions in the art, such as dimethylacetamide.

In another aspect, the present disclosure provides a pharmaceutical composition comprising the bispecific antibody-drug conjugate according to any one of the embodiments of the present disclosure, and a pharmaceutically acceptable carrier or excipient.

A further object of the present disclosure is to provide a method for preparing the pharmaceutical composition of the present disclosure, comprising combining the bispecific antibody-drug conjugate according to any one of the embodiments of the present disclosure, or a pharmaceutically acceptable form thereof, or a mixture thereof, with one or more pharmaceutically acceptable carriers or excipients.

In the present disclosure, the pharmaceutically acceptable carrier used in the pharmaceutical composition includes examples of suitable pharmaceutically acceptable carriers as described in Remington's Pharmaceutical Sciences (2005).

In the present disclosure, the pharmaceutical composition may be administered in any form, as long as it achieves the prevention, alleviation, inhibition, or cure of symptoms in human or animal patients. For example, various suitable dosage forms may be prepared according to the route of administration.

In some other embodiments, administration of the bispecific antibody-drug conjugate according to any one of the embodiments of the present disclosure, or the pharmaceutical composition, may be combined with an additional therapeutic method. The additional therapeutic method may be selected from, but not limited to, radiation therapy, chemotherapy, immunotherapy, or a combination thereof.

In another aspect, the present disclosure provides a pharmaceutical preparation comprising the bispecific antibody-drug conjugate according to any one of the embodiments of the present disclosure, or a pharmaceutically acceptable form thereof, or a mixture thereof as an active ingredient, or the pharmaceutical composition according to any one of the embodiments of the present disclosure. In some embodiments, the preparation is in the form of a solid preparation, a semi-solid preparation, a liquid preparation, or a gaseous preparation.

In another aspect, the present disclosure provides a use of the bispecific antibody-drug conjugate according to any one of the embodiments of the present disclosure, or the pharmaceutical composition according to any one of the embodiments of the present disclosure in the manufacture of a medicament for treating and/or preventing cancer; preferably, the cancer is cancer with positive expression of B7H3 and/or PD-L1.

In another aspect, the present disclosure provides a method for treating and/or preventing cancer, comprising administering to a subject in need thereof the bispecific antibody-drug conjugate according to any one of the embodiments of the present disclosure, or the pharmaceutical composition according to any one of the embodiments of the present disclosure; preferably, the cancer is cancer with positive expression of B7H3 and/or PD-L1.

In another aspect, the present disclosure provides the bispecific antibody-drug conjugate according to any one of the embodiments of the present disclosure, or the pharmaceutical composition according to any one of the embodiments of the present disclosure for use in treating and/or preventing cancer; preferably, the cancer is cancer with positive expression of B7H3 and/or PD-L1.

In some embodiments, the cancer according to the present disclosure is selected from the group consisting of one or more of lung cancer, gastric cancer, liver cancer, colorectal cancer, melanoma, nephroma, ovarian cancer, prostate cancer, bladder cancer, breast cancer, esophageal cancer, carcinoma of large intestine, nasopharyngeal cancer, brain tumor, cervical cancer, blood cancer, bone cancer, lymphoma, pancreatic cancer, and Ewing's sarcoma; preferably, the cancer is lung cancer, prostate cancer, breast cancer, ovarian cancer, or melanoma.

In some embodiments, the route of administration of the present disclosure includes, but is not limited to, oral, intravenous, subcutaneous, intramuscular, intra-arterial, intraarticular (e.g., in arthritic joints), inhalation, aerosol delivery,

intratumoral administration, etc.

In some embodiments, the present disclosure provides one or more therapies (e.g., treatment modalities and/or other therapeutic agents) co-administered in a therapeutically effective amount to a subject. In some embodiments, the therapy comprises surgical treatment and/or radiation therapy.

In some embodiments, the method or use provided by the present disclosure further comprises administering one or more therapies (e.g., treatment modalities and/or other therapeutic agents) to an individual. The antibody-drug conjugate of the present disclosure or a pharmaceutically acceptable salt thereof may be used alone or in combination with other therapeutic agents in therapy. For example, it may be co-administered with at least one additional therapeutic agent.

In another aspect, the present disclosure provides a pharmaceutical combination comprising the anti-B7H3 and PD-L1 bispecific antibody-drug conjugate as described herein, or a pharmaceutically acceptable salt thereof or a mixture thereof, or the pharmaceutical composition as described herein, and one or more additional therapeutic agents.

In another aspect, the present disclosure provides a kit comprising the anti-B7H3 and PD-L1 bispecific antibody-drug conjugate according to any one of the embodiments of the present disclosure, or a pharmaceutically acceptable salt thereof or a mixture thereof, or the pharmaceutical composition according to any one of the embodiments of the present disclosure, preferably further comprising a drug delivery device.

**Definitions of Terms**

Unless otherwise specified, the implementation of the present disclosure will employ conventional techniques of molecular biology (including recombinant technology), microbiology, cell biology, biochemistry, and immunology, all of which fall within the technical scope of the art.

To facilitate a better understanding of the present disclosure, certain technical terms are specifically defined as follows. Unless otherwise expressly defined elsewhere herein, all technical terms used herein have the meanings commonly understood by those of ordinary skill in the art to which the present disclosure pertains. For definitions and terms in the art, one skilled in the art may refer to *Current Protocols in Molecular Biology* (Ausubel). The abbreviations for amino acid residues are the standard three-letter and/or one-letter codes used in the art to refer to one of the 20 commonly used L-amino acids.

In the present disclosure, the term "B7H3", also known as CD276 antigen, refers to a type I transmembrane protein belonging to the B7 family, which possesses an extracellular domain consisting of a single IgV-IgC domain. B7 family proteins contain extracellular IgV-like and IgC-like domains and possess short cytoplasmic tails. B7H3 is an immune checkpoint molecule that is abnormally overexpressed in various cancers. The amino acid sequence of B7H3 protein comprises full-length B7H3 proteins (e.g., human 4IgB7H3 protein or human 2IgB7H3 protein), or the extracellular domain of B7H3 (B7H3 ECD) or fragments containing the B7H3 ECD; B7H3-ECD fusion proteins. Exemplary sequences of B7H3 protein are shown in Uniprot ID: Q5ZPR3 (human 4IgB7H3), Genebank accession numbers NP_001019907 (human), NP_001316557 (human), NP_001316558 (human), NP_079516 (human), and NP_598744 (mouse). The amino acid sequence homology of cynomolgus monkey B7H3 with human B7H3 and mouse B7H3 is approximately 97% and 88%, respectively.

In the present disclosure, the term "PD-L1", programmed cell death 1 ligand 1 (PD-L1), also known as cluster of differentiation 274 (CD274) or B7 homolog 1 (B7-H1), is a 40 kDa type I transmembrane protein. PD-L1 is the surface glycoprotein ligand of PD-1, which is a key immune checkpoint receptor expressed by activated T cells and B cells and mediates immune suppression.

In the present disclosure, the term "approximately" when used in conjunction with a numerical value is intended to encompass numerical values within a range having a lower limit of 5% less than the specified numerical value and an upper limit of 5% greater than the specified numerical value, including, but not limited to, ±5%, ±2%, ±1%, and ±0.1%, as such variations are suitable for performing the disclosed methods.

In the present disclosure, the term "and/or" should be understood to mean any one of the options or a combination of any two or more of the options.

In the present disclosure, the term "or" should be understood to have the same meaning as "and/or" as defined above. For example, when separating items in a list, "or" or "and/or" should be construed as being inclusive, that is, including at least one, but also more than one, of the quantities or elements in the list, and optionally, additional unlisted items. Only terms expressly indicated to the contrary, such as "only one" or "exactly one", or "consisting of" used in the claims, will refer to only one number or only one element in the list.

In the present disclosure, the words "a" and "an" should be understood as "at least one", unless the context clearly indicates otherwise.

In the present disclosure, the term "antibody-drug conjugate" generally refers to an antibody connected to a biologically active cytotoxic drug via a stable linker unit. In the present disclosure, an "antibody-drug conjugate" may be a bispecific antibody-drug conjugate, which may refer to a bispecific antibody or an antigen-binding fragment thereof connected to a biologically active cytotoxic drug fragment via a stable linker unit.

In the present disclosure, the term "cytotoxic drug" generally refers to a toxic drug, which may have chemical molecules capable of significantly disrupting the normal growth of tumor cells. Cytotoxic drugs can kill tumor cells at sufficiently high concentrations. The "cytotoxic drug" may include payloads, such as small molecule payloads or enzymatically active payloads derived from bacterial, fungal, plant, or animal sources, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, or radioactive isotopes of Lu), toxic drugs, chemotherapeutic drugs, antibiotics, or nucleolytic enzymes, or their derivatives. For example, cytotoxic drugs may be toxic drugs, including, but not limited to, camptothecin derivatives, such as Exatecan (chemical name: (1S,9S)-1-amino-9-ethyl-5-fluoro-2,3-dihydro-9-hydroxy-4-methyl-1H,12H-benzo[de]pyrano[3',4':6,7]imidazo[1,2-b]quinoline-10,13(9H,15H)-dione).

In the present disclosure, the term "antibody" generally refers to an immunoglobulin that is reactive to a specified protein or peptide or a fragment thereof. The antibody may be antibodies from any class, including, but not limited to, IgG, IgA, IgM, IgD, and IgE, as well as antibodies from any subclass (e.g., IgG1, IgG2, IgG3, and IgG4). The antibody may have a heavy chain constant region selected from, for example, IgG1, IgG2, IgG3, or IgG4. The antibody may also have a light chain selected from, for example, kappa ($\kappa$) or lambda ($\lambda$). The antibody of the present disclosure may be derived from any species. The term "antibody" may include intact polyclonal antibodies, intact monoclonal antibodies, multispecific antibodies (e.g., bispecific antibodies), chimeric antibodies, humanized antibodies, human antibodies, fusion proteins comprising antibodies, and any other modified immunoglobulin molecules, provided that such antibodies exhibit the desired biological activity.

In the present disclosure, the term "antigen-binding fragment" generally refers to a portion of an antibody molecule that contains amino acids responsible for the specific binding between an antibody and an antigen. The portion of an antigen that is specifically recognized and bound by an antibody is referred to as an "epitope" as described above. As described above, the antigen-binding domain may typically comprise an antibody light chain variable region (VL) and an antibody heavy chain variable region (VH); however, it is not necessary to comprise both. An Fd fragment, for example, comprises two VH regions and generally retains some antigen-binding functionality of the intact antigen-binding domain. Examples of antigen-binding fragments of an antibody include (1) a Fab fragment, a monovalent fragment having VL, VH, constant light chain (CL), and CH1 domains; (2) an F(ab')2 fragment, a bivalent fragment comprising two Fab fragments linked by a disulfide bridge at the hinge region; (3) an Fd fragment having two VH and CH1 domains; (4) an Fv fragment having VL and VH domains of a single arm of an antibody; (5) a dAb fragment (Ward et al., "Binding Activities of a Repertoire of Single Immunoglobulin Variable Domains Secreted From Escherichia coli", Nature 341: 544-546 (1989), the contents of which are incorporated herein by reference in their entirety) having a VH domain; (6) an isolated complementarity determining region (CDR); (7) a single-chain Fv (scFv), such as one derived from an scFv library. Although the two domains of the Fv fragment, VL and VH, are encoded by separate genes, they can be joined using recombinant methods via a synthetic linker, enabling their preparation as a single protein chain (referred to as single-chain Fv (scFv)) in which the VL and VH regions are paired to form a monovalent molecule (see, e.g., Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85:5879-5883 (1988)); (8) "VHH" refers to the variable antigen-binding domain derived from a camelid (camel, dromedary, llama, alpaca, etc.) heavy-chain antibody (see Nguyen V.K. et al., 2000, The EMBO Journal, 19, 921-930; Muyldermans S., 2001, J Biotechnol., 74, 277-302; and for review Vanlandschoot P. et al., 2011, Antiviral Research 92, 389-407). VHH may also be referred to as a nanobody (Nb).

In the present disclosure, the term "variable region" or "variable domain" generally refers to the domain of an antibody heavy or light chain that is involved in binding of the antibody to an antigen. In the present disclosure, the term "variable" generally refers to certain portions of the sequence of the variable domains of an antibody that exhibit substantial variation, resulting in the binding and specificity of various specific antibodies to their respective antigens. Variability is not uniformly distributed throughout the variable region of an antibody. It is concentrated in three segments of the light chain variable region and heavy chain variable region, referred to as complementarity-determining regions (CDRs) or hypervariable regions (HVRs), namely LCDR1, LCDR2, LCDR3, HCDR1, HCDR2, and HCDR3. The more highly conserved portions within the variable domains are referred to as framework regions (FRs). The natural heavy and light chain variable domains each comprise four FRs (H-FR1, H-FR2, H-FR3, H-FR4, L-FR1, L-FR2, L-FR3, L-FR4), predominantly adopting a $\beta$-sheet conformation, connected by three CDR structural loop regions. The CDRs in each chain are closely juxtaposed via the FRs and, together with the CDRs from the other chain, form the antigen-binding site of the antibody.

In the present disclosure, the variable region of an antibody or the CDRs of an antibody can be encoded or delineated by various methods in the art, such as the Kabat numbering scheme and definition rules based on sequence variability (see, Kabat et al., Sequences of Proteins of Immunological Interest, 5th Edition, National Institutes of Health, Bethesda, MD (1991)), the Chothia numbering scheme and definition rules based on the location of structural loop regions (see, A1-Lazikani et al., JMol Biol 273:927-48, 1997), the IMGT numbering scheme and definition rules based on amino acid sequence alignment of germline V genes by Lefranc et al., as well as the Honneger's numbering scheme (AHo's), the Martin numbering scheme, the Gelfand numbering scheme, etc., which can be referred to in Mathieu Dondelinger et al., Understanding the Significance and Implications of Antibody Numbering and Antigen-Binding Surface/Residue Definition,

Front. Immunol., 16 October 2018.

In the present disclosure, the term "monoclonal antibody" refers to an antibody derived from a substantially homogeneous population of antibodies, i.e., the individual antibodies constituting the population are identical, except for possible naturally occurring mutations that may be present in minor quantities. Monoclonal antibodies are highly specific and are directed against a single antigenic epitope. In contrast, conventional (polyclonal) antibody preparations typically comprise a large number of antibodies directed against (or specific for) different epitopes. The modifier "monoclonal" indicates the characteristics of an antibody derived from a substantially homogeneous population of antibodies and should not be construed as requiring the antibody to be produced by any particular method.

In the present disclosure, the term "multispecific antibody" refers to an antibody comprising two or more antigen-binding domains capable of binding to two or more different epitopes (e.g., two, three, four, or more different epitopes), which may be located on the same or different antigens. Examples of multispecific antibodies include "bispecific antibodies" that bind to two different antigens or two different epitopes. Bispecific antibodies targeting B7H3 and PD-L1 disclosed herein may be referred to as, for example, "anti-B7H3/PD-L1" or "anti-B7H3×PD-L1" or "B7H3×PD-L1" bispecific molecules, or other similar terms.

In the present disclosure, the term "nanobody" refers to a heavy-chain single-domain antibody VHH (variable domain of heavy chain of heavy-chain antibody), which comprises only one heavy chain variable region (VHH) as well as CH2 and CH3 domains, and is naturally devoid of a light chain compared to other antibodies. It is composed of heavy chain variable regions of camelids (camels, llamas, alpacas, and their closely related species). Nanobody crystals have a diameter of 2.5 nm and a length of 4 nm, being the smallest naturally occurring fragments that can bind to antigens.

In the present disclosure, the term "domain antibody" refers to an immunologically functional immunoglobulin fragment containing only a heavy chain variable region or a light chain variable region. In certain circumstances, two or more VH regions are covalently linked to a peptide linker to form a bivalent domain antibody. The two VH regions of the bivalent domain antibody may target the same or different antigens.

In the present disclosure, the term "humanized antibody" refers to an antibody form containing sequences derived from human and non-human (e.g., mouse, rat) antibodies. Generally, a humanized antibody comprises substantially all of at least one, typically two, variable domains, wherein all or substantially all of the hypervariable loops correspond to those of a non-human immunoglobulin, while all or substantially all of the framework regions (FRs) are those of a human immunoglobulin sequence. The humanized antibody may optionally comprise at least a portion of a human immunoglobulin constant region (Fc).

In the present disclosure, an antibody "isotype" refers to the class of antibody provided by the heavy chain constant region gene (e.g., IgM, IgE, IgG such as IgG1, IgG2, or IgG4). Isotypes also include modified forms of one of these classes, where modifications have been made to alter Fc function, for example, to enhance or weaken effector function or binding to Fc receptors.

In the present disclosure, the term "Fc region" is used herein to define the C-terminal region of an immunoglobulin heavy chain comprising at least a portion of the constant region. The term includes both native sequence Fc regions and variant Fc regions. In some embodiments, the human IgG heavy chain Fc region extends from Cys226 or Pro230 to the carboxyl terminus of the heavy chain. However, the C-terminal lysine (Lys447) of the Fc region may be present or absent (the numbering in this paragraph is based on the EU numbering system, also known as the EU index, as described in Rabat et al., Sequences of Proteins of Immunological Interest, 5th Ed., Public Health Service, National Institutes of Health, Bethesda, MD, 1991).

In the present disclosure, the term "cross reaction" refers to the binding to antigenic fragments of the same target molecule of human, monkey, and/or murine (mouse or rat) origin. Therefore, "cross reaction" should be understood as an interspecies reaction of an antigen-binding molecule (e.g., an antibody) with a cognate molecule (e.g., BDCA2) expressed in a different species. The specificity of the cross reaction of monoclonal antibodies recognizing human BDCA2, monkey BDCA2, and/or murine (mouse or rat) BDCA2 may be determined by FACS analysis.

In the present disclosure, "affinity" or "binding affinity" refers to the inherent binding affinity that reflects the interaction between members of a binding pair. The affinity of molecule X for its partner Y can be typically represented by the equilibrium dissociation constant ($K_D$), which is the ratio of the dissociation rate constant to the binding rate constant ($k_{dis}$ and $k_{on}$, respectively). Affinity may be measured by common methods known in the art. In some embodiments of the present disclosure, surface plasmon resonance (SPR) technology is used to measure affinity, such as the affinity between an antibody and an antigen of the present disclosure. In some preferred embodiments of the present disclosure, one specific method for measuring affinity is the BIAcore method disclosed herein.

In the present disclosure, the term "non-binding" protein or cell refers to not binding to the protein or cell, or not binding to the protein or cell with high affinity, i.e., binding to the protein or cell with a $K_D$ of $1.0 \times 10^{-6}$ M or higher, more preferably $1.0 \times 10^{-5}$ M or higher, further more preferably $1.0 \times 10^{-4}$ M or higher, $1.0 \times 10^{-3}$ M or higher, still more preferably $1.0 \times 10^{-2}$ M or higher.

In the present disclosure, the term "high affinity" for an IgG antibody refers to a $K_D$ for the antigen of $1.0 \times 10^{-6}$ M or lower, preferably $5.0 \times 10^{-8}$ M or lower, more preferably $1.0 \times 10^{-8}$ M or lower, $5.0 \times 10^{-9}$ M or lower, and further more

preferably 1.0 × $10^{-9}$ M or lower. For other antibody isotypes, "high-affinity" binding may vary. For example, "high-affinity" binding for an IgM isotype refers to a $K_D$ of $10^{-6}$ M or lower, preferably $10^{-7}$ M or lower, and more preferably $10^{-8}$ M or lower.

In the present disclosure, the term "percent (%) amino acid sequence identity" or simply "identity" is defined as the percentage of amino acid residues in a candidate amino acid sequence that are identical to those in a reference amino acid sequence after aligning the amino acid sequences (and introducing gaps if necessary) to achieve maximum percent sequence identity, without considering any conservative substitutions as part of the sequence identity. Sequence alignment may be performed in order to determine percent amino acid sequence identity using various methods in the art, for example, using publicly available computer software such as BLAST, BLAST-2, ALIGN, or MEGALIGN (DNASTAR) software. Those skilled in the art may determine suitable parameters for measurement comparison, including any algorithm required to achieve maximum alignment of the full-length sequences being compared.

In the present disclosure, the term "halogen" generally refers to fluorine, chlorine, bromine, or iodine, for example, fluorine or chlorine.

In the present disclosure, the term "alkyl" generally refers to a residue derived from an alkane by removal of a hydrogen atom. The alkyl group may be substituted or unsubstituted, or replaced or unreplaced. The term "alkyl" generally refers to a saturated linear or branched aliphatic hydrocarbon group having a residue derived from the parent alkane by removal of hydrogen atoms from the same carbon atom or two different carbon atoms, which may be a linear or branched group containing 1 to 20 carbon atoms, for example, a chain alkyl group containing 1 to 12 carbon atoms, such as 1 to 6 carbon atoms. Non-limiting examples of alkyl groups include, but are not limited to, methyl, ethyl, propyl, butyl, etc. The alkyl group may be substituted or unsubstituted, or replaced or unreplaced. For example, when it is substituted, the substituent may be substituted at any available linking site.

In the present disclosure, the term "alkylene" generally refers to a saturated linear or branched aliphatic hydrocarbon group having two residues derived from the parent alkane by removal of two hydrogen atoms from the same carbon atom or two different carbon atoms, which may be a linear or branched group containing 1 to 20 carbon atoms. For example, the term "methylene" may refer to a residue derived from a group containing one carbon atom by removal of two hydrogen atoms. The methylene group may be substituted or unsubstituted, or replaced or unreplaced. For example, an alkylene group containing 1 to 12 carbon atoms, such as 1 to 6 carbon atoms. Non-limiting examples of alkylene groups include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), 1,5-butylene ($-CH_2CH_2CH_2CH_2CH_2-$), etc. The alkylene group may be substituted or unsubstituted, or replaced or unreplaced. For example, when it is substituted, the substituent may be substituted at any available linking site.

In the present disclosure, the term "alkoxy" refers to -O-(alkyl) and -O-(unsubstituted cycloalkyl), wherein the definitions of alkyl or cycloalkyl are as described herein. Non-limiting examples of alkoxy groups include methoxy, ethoxy, propoxy, butoxy, cyclopropoxy, cyclobutoxy, cyclopentoxy, cyclohexoxy.

In the present disclosure, the term "cycloalkyl" refers to a saturated or partially unsaturated monocyclic or polycyclic hydrocarbon substituent containing 3 to 20 carbon atoms, preferably 3 to 12 carbon atoms, preferably 3 to 10 carbon atoms, preferably 3 to 8 carbon atoms, more preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro, fused, and bridged cycloalkyl groups. The cycloalkyl group may be substituted or unsubstituted. For example, when it is substituted, the substituent may be substituted at any available linking site.

In the present disclosure, the term "partially unsaturated" generally refers to a cyclic structure containing at least one double bond or triple bond between ring atoms. The term "partially unsaturated" encompasses cyclic structures with multiple unsaturations, but is not intended to encompass aromatic or heteroaromatic rings as defined in the present disclosure. The term "unsaturated" means that the moiety has one or more degrees of unsaturation.

In the present disclosure, the term "heterocyclyl" refers to a saturated or partially unsaturated monocyclic or polycyclic hydrocarbon substituent containing 3 to 20 ring atoms, wherein one or more of the ring atoms are heteroatoms selected from nitrogen, oxygen or sulfur, and the remaining ring atoms are carbon; preferably comprising 3 to 12 ring atoms, 1 to 4 of which are heteroatoms; more preferably comprising 3 to 8 ring atoms, 1 to 3 of which are heteroatoms; still more preferably comprising 3 to 6 ring atoms, 1 to 3 of which are heteroatoms; most preferably comprising 5 or 6 ring atoms, 1 to 3 of which are heteroatoms. Non-limiting examples of monocyclic heterocyclyl groups include pyrrolidinyl, tetrahydropyranyl, piperidinyl, morpholinyl, thiomorpholinyl, homopiperazinyl, etc. Polycyclic heterocyclyl groups include spiro, fused, and bridged heterocyclyl groups. The heterocyclyl ring may be fused to an aryl, heteroaryl, or cycloalkyl ring, wherein the ring attached to the parent structure is a heterocyclyl ring. The heterocyclyl group may be substituted or unsubstituted. For example, when it is substituted, the substituent may be substituted at any available linking site.

In the present disclosure, the term "ring-forming atom" generally refers to atoms contained in a cyclic structure. For example, the ring-forming atom may be a carbon atom on a benzene ring or a nitrogen atom on a pyridine ring. When a ring-forming atom is attached to a hydrogen atom, the ring-forming atom may be substituted or unsubstituted. For

example, when it is substituted, the substituent may be substituted at any available linking site.

In the present disclosure, the term "each independently" generally refers to a variable applicable to any circumstance, regardless of the presence or absence of variables with identical or different definitions in the same compound. For example, the variables may refer to the type or number of substituents of a compound, or the type of atoms in the compound. For example, when R appears twice in a compound and R is defined as "independently carbon or nitrogen", both R may be carbon, both R may be nitrogen, or one R may be carbon while the other R may be nitrogen.

In the present disclosure, the term "optional" or "optionally" generally means that the subsequently described event or circumstance may but does not necessarily occur, and that the description includes both cases where the event or circumstance does or does not occur. For example, "heterocyclyl optionally substituted by an alkyl group" means that the alkyl group may but does not necessarily have to be present, and that the description includes the case where the heterocyclyl group is substituted by an alkyl group and the case where the heterocyclyl group is not substituted by an alkyl group.

In the present disclosure, the term "substituted" generally means that one or more hydrogen atoms (e.g., up to 5 hydrogen atoms, such as 1 to 3 hydrogen atoms) are independently substituted by a corresponding number of substituents in a group. Substituents are only located at their possible chemical positions, and those skilled in the art are able to determine (experimentally or theoretically) possible or impossible substitutions without undue effort. For example, an amino or hydroxyl group with a free hydrogen may be unstable when bonded to a carbon atom with an unsaturated (e.g., olefinic) bond.

In the present disclosure, unless otherwise specified, the "linking" between groups may generally be in any orientation; the "linking" of group X to group Y may generally be in any orientation, which generally means that when group X serves as a linker between group Y and group Z, two or more linking sites of the group X may be arbitrarily linked to group Y or group Z.

In the present disclosure, as known to those skilled in the art, terms such as "alkyl", "alkenyl", and "cycloalkyl" may be preceded by an identifier indicating the number of atoms present in the group under specific circumstances, for example, $C_1$-$C_4$ alkyl, $C_3$-$C_7$ cycloalkoxy, and $C_1$-$C_4$ alkylcarbonylamino, where the subscript number following "C" indicates the number of carbon atoms present in the group. For example, $C_3$ alkyl refers to an alkyl group having three carbon atoms (e.g., n-propyl, isopropyl); for $C_{1-10}$, the members of the group may have any number of carbon atoms falling within the range of 1 to 10.

In the present disclosure, the cytotoxic drug of the present disclosure may be a tautomer, mesomer, racemate, enantiomer, and/or diastereomer thereof. In the present disclosure, the term "diastereomer" generally refers to a stereoisomer having two or more chiral centers and whose molecules are not mirror images of each other. Diastereomers may exhibit distinct physical properties, such as melting point, boiling point, spectral properties, and reactivity. In the present disclosure, the terms "tautomer" or "tautomeric form" are used interchangeably and generally refer to structural isomers of different energy levels that can be interconverted via a low energy barrier. For example, proton tautomers (also known as prototropic tautomers) include interconversions via proton migration, such as keto-enol isomerization and imine-enamine isomerization. Valence tautomers include interconversions through the reorganization of some bonding electrons. In the present disclosure, the term "mesomer" generally refers to a molecule containing asymmetric atoms, but having symmetric factors that result in a total optical rotation of zero within the molecule. The term "racemate" or "racemic mixture" refers to a composition consisting of two enantiomeric substances in equimolar amounts.

In the present disclosure, the term "linker unit" or "linker structure" generally refers to a chemical structural fragment or bond that is linked to a ligand at one end and to a cytotoxic drug at the other end, and may also be linked to other linkers before being linked to the cytotoxic drug. The direct or indirect linking of a ligand may mean that the group is directly linked to the ligand via a covalent bond, or may be linked to the ligand via a linker structure. For example, a chemical structural fragment or bond comprising an acid-labile linker structure (e.g., hydrazone), a protease-sensitive (e.g., peptidase-sensitive) linker structure, a photolabile linker structure, a dimethyl linker structure, or a disulfide-containing linker structure may be used as a linker structure.

In the present disclosure, the term "optionally linked to other molecular moieties" generally means that the structure is not linked to any other chemical structure, or that the structure is linked (e.g., via a chemical bond or a linker structure) to one or more other chemical structures (e.g., ligands described in the present disclosure) different from the structure.

In the present disclosure, the term "drug loading" generally refers to the average amount of cytotoxic drug loaded per ligand, which may also be expressed as the ratio of the amount of cytotoxic drug to the amount of antibody. The cytotoxic drug loading may range from 0 to 12 (e.g., 1 to 10) cytotoxic drugs per ligand (Ab). In an embodiment of the present disclosure, the drug loading is denoted as p, t, or n, and exemplary values may be an average of 1, 2, 3, 4, 5, 6, 7, 8, 9, or 10. The drug loading per ADC molecule after the coupling reaction may be characterized by conventional methods such as UV/visible spectroscopy, mass spectrometry, ELISA, and HPLC.

In the present disclosure, certain atoms of the compounds or antibody-drug conjugates of the present disclosure may be present in more than one isotopic form. For example, hydrogen may occur as protium ($^1H$), deuterium ($^2H$), and

tritium ($^3H$), while carbon may naturally occur as three different isotopes ($^{12}C$, $^{13}C$, and $^{14}C$). Examples of isotopes that may be incorporated into the compounds of the present disclosure also include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Therefore, relative to the natural abundance of these isotopes, the compounds or antibody-drug conjugates of the present disclosure may be enriched in one or more of these isotopes. As known to those skilled in the art, such isotopically enriched compounds may be used for a variety of purposes. For example, substitution with heavy isotopes such as deuterium ($^2H$) may provide certain therapeutic advantages, possibly due to higher metabolic stability. For example, the natural abundance of deuterium ($^2H$) is approximately 0.015%. Accordingly, one out of approximately 6,500 hydrogen atoms in nature is a deuterium atom. Therefore, the deuterium-containing compound or antibody-drug conjugate of the present disclosure has a deuterium abundance of greater than 0.015% at one or more sites, as the case may be. Unless otherwise indicated, the structures described in the present disclosure may also include compounds or antibody-drug conjugates that differ only in the presence or absence of one or more isotopically enriched atoms. For example, except for the substitution of hydrogen atoms with deuterium or tritium or the substitution of carbon atoms with carbon-13 or carbon-14, compounds or antibody-drug conjugates whose structures are consistent with the present disclosure are within the scope of the present disclosure.

In the present disclosure, the term "pharmaceutical composition" generally refers to a mixture containing one or more of the compounds described in the present disclosure or a physiologically/pharmaceutically acceptable salt or prodrug thereof, with other chemical components, as well as additional components such as physiologically/pharmaceutically acceptable carriers and excipients. The pharmaceutical composition may facilitate administration to an organism, promote absorption of active ingredients, and thus exert biological activity. The preparation of conventional pharmaceutical compositions can be found in the Chinese Pharmacopoeia. The pharmaceutical composition may be in the form of a sterile injectable aqueous or oily suspension for intramuscular and subcutaneous administration. The suspension may be formulated according to known techniques using suitable dispersing or wetting agents and suspending agents as described above. The sterile injectable preparation may also be a sterile injectable solution or suspension prepared in a parenterally acceptable non-toxic diluent or solvent, such as a solution prepared in 1,3-butanediol. In addition, a sterile fixed oil may be conveniently used as a solvent or suspending medium. For example, any fixed oil including synthetic mono- or diglycerides may be used. In addition, fatty acids such as oleic acid may also be used in the preparation of injections.

In the present disclosure, the term "pharmaceutically acceptable salt" or "medicinal salt" generally refers to a salt of the compound or antibody-drug conjugate of the present disclosure, or a salt of the compound or antibody-drug conjugate described in the present disclosure. Such salts may be safe and/or effective when used in mammals and may have the desired biological activity. The compound or antibody-drug conjugate of the present disclosure may form a salt with an acid. Non-limiting examples of pharmaceutically acceptable salts include hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

In the present disclosure, the term "pharmaceutically acceptable carrier" generally refers to a carrier or vehicle for administering therapeutic agents, such as antibodies or polypeptides, genes, and other therapeutic agents. The term refers to any pharmaceutical carrier that does not itself induce the production of antibodies harmful to the individual receiving the composition and that may be administered without undue toxicity. Suitable carriers may be large, slowly metabolized macromolecules, such as proteins, polysaccharides, polylactic acids, polyglycolic acids, polyamino acids, amino acid copolymers, lipid aggregates, and inactivated viral particles. Such carriers are well known to those skilled in the art. Pharmaceutically acceptable carriers in therapeutic compositions may include liquids, such as water, saline, glycerol, and ethanol. Auxiliary substances, such as wetting or emulsifying agents, or pH buffering substances, may also be present in these carriers.

In the present disclosure, the terms "treatment" and "treating" generally refer to a method for achieving beneficial or desired results including, but not limited to, therapeutic benefits. Therapeutic benefits include, but are not limited to, eradication, inhibition, reduction, or amelioration of the underlying disorder being treated. In addition, therapeutic benefits are achieved by eradicating, inhibiting, reducing, or ameliorating one or more physiological symptoms associated with the underlying disorder, such that improvements are observed in the patient, but the patient may still suffer from the underlying disorder.

In the present disclosure, the terms "prevention" and "preventing" generally refer to a method for achieving beneficial or desired results including, but not limited to, prophylactic benefits. For the purpose of prophylactic benefits, a pharmaceutical composition may be administered to a patient at risk of developing a particular disease, or to a patient who reports to have one or more physiological symptoms of a disease, even if the disease has not yet been diagnosed.

In the present disclosure, the term "subject" or "patient" generally refers to a human (i.e., a male or female of any age group, for example, a pediatric subject (e.g., an infant, a child, or an adolescent) or an adult subject (e.g., a young adult, a middle-aged adult, or an elderly adult) and/or other primates (e.g., a cynomolgus monkey or a rhesus monkeys); a mammal, including commercially relevant mammals such as cattle, pigs, horses, sheep, goats, cats, and/or dogs; and/or a

poultry, including commercially relevant poultries such as chickens, ducks, geese, quails, and/or turkeys.

In the present disclosure, the terms "therapeutically effective amount", "therapeutically effective dose", and "effective amount" refer to an amount of the compound or antibody-drug conjugate of the present disclosure that is effective in preventing or ameliorating one or more symptoms of a disease or condition or the progression of the disease or condition, when administered alone or in combination with other therapeutic drugs to a cell, tissue, or subject. The therapeutically effective dose also refers to a dose sufficient to cause an improvement in symptoms, such as an amount for treating, curing, preventing, or ameliorating a relevant medical condition or promoting the treatment, cure, prevention, or amelioration of such condition. When an active ingredient is administered alone to a subject, the therapeutically effective dose refers solely to the amount of the ingredient. In the case of administration in combination, the therapeutically effective dose refers to the combined amount of active ingredients that produce a therapeutic effect, regardless of whether these active ingredients are administered in combination, sequentially, or simultaneously. An effective amount of a therapeutic agent will result in an increase in diagnostic criteria or parameters by at least 10%, generally at least 20%, preferably at least about 30%, more preferably at least 40%, and most preferably at least 50%.

In the present disclosure, the term "cancer" refers to a group of cells that exhibit abnormally high levels of proliferation and growth. Cancer may be benign (also known as a benign tumor), premalignant, or malignant. Cancer cells may be solid tumor cells or leukemic cancer cells. In the present disclosure, the term "tumor" refers to one or more cells comprising cancer. In the present disclosure, the term "tumor growth" refers to the proliferation or growth of one or more cells comprising cancer, which results in a corresponding increase in the size or extent of the cancer.

On the basis of common knowledge in the art, the above preferred conditions can be arbitrarily combined to obtain various preferred examples of the present disclosure.

The reagents and starting materials used in the present disclosure are commercially available.

The positive and progressive effects of the present disclosure are as follows:

In the bispecific antibody-drug conjugate according to the present disclosure, the bispecific antibody has the following advantages:

1. The bispecific antibody of the present disclosure is capable of simultaneously binding to B7H3 and PD-L1, targeting tumor cells while relieving PD-L1-mediated inhibition of T cells, and exhibits superior anti-tumor activity compared to monoclonal antibody combination therapy.
2. Compared to related monoclonal antibody combination therapy, the bispecific antibody of the present disclosure has the advantages of good compliance and controllable quality.
3. The stability characterization of the bispecific antibody in the present disclosure is mainly reflected in the study of monomer purity and thermal stability. After a single affinity purification, the monomer content of the bispecific antibody may be up to 95%, even surpassing the purity achieved through multiple secondary purifications in the industry. The results of structure and activity analysis of the antibody after heat treatment demonstrate that the antibody can still maintain favorable molecular conformation and intact biological activity under harsh conditions, which is beneficial for industrial production, packaging, and storage of the antibody. In general, the present disclosure constructs a B7H3/PD-L1 bispecific antibody in the form of IgG-VHH2, which demonstrates favorable molecular stability and exhibits significantly superior *in vitro* activity (at both molecular binding and cellular levels) compared to Avelumab and MGA271. *In vivo* data demonstrate that the bispecific antibody exhibits superior anti-tumor activity compared to the B7H3 monoclonal antibody combination therapy in $B7H3^+$ A375 tumor cells. Therefore, the bispecific antibody of the present disclosure has broad application prospects due to its excellent developability and activity.

The bispecific antibody-drug conjugate of the present disclosure has one or more of the following advantages:

1. Compared to related monoclonal antibody-drug conjugates, the bispecific antibody-drug conjugate of the present disclosure exhibits significantly enhanced anti-proliferative activity against tumor cells *in vitro,* particularly against B7H3- and PD-L1-positive A375, NCI-H1975, NCI-H441, and NCI-H358 cells.
2. Compared to related monoclonal antibody-drug conjugates, the bispecific antibody-drug conjugate of the present disclosure can more significantly induce downregulation of PD-L1 expression, indicating that the bispecific antibody-drug conjugate of the present disclosure can exert stronger immune suppression relief by reducing target expression levels.
3. Compared to related monoclonal antibody-drug conjugates, the bispecific antibody-drug conjugate of the present disclosure exhibits significantly enhanced *in vivo* tumor growth inhibiting activity, such as against CT26 cells, particularly a CT26-implanted syngeneic mouse model.
3. Compared to related monoclonal antibody-drug conjugates, the bispecific antibody-drug conjugate of the present disclosure can significantly inhibit the growth of melanoma, esophageal squamous cell carcinoma, small cell lung cancer, liver cancer, breast cancer, or non-small cell lung cancer, particularly against A375 melanoma cells, KYSE-150 esophageal squamous cell carcinoma cells, NCI-H292 small cell lung cancer cells, Huh7 liver cancer

cells, MDA-MB-231 breast cancer cells, or NCI-H1975 non-small cell lung cancer cells.

4. Compared to related monoclonal antibody-drug conjugates and monoclonal antibody combination therapy, the bispecific antibody-drug conjugate of the present disclosure can significantly inhibit the growth of melanoma, esophageal squamous cell carcinoma, small cell lung cancer, liver cancer, breast cancer, or non-small cell lung cancer, particularly against MDA-MB-231 breast cancer cells or NCI-H1975 non-small cell lung cancer cells.

5. Compared to related monoclonal antibody-drug conjugates, the bispecific antibody-drug conjugate of the present disclosure exhibits a significantly enhanced endocytosis effect.

6. The bispecific antibody-drug conjugate of the present disclosure has a favorable safety profile.

The bispecific antibody-drug conjugate according to any one of the embodiments of the present disclosure may have one or more effects selected from the group consisting of (1) inhibitory activity against the proliferation of tumor cells *in vitro;* (2) targeted inhibitory effect; (3) plasma stability; (4) *in vivo* tumor suppression effect; (5) bystander killing effect; (6) antitransporter transport capability; (7) *in vivo* tumor-targeting ability; (8) enhanced tumor suppression effect in an individual with a robust immune system; and (9) favorable *in vivo* safety profile.

BRIEF DESCRIPTION OF THE DRAWINGS

FIG. 1 shows the affinity screening of the anti-B7H3 VHH humanized antibody prepared by the present disclosure against B7H3.

FIG. 2 shows a schematic diagram of the structure of the bispecific antibody DSYE001 in the bispecific antibody-drug conjugate DSYE001-X1 or DSYE001-X2 according to the present disclosure.

FIG. 3 shows the SDS-polyacrylamide gel electrophoresis of the anti-B7H3 and PD-L1 bispecific antibody DSYE001 according to the present disclosure.

FIG. 4 shows the SEC-HPLC purity determination of the anti-B7H3 and PD-L1 bispecific antibody DSYE001 according to the present disclosure.

FIG. 5 shows the Tm value determination (DSF) of the anti-B7H3 and PD-L1 bispecific antibody DSYE001 according to the present disclosure.

FIG. 6 shows the binding ELISA of the anti-B7H3 and PD-L1 bispecific antibody DSYE001 according to the present disclosure before and after heat treatment at 60°C, wherein a represents the binding ELISA with PD-L1, and b represents the binding ELISA with B7H3.

FIG. 7 shows the binding ELISA of the anti-B7H3 and PD-L1 bispecific antibody DSYE001 according to the present disclosure, wherein a represents the binding ELISA with PD-L1, and b represents the binding ELISA with B7H3.

FIG. 8 shows the affinity of the anti-B7H3 and PD-L1 bispecific antibody DSYE001 according to the present disclosure against B7H3-his or PD-L1-his at 5 different concentrations as determined by BLI, where a represents the affinity curve with PD-L1, and b represents the affinity curve with B7H3.

FIG. 9 shows the PD-L1/CHO-PD1 blocking curve of the anti-B7H3 and PD-L1 bispecific antibody DSYE001 according to the present disclosure.

FIG. 10 shows the induction of IFN-$\gamma$ secretion by T cells mediated by the anti-B7H3 and PD-L1 bispecific antibody DSYE001 according to the present disclosure.

FIG. 11 shows the promotion of T cell proliferation by the anti-B7H3 and PD-L1 bispecific antibody DSYE001 according to the present disclosure.

FIG. 12 shows the ADCC effect of the anti-B7H3 and PD-L1 bispecific antibody DSYE001 according to the present disclosure on cancer cells, wherein a represents the cytotoxic effect on human breast cancer cells MDA-MB-231, and b represents the cytotoxic effect on human ovarian clear cell carcinoma cells ES-2.

FIG. 13 shows the *in vivo* tumor suppression effect of the anti-B7H3 and PD-L1 bispecific antibody DSYE001 according to the present disclosure.

FIG. 14 shows the effect of the anti-B7H3 and PD-L1 bispecific antibody DSYE001 according to the present disclosure on the body weight of mice.

FIG. 15 shows the test results of endocytosis activity of the bispecific antibody-drug conjugate DSYE001-X2 according to the present disclosure.

FIG. 16 shows the *in vitro* proliferation inhibition assay of the bispecific antibody-drug conjugate DSYE001-X1 (DAR8) according to the present disclosure on tumor cells.

FIG. 17 shows the effect of the bispecific antibody-drug conjugate DSYE001-X1 (DAR6) according to the present disclosure on PD-L1 expression in NCI-H1975 cells.

FIG. 18 shows an evaluation of the efficacy of the bispecific antibody-drug conjugate DSYE001-X2 according to the present disclosure in a syngeneic mouse model.

FIG. 19 shows an evaluation of the efficacy of the bispecific antibody-drug conjugate DSYE001-X1 (DAR4) according to the present disclosure in NCI-H1975 human non-small cell lung cancer xenograft mice.

FIG. 20 shows an evaluation of the efficacy of the bispecific antibody-drug conjugate DSYE001-X1 (DAR6) according to the present disclosure in MDA-MB-231 human breast cancer xenograft mice.

FIG. 21 shows an evaluation of the efficacy of the bispecific antibody-drug conjugate DSYE001-X1 (DAR6) according to the present disclosure in NCI-H1975 human non-small cell lung cancer xenograft mice.

DETAILED DESCRIPTION OF THE PREFERRED EMBODIMENT

The present disclosure is further described below by way of examples, but the present disclosure is not thereby limited to the scope of the described examples. Experimental methods for which specific conditions are not indicated in the following examples are selected according to conventional methods and conditions, or according to the commodity instructions.

Experimental methods for which specific conditions are not indicated in the following examples are selected according to conventional methods and conditions, or according to the commodity instructions.

**Sample detection**

**1. ADC DAR value analysis method - HIC-HPLC (hydrophobic interaction chromatography)**

High performance liquid chromatograph: Waters e2965 high performance liquid chromatography system.

Chromatographic column: MabPac™ HIC-Butyl 5 $\mu$m 4.6 $\times$ 100 mm (manufacturer: Thermo).

Mobile phase A: 1.5 M $(NH_4)_2SO_4$ + 50 mM $K_2HPO_4$ (pH 7.0).

Mobile phase B: 50 mM $K_2HPO_4$ (pH 7.0) / isopropanol (75:25 V/V).

Elution was carried out according to the following elution procedure:

| Time | Mobile phase B |
|---|---|
| 0-2 min | 0%-10% |
| 2-22 min | 10%-65% |
| 22-24 min | 65%-100% |
| 24-26 min | 100%-0% |
| 26-30 min | 0% |

Detection conditions: The flow rate of the mobile phase was set to 1 mL/min, the detection wavelength was set to 280 nm, and the column temperature was set to 30°C.

**2. SEC purity analysis - SEC-HPLC (size exclusion chromatography)**

High performance liquid chromatograph: 1260 Agilent liquid chromatograph.

Chromatographic column: Waters Xbridge BEH200 SEC (7.8 $\times$ 300 mm, 3.5 $\mu$m)

Mobile phase: 50 mM $NaH_2PO_4$ + 200 mM arginine (pH 6.80) + 10% isopropanol

| Time | Mobile phase |
|---|---|
| 0-30 min | 100% |

Detection conditions: The flow rate of the mobile phase was set to 0.5 mL/min, the detection wavelength was set to 280 nm, and the column temperature was set to 30°C.

The present disclosure includes all combinations of the specific embodiments described herein. The full scope of further embodiments and their applicability of the present disclosure will become apparent from the detailed description provided below. However, it should be understood that, while the detailed description and specific examples indicate preferred embodiments of the present disclosure, these descriptions and examples are provided for illustrative purposes only, as various changes and modifications within the spirit and scope of the present disclosure will become apparent to those skilled in the art from the detailed description. For all purposes, all publications, patents, and patent applications

referenced herein, including citations, are incorporated herein by reference in their entirety.

**Examples**

The following examples are provided to demonstrate and further explain some of the preferred embodiments and aspects of the present disclosure and should not be construed as limiting its scope.

**Example 1: Preparation and testing of bispecific antibody DSYE001**

In the anti-B7H3 and PD-L1 bispecific antibody-drug conjugate of the present disclosure, the anti-B7H3 and PD-L1 bispecific antibody or an antigen-binding fragment thereof was prepared with reference to the method described in PCT/CN2022/125089, as specifically shown below (the CDR region of the bispecific antibody was determined according to the Kabat numbering scheme):

1. Humanization of camelid anti-B7H3

The camelid anti-B7H3 nanobody was humanized using germline genes of human antibodies as a template by framework shuffling. The corresponding VHH frameworkshuffled libraries were generated entirely *in vitro* by overlap PCR. Subsequently, the VHH phage library was constructed, followed by cloning, screening, and identification.

More specifically, a one-step strategy was employed to humanize the camelid anti-B7H3 nanobody. Approximately 1,000 clones were screened from the sub-library, and the selected positive clones were screened for thermal stability at the phage level by ELISA. 5 μg/mL huB7H3 antigen was coated in a high-binding 96-well ELISA plate. The supernatant from the overnight-amplified phages was reacted, and clones displaying high OD450 readings were selected.

The phage clones were sequenced to obtain the B7H3 VHH gene sequence, which was then fused at the C-terminus with the human Fc protein gene to construct B7H3-Fc for expression. Bio-layer interferometry was performed using a Protein A probe to capture 100 nM B7H3 VHH-Fc, which was then bound to B7H3 antigen starting at a concentration of 200 nM with a 2-fold serial dilution. The KD value for antibody-antigen binding was calculated. The results demonstrated that 75-16 (with the amino acid sequence of SEQ ID NO.: 15, CDR1 sequence of SEQ ID NO.: 12, CDR2 sequence of SEQ ID NO.: 13, and CDR3 sequence of SEQ ID NO.: 14) exhibited the highest affinity for B7H3, achieving a KD of $3.24 \times 10^{-9}$ M (as shown in FIG. 1). Consequently, it was selected for the subsequent construction of the bispecific antibody.

2. Construction and expression of anti-B7H3 and PD-L1 bispecific antibodies

The amino acid sequences of the light chain (with the amino acid sequence of SEQ ID NO.: 9, variable region sequence of SEQ ID NO.: 4, CDR1 sequence of SEQ ID NO.: 1, CDR2 sequence of SEQ ID NO.: 2, and CDR3 sequence of SEQ ID NO.: 3) and the heavy chain (with the amino acid sequence of SEQ ID NO.: 10, variable region sequence of SEQ ID NO.: 8, CDR1 sequence of SEQ ID NO.: 5, CDR2 sequence of SEQ ID NO.: 6, and CDR3 sequence of SEQ ID NO.: 7) of the PD-L1 monoclonal antibody were derived from an existing PD-L1 hIgG1 humanized monoclonal antibody. The N-terminus of the anti-B7H3 VHH (75-16 as described above) was connected to the C-terminus of the Fc fragment (with the structure shown in FIG. 2) via a linker peptide (SEQ ID NO.: 11).

The DNA sequences were synthesized and subcloned into the pcDNA3.1 vector, followed by amplification in *E. coli.* The purified plasmid was transfected into HEK293 cells using PEI. The cells were then suspended in OPM-CD05 expression medium for incubation. After 6 days of incubation, the cell culture supernatant was collected, and the antibody was purified by Protein A column. The purified IgG1 was dialyzed in phosphate-buffered saline (PBS), rapidly frozen, and stored at -80°C.

For the purified anti-B7H3 and PD-L1 bispecific antibody DSYE001, the heavy chain amino acid sequence is as shown in SEQ ID NO.: 16, and the light chain amino acid sequence is as shown in SEQ ID NO.: 9.

Test section

Hereinafter, unless otherwise specified, the term "bispecific antibody" refers to the anti-B7H3 and PD-L1 bispecific antibody of the present disclosure, which is also referred to as "B7H3/PD-L1 bispecific antibody", "B7H3/PD-L1 BsAb", or "BsAb".

Test methods:

(1) Sodium dodecyl sulfate-polyacrylamide gel electrophoresis (SDS-PAGE)

5 μg of the bispecific antibody was mixed with protein loading buffer under reducing and non-reducing conditions, and the system was supplemented with PBS to a final volume of 10 μL. The mixture was heated at 100°C for 10 minutes to fully denature the proteins, and 9 μL of the sample was loaded into the wells of a precast polyacrylamide gel (Bio-Rad). Electrophoresis was performed initially at 80 V for 30 minutes, followed by 120 V for 60 minutes for separation. After electrophoresis, the gel was stained with Coomassie Brilliant Blue staining solution for 30 minutes, and destained with a destaining solution (acetic acid: ethanol: water = 1:3:6) for 15 minutes. The destaining step was repeated three times until the background was clear. The image was then captured using a gel imaging system. The results demonstrated that under non-reducing conditions, the B7H3/PD-L1 bispecific antibody exhibited good monomer purity, while under reducing conditions, two bands corresponding to the heavy and light chains were observed due to the cleavage of disulfide bonds between the heavy and light chains, with no impurity bands observed (as shown in FIG. 3).

(2) Size exclusion chromatography (SEC-HPLC)

SEC-HPLC analysis was used to assess the monomer purity of the bispecific antibody. The B7H3/PD-L1 bispecific antibody, along with PD-L1 monoclonal antibody and B7H3 VHH-Fc, was analyzed on a Thermo MAbPac SEC-1 column (5 μm, 7.8 × 300 mm, P/N 088460) using a 1260 HPLC system (Agilent, Santa Clara, CA). The mobile phase used was phosphate-buffered saline (PBS). The flow rate was set to 0.7 mL/min with an injection volume of 15 μL. SEC chromatograms were recorded by monitoring the absorbance at 280 nm using a UV detector at a constant temperature of 25°C. As shown in FIG. 4. All detected antibodies exhibited an extremely high proportion of monomer peaks, with the monomer peak area accounting for 95% or more, which indicates that the bispecific antibody maintains good monomer purity with minimal aggregate formation in PBS.

(3) Differential scanning fluorimetry (DSF) for Tm value determination of antibodies

DSF was performed using a real-time PCR instrument (Biorad cfx96, USA). The B7H3/PD-L1 bispecific antibody, B7H3 VHH-Fc, and PD-L1 mAb were diluted to 1 mg/mL in PBS. SYPRO Orange was diluted 1000-fold from a 5000× concentrate stock solution with $ddH_2O$, and 20 μL of the sample was loaded into a PCR tube. The working solution of SYPRO Orange was added to the reaction to prevent bleaching, followed by a brief centrifugation to pool the liquid at the bottom of the PCR tube. The qPCR instrument was programmed to ramp from 25°C to 95°C at a rate of 0.3°C per second. Data were collected, and temperature versus signal values were plotted to calculate the melting temperature (Tm). The results demonstrated that the B7H3/PD-L1 bispecific antibody exhibited excellent thermal stability, with Tm values of 69°C and 90°C for the Fc and Fab regions, respectively, which were comparable to those of the B7H3 VHH-Fc and PD-L1 mAb (as shown in FIG. 5).

(4) Binding ELISA for validation of thermal stability and binding activity of antibodies

A 96-well ELISA plate was coated with 2 μg/mL His-tagged PD-L1 or B7H3 antigen at 4°C overnight. The following day, each well was blocked with 100 μL of Casein blocking buffer at 37°C for 1 hour. The B7H3/PD-L1 bispecific antibody (for testing thermal stability, the bispecific antibody was incubated in a water bath at 60°C for 1 hour) and monoclonal antibodies (for testing binding of the bispecific antibody to B7H3 by ELISA, the control monoclonal antibodies used were MGA271, Isotype hIgG1, and B7H3 VHH-Fc; for testing binding to PD-L1, the control monoclonal antibodies were PD-L1 mAb, Avelumab, and Isotype hIgG1) were 3-fold serially diluted and added to the plate. After incubation at 37°C for 1 hour, the unbound antibodies were washed away with 0.1% PBST, and the bounded antibodies were detected using horseradish peroxidase (HRP)-conjugated goat anti-human IgG (H + L) antibody (Jackson ImmunoResearch, USA). The reaction was developed with 50 μL of 3,3',5,5'-tetramethylbenzidine (TMB) substrate for 5 minutes and stopped with 50 μL of 2 M sulfuric acid. The absorbance at 450 nm was then measured using a SpectraMax M5e microplate reader (Molecular Devices). Antibody concentration was plotted against OD450 readings using a four-parameter logistic fit, and the $EC_{50}$ was calculated. a and b of FIG. 6 show the binding curves of the bispecific antibody to PD-L1 and B7H3 before and after treatment at 60°C for 1 hour. As can be seen from the figure, whether for PD-L1 or B7H3, the binding curves of the bispecific antibody before and after heating substantially overlapped, indicating that the bispecific antibody was able to withstand high temperatures of 60°C while maintaining strong binding activity to both PD-L1 and B7H3 targets. On the other hand, the bispecific antibody exhibited similar binding potency to both targets as the positive antibody, PD-L1 mAb, and B7H3 VHH-Fc. The $EC_{50}$ values for the bispecific antibody, PD-L1 mAb, and control Avelumab binding to human PD-L1 were 0.3056 nM, 0.4407 nM, and 0.1563 nM, respectively, falling within the same order of magnitude (as shown in a of FIG. 7). Similarly, the bispecific antibody, B7H3 VHH-Fc, and MGA271 exhibited similar binding activity to human B7H3, with $EC_{50}$ values of 0.04105 nM, 0.02515 nM, and 0.05476 nM, respectively (as shown in b of FIG. 7).

(5) Determination of bispecific antibody-antigen binding affinity by BLI

The B7H3/PD-L1 bispecific antibody, PD-L1 mAb, and B7H3 VHH-Fc were diluted to 100 nM in sample buffer (0.02% Tween 20 and 0.1% BSA in PBS). The binding affinity of the bispecific antibody to human B7H3 and human PD-L1 antigens was analyzed using OCTET 96, with a Protein A probe used to immobilize the antibodies. B7H3-His and PD-L1-His antigens were diluted to an initial concentration of 200 nM in sample buffer, followed by a 2-fold serial dilution to establish multiple antigen gradients for antibody binding to determine rate constants and affinity. The Kon and Koff values were calculated using the software provided by the manufacturer, and the KD values of the antibodies were subsequently derived. As can be seen from the figure, the bispecific antibody exhibited high affinity for both PD-L1 and B7H3, with KD values of $5.85 \times 10^{-10}$ M and $8.11 \times 10^{-9}$ M, respectively (as shown in FIG. 8).

(6) Flow cytometry analysis of PD1/PD-L1 pathway blockade by bispecific antibody

The ability of the B7H3/PD-L1 bispecific antibody to block the binding of human PD-L1 to human PD1-CHO cells was assessed by flow cytometry, followed by comparison with Avelumab and PD-L1 mAb. $2 \times 10^5$ human PD1-CHO cells were evenly seeded in a 96-well culture plate and incubated at room temperature for 30 minutes with a mixture of serially diluted antibodies (starting from 400 nM) (B7H3/PD-L1 bispecific antibody, Avelumab, PD-L1 mAb, Isotype hIgG1, and B7H3 VHH-Fc) and biotinylated PD-L1 antigen (50 nM). The mixture was then incubated with cells at 4°C for 45 minutes, followed by washing with PBS to remove unbound antigen. The cells were then fluorescently stained with PE-streptavidin. Finally, the mean fluorescence intensity (MFI) in the PE channel was measured using a flow cytometer. Antibody concentration was plotted against MFI, and the $IC_{50}$ of the antibody was calculated using a four-parameter logistic fit. The results of flow cytometry analysis demonstrated that the bispecific antibody was able to block the binding of PD-L1 to CHO-PD1 cells, with an $IC_{50}$ of 106.4 nM, comparable to PD-L1 mAb ($IC_{50}$ of 94.20 nM) and Avelumab ($IC_{50}$ of 115.0 nM) (as shown in FIG. 9).

(7) Mixed lymphocyte reaction (MLR) to assess T cell activation by bispecific antibody

Monocytes isolated from peripheral blood mononuclear cells (PBMCs) were cultured *in vitro* for 7 days using a monocyte isolation kit (Miltenyi Biotec, Germany) with 500 U/mL interleukin-4 (IL-4) and 250 U/mL GM-CSF to induce the generation of dendritic cells (DCs). $CD4^+$ T cells ($1 \times 10^5$) and allogeneic DCs ($1.25 \times 10^4$) were co-cultured in RPMI 1640 complete medium containing 10% FBS at a constant temperature of 37°C under 5% $CO_2$. Experimental groups included no antibody control, as well as groups of varying concentrations of B7H3/PD-L1 bispecific antibody, PD-L1 mAb, Avelumab, B7H3 VHH-Fc, MGA271, and Isotype hIgG1. After 5 days, the concentration of IFN-γ in the culture supernatant was analyzed using an IFN-γ ELISA kit. The results of MLR demonstrated that the bispecific antibody stimulated IFN-γ secretion by $CD4^+$ T cells, outperforming PD-L1 mAb and Avelumab at both low and high concentrations in terms of T cell activation capability. Additionally, in the presence of the B7H3 antibody alone, although not as pronounced as with the PD-L1 signal-blocking antibody, the bispecific antibody partially activated T cells by blocking B7H3-mediated inhibitory signaling (as shown in FIG. 10).

(8) T cell proliferation assay

A 96-well cell culture plate (Corning, USA) was coated with 1 μg/mL CD3 antibody (Clone HIT3a), 1 μg/mL CD28 antibody (Clone CD28.2), and 5 μg/mL human PD-L1 at 4°C for 1 hour. Control wells were coated with mouse IgG2a isotype control alone or with CD3 and CD28 antibodies using the same method. $CD4^+$ T cells were isolated using a Dynabeads™ CD4 Positive Isolation Kit. $CD4^+$ T cells were cultured in a pre-coated 96-well plate with varying concentrations of B7H3/PD-L1 bispecific antibody, Avelumab, and PD-L1 mAb in RPMI 1640 medium containing 10% FBS (Gibco) at 37°C for 4 days. After 4 days, the changes in the number of T cells were detected using a CCK8 kit. The data indicated that freshly isolated human $CD4^+$ T cells cultured in the plate coated with anti-CD3 and anti-CD28 antibodies exhibited increased proliferation, and the proliferative capacity was significantly decreased when PD-L1 was added to the plate, confirming that PD-L1 provided inhibitory signals to T cells. Avelumab, PD-L1 mAb, and bispecific antibody significantly promoted T cell proliferation at concentrations of 100 nM and 500 nM (as shown in FIG. 11).

(9) Antibody-dependent cellular cytotoxicity (ADCC)

The primary anti-tumor effects of MGA271 and Avelumab originate from the ADCC function of the antibodies, which is associated with their IgG1 subtype. The bispecific antibody also possesses an IgG1 domain. The ADCC of the antibodies was measured using an LDH cytotoxicity assay kit. Human PBMCs were purified from leukocytes by Ficoll gradient centrifugation, and NK cells were isolated from human PBMCs using negative selection magnetic beads (Miltenyi

Biotec, Auburn, CA). NK cells ($3 \times 10^6$) and MDA-MB-231 or ES-2 cells ($3 \times 10^5$) were co-cultured with or without varying concentrations of bispecific antibody and Avelumab at the initiation of the assay. After 18 hours, the secretion of lactate dehydrogenase (LDH) in the culture supernatant was analyzed by ELISA. When B7H3$^+$ PD-L1$^+$ MDA-MB-231 cells were used as target cells, both the bispecific antibody and Avelumab demonstrated ADCC activity, but the bispecific antibody exhibited stronger activity at low concentrations. When PD-L1$^+$ ES-2 cells were used as target cells, it was observed that the bispecific antibody demonstrated comparable activity to Avelumab at a dose of 100 nM and also exhibited strong ADCC activity at low concentrations (as shown in FIG. 12).

(10) Study on *in vivo* anti-tumor activity of bispecific antibody

Human PBMCs ($6.67 \times 10^6$) were injected into 41 NPSG mice via the tail vein one day before inoculation with A375 tumor cells. On the following day, the mice were subcutaneously injected with $5 \times 10^6$ A375 tumor cells. Subcutaneous tumors were observed five days after tumor inoculation. The mice were divided into groups of 10 animals each, including an isotype control group, monoclonal antibody combination therapy group (Pembrolizumab + MGA271 and Avelumab + MGA271), and a bispecific antibody group. Administration was initiated on day 5 after successful modeling, with dosing performed twice weekly until the end of the experiment. Tumor volume and animal body weight were measured and recorded on days 0, 4, 7, 11, 14, 18, 21, 25, 28, 32, 35, 39, and 42 of the experiment. Efficacy and safety were evaluated based on the tumor growth inhibition (TGI) determined by relative tumor volume (RTV) and changes in animal body weight. The bispecific antibody consistently exhibited significantly stronger activity than the PD1 mAb + MGA271 combination therapy group. As the experiment progressed, the bispecific antibody gradually demonstrated superior efficacy compared to the Avelumab + MGA271 group, with TGI values of 39.29% and 26.45%, respectively, at the end of the experiment (as shown in FIG. 13). Additionally, no significant body weight loss was observed in mice during the experiment, confirming the safety of the bispecific antibody treatment (as shown in FIG. 14).

As can be seen from the test results described in (1) to (10), the bispecific antibody constructed by the present disclosure was capable of simultaneously binding to B7H3 and PD-L1, targeting tumor cells while relieving PD-L1-mediated inhibition of T cells, and exhibited superior anti-tumor activity compared to monoclonal antibody combination therapy.

The anti-B7H3 antibody DSYE002 served as a reference antibody for the bispecific antibody DSYE001 in which the PD-L1 antibody was removed; the anti-B7H3 antibody DSYE003 served as a reference antibody for the bispecific antibody DSYE001 in which the PD-L1 antibody was replaced (the variable regions of the PD-L1 antibody in the bispecific antibody were replaced with Human Anti-HIV-1 gp120 clone b12 VH and VL); the anti-PD-L1 antibody DSYE004 served as a reference antibody for the PD-L1 antibody in the bispecific antibody DSYE001.

Antibodies DSYE002, DSYE003, and DSYE004 were prepared using conventional methods. For example, vector construction was performed, followed by transfection into eukaryotic cells, such as HEK293 cells or CHO cells, for purification and expression.

The amino acid sequences of the anti-B7H3 and PD-L1 bispecific antibody DSYE001, the anti-B7H3 antibodies DSYE002 and DSYE003, and the anti-PD-L1 antibody DSYE004 are shown in the sequence listing.

**Example 2: Preparation of bispecific antibody-drug conjugate (ADC)**

**2.1. Preparation of linker-payload**

**Linker-payload X1:**

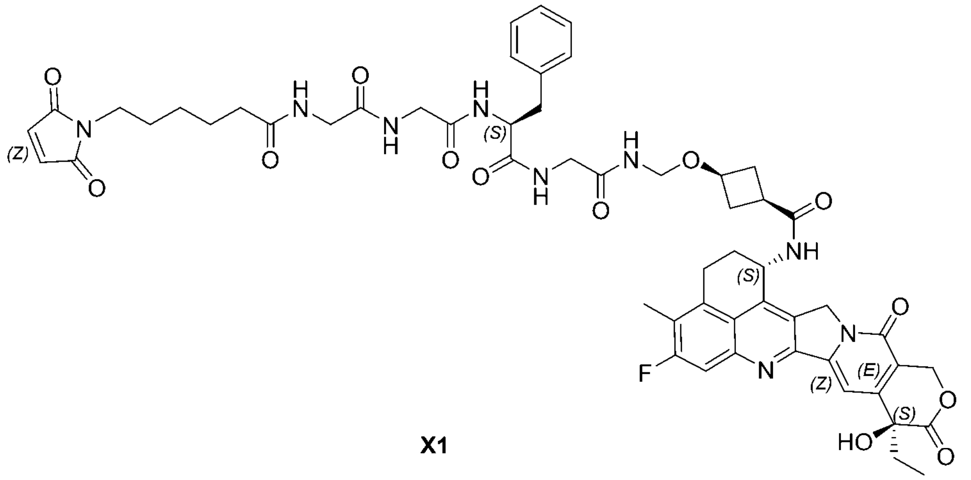

X1

Synthetic route:

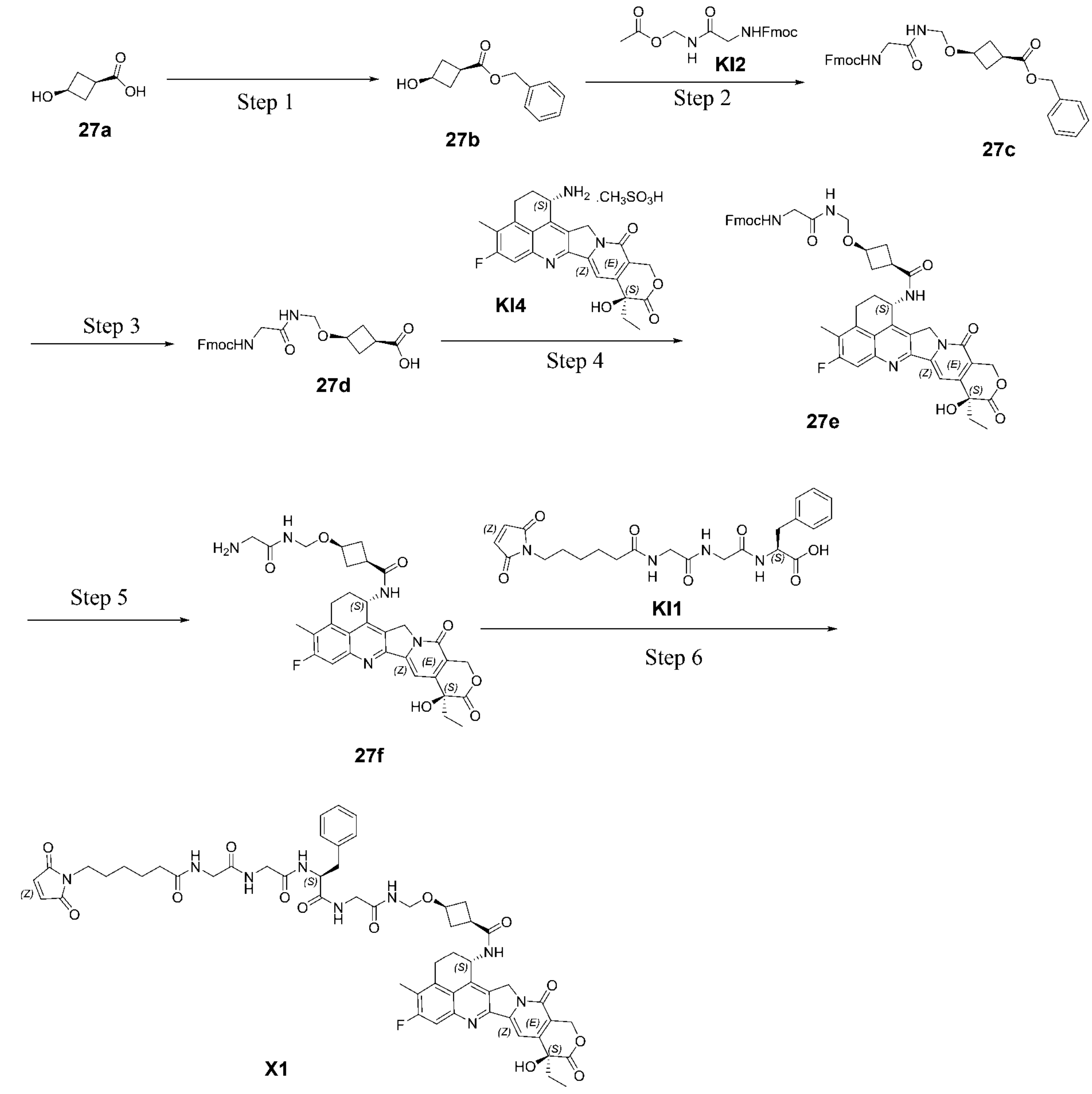


Step 1:

To a solution of 27a (5.00 g, 43.0 mmol) and $NaHCO_3$ (10.9 g, 129 mmol) in DMF (50 mL) was added dropwise benzyl bromide (11.0 g, 64.6 mmol) under a nitrogen atmosphere, and the mixture was reacted at 25°C for 17 hours. TLC (PE/EA = 2/1) indicated that the reaction was complete. The reaction mixture was added to 500 mL of water and extracted twice with EA (250 mL). The phases were separated, and the organic phase was washed with saturated sodium chloride aqueous solution (500 mL), dried over anhydrous $Na_2SO_4$, and concentrated. The residue was purified by column chromatography (PE: EA = 3:2) to obtain 5.1 g of colorless liquid with a yield of 57.1%.

Step 2:

To a solution of KI2 (4.00 g, 10.9 mmol) and TsOH (800 mg, 4.65 mmol) in THF (30 mL) was added dropwise a solution of 27b (4.50 g, 21.8 mmol) in THF (10 mL) at 0°C under a nitrogen atmosphere, and the mixture was reacted at 25°C for 2 hours. TLC (PE/EA = 1/2) indicated that the reaction was complete. The reaction mixture was added to 200 mL of water and extracted twice with EA (200 mL). The phases were separated, and the organic phase was dried over anhydrous $Na_2SO_4$ and concentrated. The residue was purified by column chromatography (PE/EA = 3/2) to obtain 1.56 g of white solid with a yield of 26%.

Step 3:

To a mixed solution of 27c (800 mg, 1.55 mmol) in EtOH (8 mL) and EA (8 mL) was added Pd/C (80 mg) at 0°C under a hydrogen atmosphere, and the mixture was stirred at 0°C for 2.5 hours. LCMS indicated that the reaction was complete. The reaction mixture was filtered through diatomite, and the filter cake was washed with EA (200 mL). The resulting mixture was concentrated, and the residue was dissolved in THF (20 mL) and subjected to rotary evaporation to dryness to obtain 600 mg of white solid with a yield of 91%.

Step 4:

To a solution of 27d (220 mg, 0.515 mmol), KI4 (250 mg, 0.47 mmol), and HATU (214 mg, 0.56 mmol) in DMF (6 mL) was added DIEA (152 mg, 1.18 mmol) at 0°C under a nitrogen atmosphere, and the mixture was reacted at 0°C for 2 hours. LCMS indicated that the reaction was complete. The reaction mixture was added to citric acid aqueous solution (pH = 4) (150 mL) and filtered, and the filter cake was washed with 175 mL of water. After filtration to dryness, the residue was dried under vacuum using an oil pump to obtain 260 mg of brown solid with a yield of 66%.

Step 5:

To a solution of 27e (260 mg, 0.309 mmol) in DCM (30 mL) was added dropwise diethylamine (8 mL) at 0°C under a nitrogen atmosphere, and the mixture was reacted at 0°C for 3 hours. LCMS indicated that the reaction was complete. The reaction mixture was added to petroleum ether solution (600 mL) at 0°C, resulting in the precipitation of a solid. The mixture was allowed to stand until the solid settled at the bottom of the flask. The solution was decanted, and the residue was dried under vacuum using an oil pump to obtain 90 mg of brown solid with a yield of 47.1%.

Step 6:

To a solution of 27f (90 mg, 0.13 mmol), KI-1 (92 mg, 0.19 mmol), and DIEA (50 mg, 0.39 mmol) in DMF (2.5 mL) was added HATU (74 mg, 0.19 mmol) at 0°C under a nitrogen atmosphere, and the mixture was reacted at 0°C for 2 hours. LCMS indicated that the reaction was nearly complete. The reaction mixture was added to citric acid aqueous solution (pH = 4) (30 mL) at 0°C, resulting in the precipitation of a flocculent solid. After filtration, the residue was purified by preparative TLC (DCM/MeOH = 10/1) to obtain 9.2 mg of light yellow solid X1 with a yield of 6%.

MS m/z (ESI): 1074 [M+1].

H-NMR (400 MHz, MeOD): 7.65 (d, 1H), 7.62 (s, 1H), 7.30 - 7.21(m, 5H), 6.79 (s, 2H), 5.69 - 5.65 (m, 1H), 5.57 (d, 1H), 5.43 - 5.10 (m, 3H), 4.70 (d, 2H), 4.48 - 4.39 (m, 2H), 4.10 - 4.05 (m, 1H), 4.01 - 3.75 (m, 5H), 3.46 (t, 2H), 3.22 - 3.15 (m, 2H), 3.07 - 3.00 (m, 1H), 2.75 (m, 1H), 2.62 (m, 1H), 2.45 (s, 3H), 2.37 - 2.20 (m, 6H), 2.10 - 2.02 (m, 2H), 2.00 - 1.92 (m, 2H) 1.68 - 1.57 (m, 6H), 1.01 (t, 3H).

Linker-payload X2:

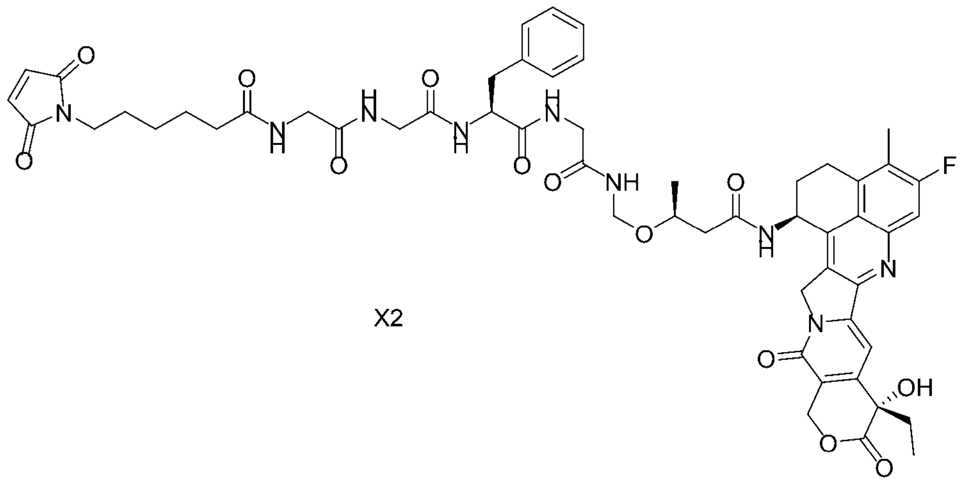

X2

Synthetic route:

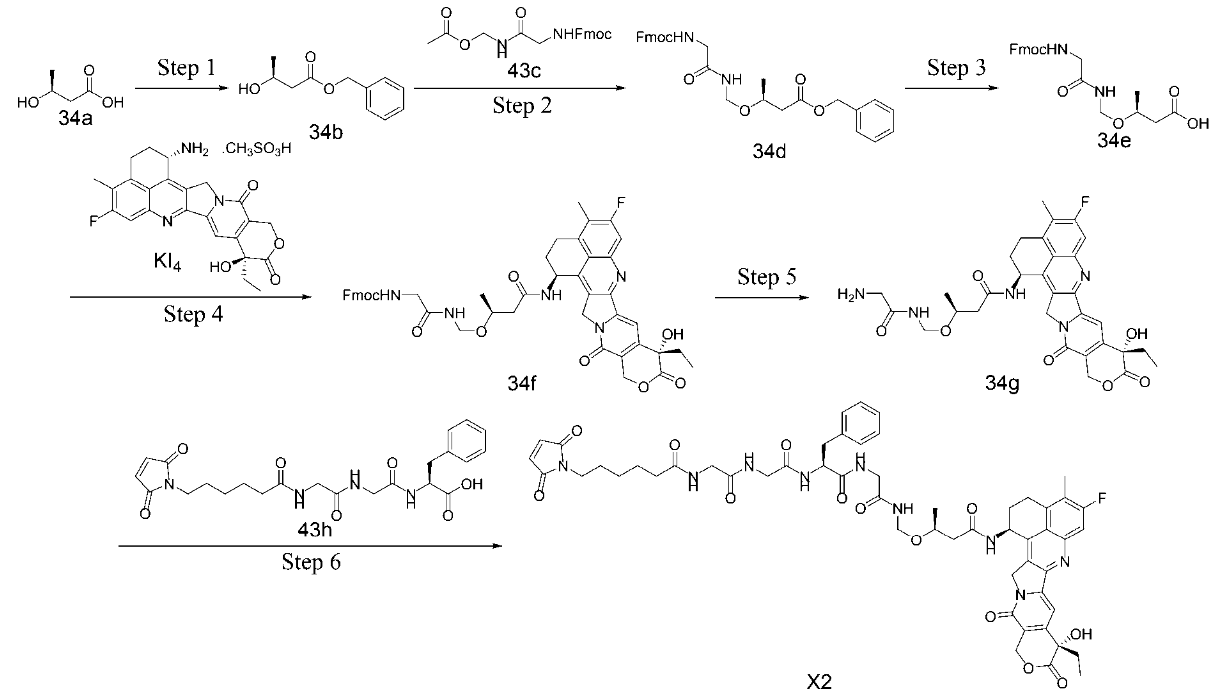


Step 1

34a (5 g, 48.0 mmol) and $K_2CO_3$ (19.9 g, 144.0 mmol) were dissolved in DMF (20 mL). Benzyl bromide (12.3 g, 72.0 mmol) was added dropwise thereto, and the mixture was reacted at 25°C for 17 hours. TLC (PE/EA = 3/1) indicated that the starting material was completely consumed. The reaction mixture was added to water (200 mL) and extracted with EA (250 mL). The phases were separated, and the organic phase was washed with saturated NaCl, dried over anhydrous $Na_2SO_4$, and concentrated. The residue was purified by column chromatography (PE: EA = 2:1) to obtain 8.7 g of colorless liquid 34b with a yield of 93%. MS-ESI: m/z 195.1 $[M+H]^+$.

Step 2

34c (7.3 g, 19.8 mmol) and TsOH (1.46 g, 8.5 mmol) were dissolved in THF (20 mL). The mixture was cooled to 0°C under a nitrogen atmosphere, and a solution of 43b (7.7 g, 39.6 mmol) in THF (10 mL) was added dropwise thereto. After the addition was completed, the mixture was reacted at 0°C for 2 hours. TLC (PE/EA = 2/1) indicated that most of the starting material was reacted. The reaction mixture was poured into 100 mL of water and extracted with DCM (100 mL). The phases were separated, and the organic phase was washed with saturated NaCl, dried over anhydrous $Na_2SO_4$, and purified by column chromatography (PE/EA = 1/1) to obtain 3.9 g of colorless viscous oil 34d with a yield of 39%. MS-ESI: m/z 503.3 $[M+H]^+$.

Step 3

To a mixed solution of 34d (1.9 g, 3.78 mmol) in EtOH (100 mL) and EA (100 mL) was added Pd/C (1 g, 10 wt.%) at 0°C under a hydrogen atmosphere, and the mixture was reacted at 0°C for 3 hours. TLC (PE/EA = 2/1) indicated that the reaction was complete. The reaction mixture was filtered through diatomite, and the filter cake was washed with EA/EtOH (1:1, 100 mL × 3). The filtrate was concentrated, and the residue was dissolved in THF (50 mL × 3) and subjected to rotary evaporation to dryness. The process was repeated three times to obtain 1 g of gray solid 34e with a yield of 64%. MS-ESI: m/z 435.2 $[M+Na]^+$.

Step 4

To a solution of 34e (426 mg, 1.03 mmol), KI4 (500 mg, 0.94 mmol), and HATU (429 mg, 1.13 mmol) in DMF (20 mL) was added dropwise DIEA (303 mg, 2.35 mmol) at 0°C under a nitrogen atmosphere. After the addition was completed, the mixture was reacted at 0°C for 2 hours. LCMS indicated that the reaction was complete. The reaction mixture was added dropwise to 300 mL of water, stirred, allowed to stand for 5 minutes, and filtered. The filter cake was dissolved in DCM/MeOH (10:1, 100 mL), dried, and subjected to rotary evaporation to dryness. The residue was mixed with silica gel and purified by column chromatography (EA: MeOH = 30:1) to obtain 600 mg of yellow solid 34f with a yield of 77%. MS-ESI: m/z 830.3 $[M+H]^+$.

Step 5

To a solution of 34f (150 mg, 0.18 mmol) in DCM (5 mL) was added dropwise diethylamine (5 mL) at 0°C under a nitrogen atmosphere, and the mixture was reacted at 0°C for 2 hours. LCMS indicated that the reaction was complete. The reaction mixture was added with petroleum ether solution (100 mL $\times$ 6), resulting in the precipitation of a solid. The mixture was allowed to stand until the solid settled, and the solution was decanted. The residue was then dried under vacuum using an oil pump to obtain 120 mg of white powder 34g. LCMS indicated that the product had a content of 70% and a yield of 76%. MS-ESI: m/z 608.3 $[M+H]^+$.

Step 6

To a solution of 34g (60 mg, 0.099 mmol), 43h (51 mg, 0.108 mmol), and DIEA (32 mg, 0.25 mmol) in DMF (1 mL) was added a solution of HATU (45 mg, 0.118 mmol) in DMF (1 mL) at 0°C under a nitrogen atmosphere, and the mixture was reacted at 0°C for 2 hours. LCMS indicated that the starting material was completely consumed. The reaction mixture was directly purified by reverse-phase column chromatography (eluent: (MeCN/MeOH = 1/1): $H_2O$ = 60%:40%) to obtain 14.8 mg of yellow solid X2 with a yield of 14%.

MS-ESI: m/z 1062.4 $[M+H]^+$.

$^1H$ NMR (400 MHz, Methanol-$d_4$) $\delta$ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

**Linker-payload X3:**

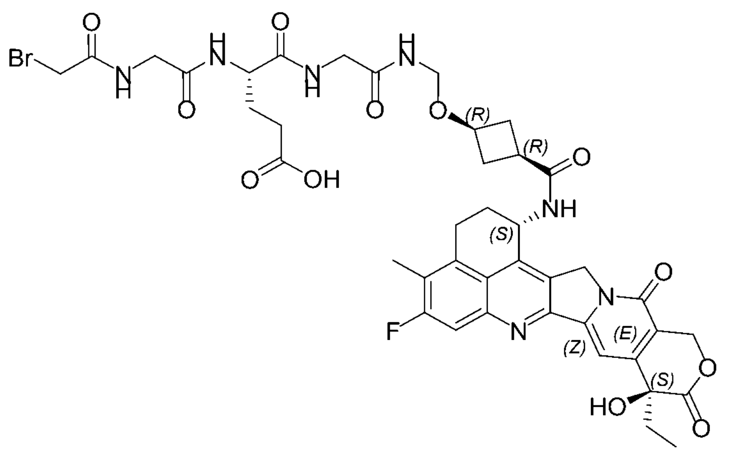

X3

**Synthetic route:**

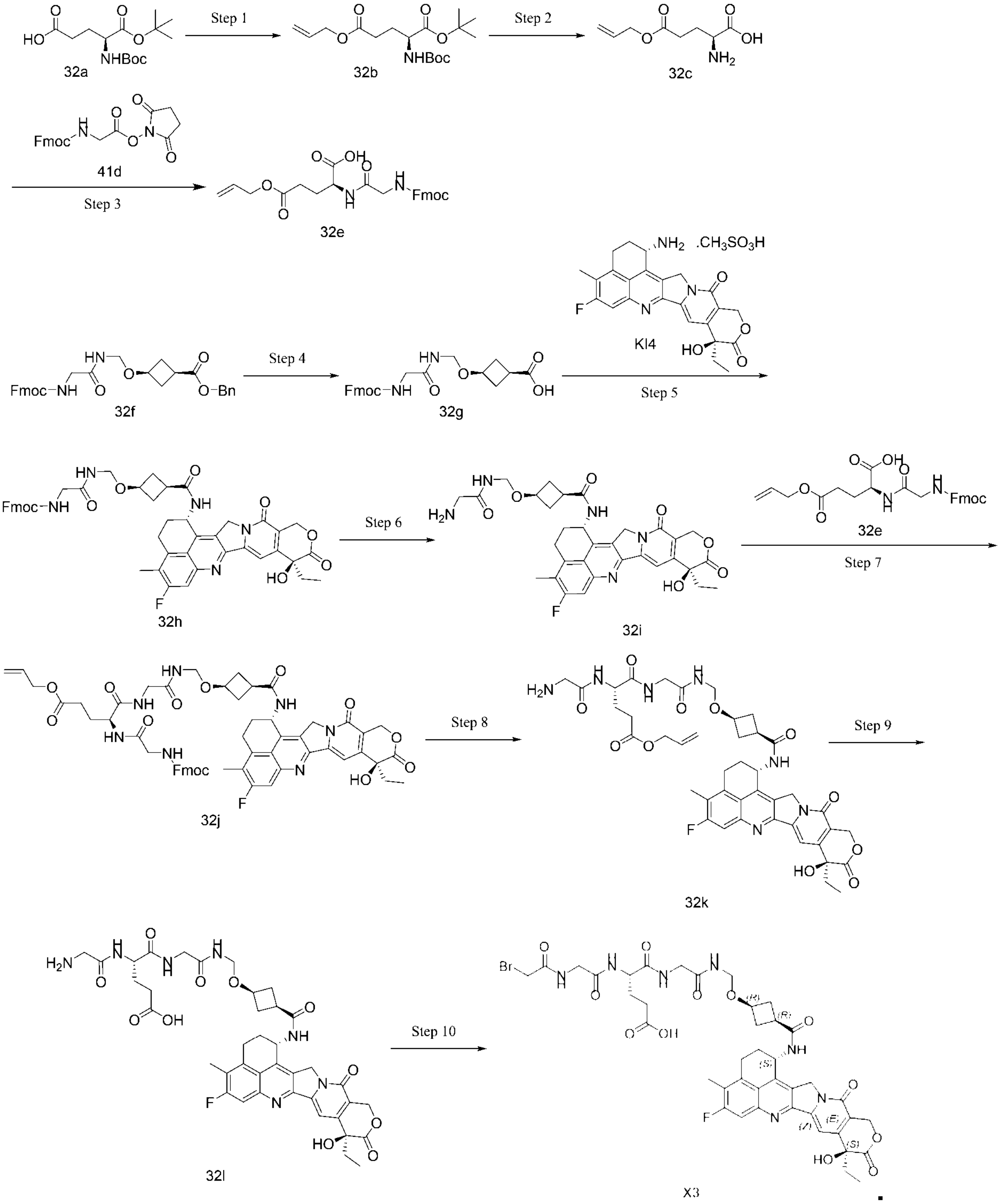


32a, 32b, 32c, 41d, 32e, Kl4, 32f, 32g, 32h, 32i, 32e, 32j, 32k, 32l, X3

Step 1:

To a solution of 32a (2.00 g, 6.6 mmol) and $K_2CO_3$ (1.82 g, 13.2 mmol) in MeCN (20 mL) was added bromopropene (960 mg, 7.92 mmol), and the mixture was reacted at 20°C for 5 hours. TLC (PE/EA = 1/2) indicated that the reaction was complete. The reaction mixture was poured into 100 mL of water to adjust the pH to 5, and extracted three times with EA (100 mL), dried over anhydrous sodium sulfate, and subjected to rotary evaporation to dryness. The residue was purified by column chromatography (PE/EA = 2/1) to obtain 1.83 g of white solid 32b with a yield of 81%.

Step 2:

To a solution of 32b (1.38 g, 4.02 mmol) in DCM (10 mL) was added TFA (10 mL), and the mixture was stirred at

25°C for 17 hours. TLC (PE/EA = 1/3) indicated that the reaction was complete. The reaction mixture was subjected to rotary evaporation to dryness to obtain 0.91 g of yellow viscous oil 32c (yield not calculated).

Step 3:

To a solution of 32c (910 mg, 4.87 mmol) and $NaHCO_3$ (613 mg, 7.3 mmol) in DME/$H_2O$ (20 mL/10 mL) was added 41d (1.92 g, 4.87 mmol), and the mixture was stirred at 25°C for 3 hours. TLC (DCM/MeOH = 1/1) indicated that the reaction was complete. The reaction mixture was poured into 100 mL of water, added with aq. HCl (1 N) to adjust the pH to 5, extracted twice with EA (150 mL), dried over anhydrous sodium sulfate, and subjected to rotary evaporation to dryness. The residue was purified by column chromatography (DCM/MeOH = 20/1) to obtain 1.53 g of white solid 32e with a yield of 67%. MS-ESI: m/z 467.4 $[M+H]^+$.

Step 4:

To a solution of 32f (3 g, 5.83 mmol) in MeOH (50 mL) was added Pd/C (600 mg), and the mixture was stirred under a hydrogen balloon at 25°C for 5 hours. TLC (EA) indicated that the reaction was complete. The reaction mixture was filtered and subjected to rotary evaporation to dryness to obtain 1.9 g of white solid 32g with a yield of 77%.

Step 5:

To a solution of 32g (789 mg, 1.86 mmol), KI4 (900 mg, 1.69 mmol), and triethylamine (342 mg, 3.38 mmol) in DMF (10 mL) was added HATU (707 mg, 1.86 mmol), and the mixture was stirred at 0°C for 3.5 hours. TLC (EA) indicated that the reaction was complete. The reaction mixture was poured into $H_2O$ (80 mL) and extracted twice with EA (100 mL). The organic phase was dried over anhydrous sodium sulfate, subjected to rotary evaporation to dryness, and purified by column chromatography (EA) to obtain 1.186 g of white solid 32h with a yield of 83%. MS-ESI: m/z 842.3 $[M+H]^+$.

Step 6:

A solution of 32h (1.186 g, 1.41 mmol) in DCM/diethylamine (20 mL, 20/1) was stirred at 25°C for 17 hours. TLC (DCM/MeOH = 10/1) indicated that the reaction was complete. The reaction mixture was poured into petroleum ether (200 mL) and filtered to obtain 768 mg of white solid 32i with a yield of 88%. MS-ESI: m/z 620.3 $[M+H]^+$.

Step 7:

To a solution of 32i (676 mg, 1.09 mmol), 32e (508 mg, 1.09 mmol), and DIEA (423 mg, 3.27 mmol) in DMF (10 mL) was added HATU (414 mg, 1.09 mmol), and the mixture was stirred at 20°C for 17 hours. TLC (PE/EA = 1/5) indicated that the reaction was complete. The reaction mixture was poured into water (30 mL) and filtered. The filter cake was purified by column chromatography (DCM/MeOH = 50/1) to obtain 511 mg of white solid 32j with a yield of 44%. MS-ESI: m/z 1068.3 $[M+H]^+$.

Step 8:

A solution of 32j (482 mg, 0.451 mmol) in diethylamine/DCM (10 mL, 1/5) was stirred at 10°C for 17 hours. TLC (EA) indicated that the reaction was complete. The reaction mixture was poured into PE (300 mL) and filtered to obtain 301 mg of white solid 32k (yield not calculated).

Step 9:

To a solution of 32k (301 mg, 0.356 mmol) and $Pd(PPh_3)_4$ (82 mg, 0.071 mmol) in THF (5 mL) was added morpholine (93 mg, 1.07 mmol), and the mixture was stirred at 25°C for 5 hours. LCMS indicated that the reaction was complete. The reaction mixture was purified by preparative TLC to obtain 108 mg of white solid 32l with a yield of 38%. MS-ESI: m/z 806.3 $[M+H]^+$.

Step 10:

To a solution of 32l (108 mg, 0.134 mmol) and triethylamine (41 mg, 0.402 mmol) in THF (2 mL) and DMF (2 mL) was added bromoacetyl bromide (27 mg, 0.134 mmol), and the mixture was stirred at 0°C for 1 hour. TLC (DCM/MeOH = 10/1) indicated that the reaction was complete. The reaction mixture was directly purified by preparative TLC to obtain 15

mg of white solid X3 with a yield of 12%.

MS-ESI: m/z 926.3 $[M+H]^+$.

$^1H$ NMR (400 MHz, DMSO-$d_6$) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

Linker-payload X4:

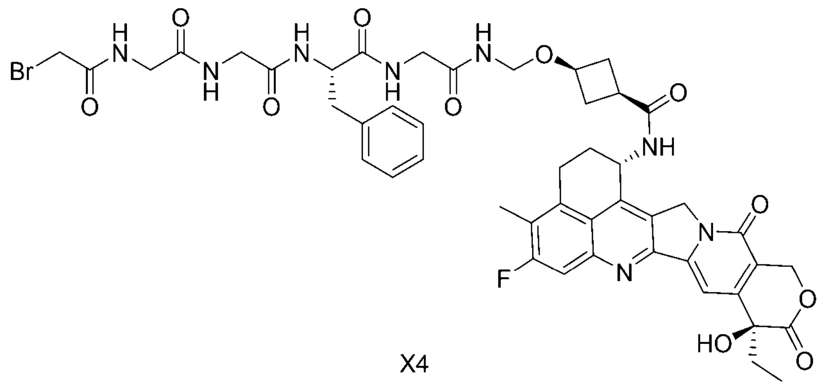

X4

Synthetic route:

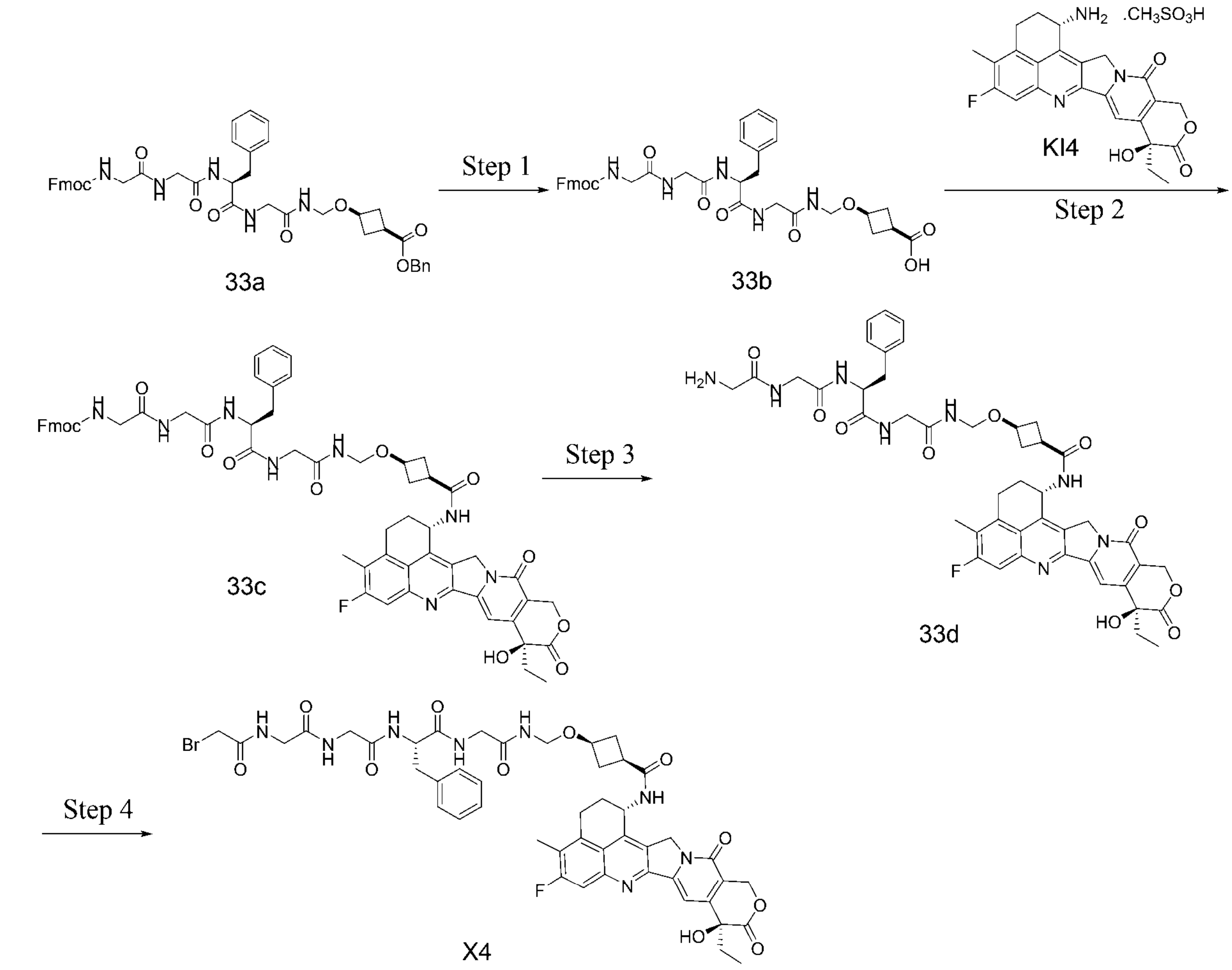


33a 33b KI4 33c 33d X4

Step 1:

To a solution of 33a (2.00 g, 2.58 mmol) in MeOH (20 mL) was added Pd/C (400 mg, 10 wt.%), and the mixture was stirred at 20°C for 5 hours. TLC (EA) indicated that the reaction was complete. The reaction mixture was filtered and subjected to rotary evaporation to dryness to obtain 1.3 g of white solid 33b with a yield of 74%.

Step 2:

To a solution of 33b (0.55 g, 0.802 mmol), KI4 (427 mg, 0.802 mmol), and DIPEA (310 mg, 2.40 mmol) in DMF (5 mL) was added HATU (305 mg, 0.802 mmol), and the mixture was stirred at 0°C for 2 hours. TLC (DCM/MeOH = 1/10) indicated that the reaction was complete. The reaction mixture was poured into water (40 mL) and filtered to obtain a crude product, which was purified by column chromatography (DCM/MeOH = 20/1) to obtain 360 mg of yellow solid 33c with a yield of 41%.

Step 3:

To a solution of 33c (360 mg, 0.326 mmol) in DCM (10 mL) was added diethylamine (2 mL), and the mixture was stirred at 25°C for 17 hours. TLC (DCM/MeOH = 5/1) indicated that the reaction was complete. The reaction mixture was poured into PE (100 mL) and filtered to obtain 205 mg of white solid 33d with a yield of 71%. MS-ESI: m/z 881.3 $[M+H]^+$.

Step 4:

To a solution of 33d (205 mg, 0.233 mmol) and triethylamine (118 mg, 1.17 mmol) in DMF (1 mL) and water (1 mL) was added a solution of bromoacetyl bromide (94 mg, 0.446 mmol) in THF (2 mL), and the mixture was stirred at 0°C for 1 hour. The reaction mixture was directly purified by preparative TLC to obtain 15 mg of white solid X4 with a yield of 6%.
MS-ESI: m/z 1001.2 $[M+H]^+$.

$^1H$ NMR (400 MHz, DMSO-$d_6$) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

**2.2. Preparation of bispecific antibody-drug conjugate**

**Preparation of bispecific antibody-drug conjugate DSYE001-X1 (DAR4):**

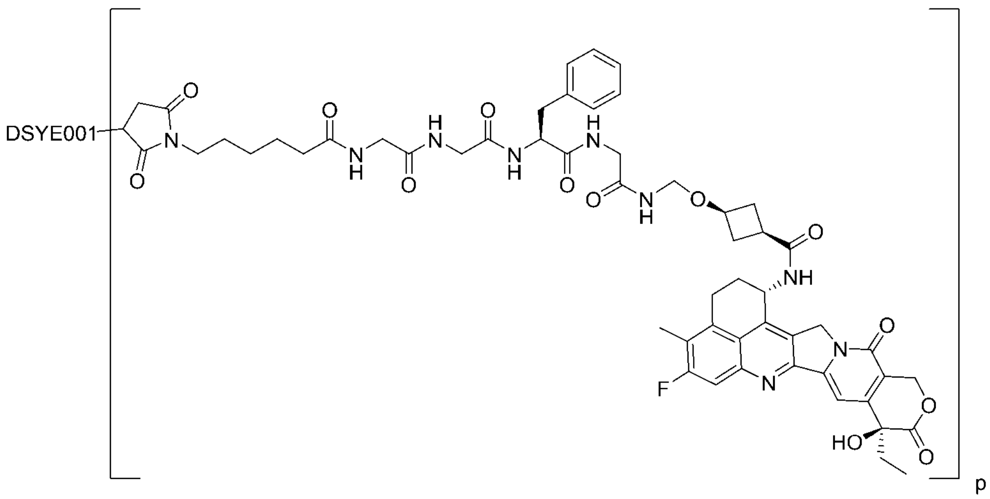

To a buffer solution of bispecific antibody DSYE001 (30 mM histidine-acetate + 20 mM EDTA, pH 5.5; 100 mg, 10.0 mg/mL, 0.578 μmol) was added the prepared tris(2-carboxyethyl)phosphine hydrochloride solution (7.0 mM, 0.215 mL, 1.503 μmol) at 37°C. The mixture was shaken in a temperature-controlled water bath shaker at 37°C for 2 hours, after which the reaction was terminated.

Linker-payload X1 (3.10 mg, 2.89 μmol) was dissolved in 1.0 mL of DMA (*N,N*-dimethylacetamide) and added to the above antibody solution. The mixture was shaken in a water bath shaker at 22°C for 2 hours, after which the reaction was terminated. The reaction mixture was desalted and purified on a Sephadex G25 gel column (elution phase: 30 mM histidine-HCl, pH 5.5), followed by ultrafiltration concentration to obtain a solution of the bispecific antibody-drug conjugate ADC DSYE001-X1 (DAR4) (30 mM histidine-HCl, pH 5.5; 93.8 mg, 12.7 mg/mL, yield: 93.8%), which was stored at 4°C in the dark.

The DAR value was determined as p = 4.02 following HIC DAR analysis.

**Preparation of bispecific antibody-drug conjugate DSYE001-X1 (DAR6):**

To a reaction vessel was added a solution of antibody DSYE001 (20 mM His/His-HCl, 90 mg/mL sucrose, 0.02% polysorbate 80, pH 6.3, 10,000 mg, 20.52 mg/mL, 57.80 μmol), followed by the addition of reducing buffer (20 mM PB, pH 6.7, 93.59 mL) and 10 mM DTPA solution (66.67 mL). The reaction mixture was added with 0.4 M $Na_2HPO_4$ solution (7.0 mL) to adjust the pH to 6.72. Subsequently, the prepared tris(2-carboxyethyl)phosphine hydrochloride solution (10 mM, 19.08 mL, 190.74 μmol) was added to the reaction vessel. The reaction mixture was stirred at 50 to 100 rpm for 3.5 hours at a controlled temperature of 25°C. The reaction mixture was added with 0.5 M $Na_2HPO_4$ solution (200 mL) to adjust the pH to 5.5. Subsequently, the reaction mixture was cooled to 20°C, followed by the addition of DMSO (32.06 mL). Linker-payload X1 (465.7 mg, 433.56 μmol) was then dissolved in 43.35 mL of DMSO and added to the above reaction system. The reaction mixture was stirred at 50 to 100 rpm for 1.25 hours at a controlled temperature of 22°C. The prepared NAC solution (50 mM, 43.35 mL, 2.17 mM) was then added thereto. The reaction mixture was stirred at 50 to 100 rpm for 0.5 hours at a controlled temperature of 22°C. The feed solution was filtered through an activated carbon filter (Cobetter, CDFCSCSDAC01PCP, 23 $cm^2$), followed by ultrafiltration and buffer exchange with an ultrafiltration membrane cassette (30 KD, 0.11 $m^2$) to obtain the exemplary product DSYE001-X1 (DAR6) (10 mM histidine-succinate, pH 5.0, 14.3 mg/mL), which was stored at -80°C.

The DAR value was determined as n = 6.1 following HIC DAR analysis.

**Preparation of bispecific antibody-drug conjugate DSYE001-X1 (DAR8):**

To a reaction vessel was added a solution of antibody DSYE001 (20 mM His/His-HCl, 90 mg/mL sucrose, 0.02% polysorbate 80, pH 6.3, 10,000 mg, 20.52 mg/mL, 57.80 μmol), followed by the addition of reducing buffer (45 mM PB, pH 7.35, 72.2 mL) and 10 mM DTPA solution (66.67 mL). Subsequently, the prepared tris(2-carboxyethyl)phosphine hydrochloride solution (10 mM, 40.46 mL, 404.6 μmol) was added to the reaction vessel. The reaction mixture was stirred at 50 to 100 rpm for 4.5 hours at a controlled temperature of 25°C. The reaction mixture was added with 0.5 M $Na_2HPO_4$ solution (200 mL) to adjust the pH to 5.5. Subsequently, the reaction mixture was cooled to 20°C, followed by the addition of DMSO (63.40 mL). Linker-payload X1 (651.9 mg, 606.91 μmol) was then dissolved in 60.69 mL of DMSO and added to the above reaction system. The reaction mixture was stirred at 50 to 100 rpm for 1.25 hours at a controlled temperature of 22°C. The prepared NAC solution (50 mM, 60.69 mL, 3.03 mM) was then added thereto. The reaction mixture was stirred at 50 to 100 rpm for 0.5 hours at a controlled temperature of 22°C. The feed solution was filtered through an activated carbon filter (Cobetter, CDFCSCSDAC01PCP, 23 $cm^2$), followed by ultrafiltration and buffer exchange with an ultrafiltration membrane cassette (30 KD, 0.11 $m^2$) to obtain the exemplary product DSYE001-X1 (DAR8) (10 mM histidine-succinate, pH 5.0, 29.5 mg/mL), which was stored at -80°C.

The DAR value was determined as n = 7.9 following HIC DAR analysis.

**Preparation of anti-B7H3 antibody-drug conjugate DSYE003-X1 (DAR6) (reference ADC-1)**

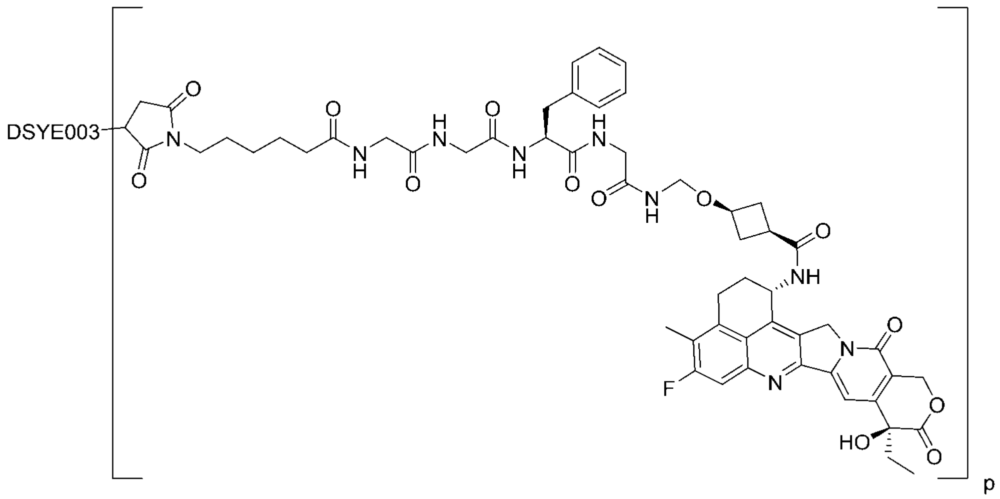

To a buffer solution of antibody DSYE003 (PBS, pH 7.4; 40 mg, 5.36 mg/mL, 0.23 μmol) was added 30 mM His-HAc buffer (pH 5.5, 1.845 mL), followed by the addition of 100 mM EDTA solution (0.5 mL) and the prepared tris(2-carboxyethyl)phosphine hydrochloride solution (6.977 mM, 0.208 mL, 1.45 μmol). The mixture was stirred in a temperature-controlled stirrer at 500 rpm for 2 hours at 37°C, after which the reaction was terminated. Subsequently, 0.750 mL of DMA was added to the above reaction system. Linker-payload X1 (2.5 mg, 2.32 μmol) was then dissolved in 0.25 mL of DMA and added to the above reaction system. The mixture was stirred in a temperature-controlled stirrer at 500 rpm for 1 hour at 4°C, after which the reaction was terminated. A 300 mg/mL suspension of activated charcoal (Charcoal, Dextran Coated, Sigma Aldrich) was added at 10% of the total reaction volume. The mixture was vortexed and mixed in a rotary mixer at 4°C for 1 hour, after which the supernatant was collected by centrifugation. An additional suspension of activated charcoal was then added at 10% of the total supernatant volume. The mixture was vortexed and mixed in a rotary mixer at 4°C for 1 hour. After centrifugation at 4,300 rcf for 10 minutes, the supernatant was filtered through a 0.22 μm syringe filter (Merck Millipore), followed by ultrafiltration concentration with 4 DVs using a 30 KD ultrafiltration tube with 30 mM His-HAc buffer (pH 5.5) to obtain a solution of the exemplary product DSYE003-X1 (DAR6) (30 mM His-HAc, pH 5.5; 35.89 mg, 6.14 mg/mL, yield: 89.74%), which was stored at -80°C.

The DAR value was determined as n = 6.32 following HIC DAR analysis.

**Preparation of anti-B7H3 antibody-drug conjugate DSYE002-X1 (DAR6) (reference ADC-2)**

Using linker-payload X1 and anti-B7H3 antibody DSYE002, DSYE002-X1 (DAR6) was prepared following the preparation method for the bispecific antibody-drug conjugate ADC DSYE001-X1.

**Preparation of anti-PD-L1 antibody-drug conjugate DSYE004-X1 (DAR6) (reference ADC-3)**

Using linker-payload X1 and anti-PD-L1 antibody DSYE004, DSYE004-X1 (DAR6) was prepared following the preparation method for the bispecific antibody-drug conjugate ADC DSYE001-X1.

**Preparation of bispecific antibody-drug conjugate DSYE001-X2**

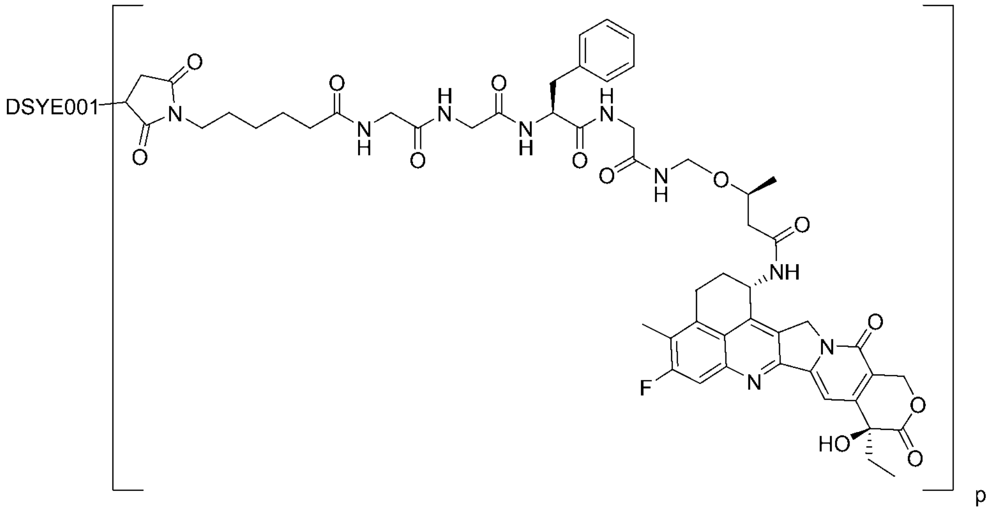

To a buffer solution of bispecific antibody DSYE001 (30 mM histidine-acetate + 20 mM EDTA, pH 5.5; 100 mg, 10.0 mg/mL, 0.578 μmol) was added the prepared tris(2-carboxyethyl)phosphine hydrochloride solution (7.0 mM, 0.207 mL, 1.449 μmol) at 37°C. The mixture was shaken in a temperature-controlled water bath shaker at 37°C for 2 hours, after which the reaction was terminated.

Linker-payload X2 (3.07 mg, 2.89 μmol) was dissolved in 1.0 mL of DMA (*N,N*-dimethylacetamide) and added to the above antibody solution. The mixture was shaken in a water bath shaker at 22°C for 2 hours, after which the reaction was terminated. The reaction mixture was desalted and purified on a Sephadex G25 gel column (elution phase: 30 mM histidine-HCl, pH 5.5), followed by ultrafiltration concentration to obtain a solution of the bispecific antibody-drug conjugate ADC DSYE001-X2 (30 mM histidine-HCl, pH 5.5; 96.5 mg, 15.8 mg/mL, yield: 96.5%), which was stored at 4°C in the dark.

The DAR value was determined as p = 3.95 following HIC DAR analysis.

**Preparation of anti-B7H3 antibody-drug conjugate DSYE002-X2 (reference ADC-4)**

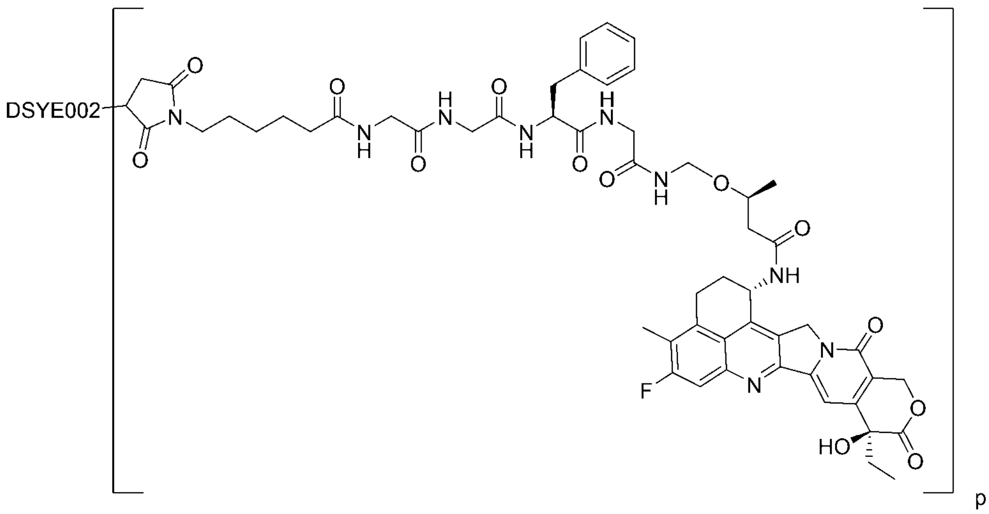

Antibody DSYE002 served as a reference antibody for the bispecific antibody DSYE001 in which the PD-L1 antibody was removed. The amino acid sequences of DSYE002 are shown in the sequence listing.

To a buffer solution of anti-B7H3 antibody DSYE002 (30 mM histidine-acetate + 20 mM EDTA, pH 5.5; 60 mg, 5.5 mg/mL, 0.755 μmol) was added the prepared tris(2-carboxyethyl)phosphine hydrochloride solution (7.0 mM, 1.08 mL, 7.55 μmol) at 37°C. The mixture was shaken in a temperature-controlled water bath shaker at 37°C for 2 hours, after which the reaction was terminated.

Linker-payload X2 (10.8 mg, 9.82 μmol) was dissolved in 1.2 mL of DMA and added to the above reaction system. The mixture was shaken in a water bath shaker at 4°C for 1 hour, after which the reaction was terminated. The reaction mixture was desalted and purified on a Sephadex G25 gel column (elution phase: 30 mM histidine-HCl, pH 5.5), followed by ultrafiltration concentration to obtain a solution of the exemplary product DSYE002-X2 (30 mM histidine-HCl, pH 5.5; 30.5 mg, 7.5 mg/mL, yield: 50.8%), which was stored at 4°C in the dark.

The DAR value was determined as p = 3.98 following HIC DAR analysis.

**Preparation of isotype control ADC**

Using isotype control antibody and linker-payload X1, isotype control ADC (DAR4), isotype control ADC (DAR6), and isotype control ADC (DAR8) were prepared following the preparation method for the bispecific antibody-drug conjugate ADC DSYE001-X1.

**Example 3: Endocytosis activity of antibody-drug conjugates**

Test purpose

The endocytosis effect of the anti-B7H3 and PD-L1 bispecific antibody-drug conjugate of the present disclosure on A375 and NCI-H1975 cells co-expressing B7H3 and PD-L1 was evaluated. Cells were co-incubated with a fixed concentration of the test compound and the endocytosis indicator pHrodo. The endocytosis capability of the test compound was assessed by monitoring the fluorescence signal generated by pHrodo entering cells concomitantly with the antibody-drug conjugate at different time points.

Experimental method:

1. A375 and NCI-H1975 cells were added at a density of $1.2 \times 10^4$ cells/well to a 95-well black culture plate pre-coated with 8 μg/mL substrate. The plate was incubated overnight at 37°C under 5% $CO_2$.
2. The test compound was mixed with Fab-pHrodo at a ratio of 1:1.2 and incubated at 37°C under 5% $CO_2$ for 0.5 hours. The mixture was 4-fold serially diluted from 100 nM to 0.0061 nM.
3. The diluted sample was added to the culture plate at 50 μL/well and incubated at 37°C under 5% $CO_2$ for 24 hours.
4. After incubation, the supernatant was removed, and the culture plate was rinsed once with 1% BSA.
5. The cells were stained with 100 μL/well of DPBS containing 1 μg/mL Hoechst 33342 and 0.5 μg/mL Calcein AM, and incubated at room temperature for 15 minutes.
6. After incubation, the supernatant was removed, and the culture plate was rinsed once with 1% BSA.
7. The fluorescent puncta formed by internalized antibodies per cell were quantified using a Perkin Elmer Operetta CLS High-Content Analysis System.

The results are shown in Table 1 and FIG. 15.

Table 1. Endocytosis activity of DSYE001-X2 cells

| | Endocytosis assay data | | | |
|---|---|---|---|---|
| | A375 | | NCI-H1975 | |
| | $EC_{50}$ (nM) | Maximum mean fluorescence intensity | $EC_{50}$ (nM) | Maximum mean fluorescence intensity |
| DSYE001-X2 | 1.83 | 16.5 | 0.93 | 8.3 |
| DSYE002-X2 | 1.82 | 11.7 | 1.31 | 7.9 |
| Isotype control ADC | N.A. | 0.08 | N.A. | 6.1 |
| Fluorescent dye control | N.A. | 0.6 | N.A. | 4.5 |

The experimental results demonstrated that, compared to the anti-B7H3 antibody-drug conjugate DSYE002-X2, the bispecific antibody-drug conjugate DSYE001-X2 of the present disclosure exhibited superior endocytosis effect in the assayed cells, as evidenced by, for example, higher maximum mean fluorescence intensity or lower $EC_{50}$ values.

**Example 4: *In vitro* proliferation inhibition assay of antibody-drug conjugates on tumor cells**

The CellTiter-Glo® luminescent cell viability assay (CTG method) was used to evaluate the anti-proliferative effects of anti-B7H3/PD-L1 bispecific antibody-drug conjugate DSYE001-X2 and anti-B7H3 antibody-drug conjugate DSYE002-X2 on B7H3- and Pd-L1-positive A375 and NCI-H1975 cells after 6 days of incubation.
A375 and NCI-H1975 cells in the logarithmic growth phase were harvested and seeded at a density of 400 cells/well and 1000 cells/well, respectively, with a volume of 50 μL per well. The cell plate was incubated overnight in an incubator at 37°C with 5% $CO_2$. On day 2, DSYE001-X2 and DSYE002-X2 were 3-fold serially diluted in complete medium to generate 8 concentration gradients (starting from 1000 nM as the highest concentration). 50 μL/well of each dilution was added to the cell culture plate with complete medium serving as a blank control, and 2 replicate wells were set up. The plate was incubated for another 6 days in an incubator at 37°C with 5% $CO_2$. After incubation, the cell culture plate was taken out and equilibrated to room temperature. 50 μL/well of CTG reagent (Promega, Cat#: G7573) was added, and the mixture was vortexed and mixed. The plate was allowed to stand for 10 minutes, and luminescence was measured using a microplate reader. S-shaped dose-response curves were plotted using a nonlinear regression model in GraphPad Prism software, and $IC_{50}$ values were calculated. Cell viability was calculated using the formula: cell viability = ($Lum_{test\ compound}$ - $Lum_{blank}$ control) / ($Lum_{vehicle\ control}$ - $Lum_{blank\ control}$) × 100%.
The experimental results demonstrated that the bispecific antibody-drug conjugate DSYE001-X2 of the present disclosure exhibited comparable anti-proliferative activity to DSYE002-X2 against B7H3- and PD-L1-positive A375 and NCI-H1975 cells.

**Example 5: *In vitro* proliferation inhibition assay of antibody-drug conjugates on tumor cells**

The CellTiter-Glo® luminescent cell viability assay (CTG method) was used to evaluate the anti-proliferative effects of anti-DSYE001-X1 (DAR8) and isotype control ADC (DAR8) on B7H3- and Pd-L1-positive human tumor cells after 7 days of incubation.
Cells in the logarithmic growth phase were harvested and seeded at a density of 1000 to 3000 cells/well. The cell plate was incubated overnight in an incubator at 37°C with 5% $CO_2$. On day 2, DSYE001-X1 (DAR8) was 3-fold serially diluted in complete medium to generate 9 concentration gradients (starting from 1000 nM as the highest concentration). 50 μL/well of each dilution was added to the cell culture plate with complete medium serving as a blank control, and 3 replicate wells were set up. The plate was incubated for another 6 days in an incubator at 37°C with 5% $CO_2$. After incubation, the cell culture plate was taken out and equilibrated to room temperature. 50 μL/well of CTG reagent was added, and the mixture was vortexed and mixed. The plate was allowed to stand for 10 minutes, and luminescence was measured using a microplate reader. S-shaped dose-response curves were plotted using a nonlinear regression model in GraphPad Prism software, and $IC_{50}$ values were calculated. Cell viability was calculated using the formula: cell viability = ($Lum_{test\ compound}$ - $Lum_{blank}$ control) / ($Lum_{vehicle}$ control - $Lum_{blank}$ control) × 100%.
The results are shown in Table 2 and FIG. 16.

Table 2. *In vitro* anti-proliferative effect of DSYE001-X1 (DAR8) on tumor cells

| Cell line | DSYE001-X1 (DAR8) ($EC_{50}$, nM) | Isotype control ADC ($EC_{50}$, nM) |
|---|---|---|
| A375 | 6.248 | 249.4 |
| NCI-H1975 | 226.8 | 431.8 |
| NCI-H441 | 14.3 | 107.7 |
| NCI-H358 | 0.03515 | 47.06 |

The experimental results demonstrated that the bispecific antibody-drug conjugate DSYE001-X1 of the present disclosure exhibited significant anti-proliferative activity against B7H3- and PD-L1-positive A375, NCI-H1975, NCI-H441, and NCI-H358 cells among a variety of tumor cells.

**Example 6: Effect of antibody-drug conjugates on PD-L1 expression in NCI-H1975 cells**

The purpose of this experiment was to investigate the differential effects of bispecific antibody-drug conjugates

versus their parental monoclonal antibody conjugates on PD-L1 expression in tumor cells.

Experimental method

1) NCI-H1975 tumor cells were cultured in RPMI 1640 medium supplemented with 10% FBS in a temperature-controlled incubator at 37°C with 5% $CO_2$.
2) The cells were trypsinized and counted to ensure a viability of 90.0% or more, and then seeded in a 6-well plate at a density of 2 × $10^6$ cells/2 mL per well.
3) On day 2, the cells were treated with medium (blank control), isotype control antibody, isotype control ADC, DSYE001-X1 (DAR6), DSYE003-X1 (DAR6), and Anti-PDL1-X1 (final concentration of 100 nM) and incubated at 37°C for 72 hours.
4) After incubation, the cells were washed twice with PBS. Each culture plate (6-well plate) was supplemented with 500 μL of ice-cold RIPA lysis buffer (containing 1% protease inhibitor and 1% phosphatase inhibitor), followed by incubation on ice for 30 min with intermittent mixing.
5) The plate was centrifuged at approximately 14,000 rpm for 10 minutes at 4°C to remove cell debris. The supernatant was transferred to a new tube for protein concentration determination and subsequent experiments.
6) Protein quantification was performed using a BCA quantification kit. Based on the quantitative results, protein samples for loading were prepared by normalizing the protein concentration to 1 to 2 μg/μL, followed by the addition of LDS loading buffer (4X) and sample reducing agent (10X). The samples were heated at a constant temperature of 100°C for 10 minutes.
7) Alternatively, the denatured samples were stored in a -80°C freezer.
8) The samples were thawed prior to loading.
9) Western blot: Loading: the samples were loaded into SDS-PAGE gel at 10 μL per well (the loading volume depended on antibody titer); electrophoresis: 80 V for 30 minutes, followed by 120 V for 90 minutes; transfer: iBlot2 transfer kit and transfer instrument with P3 program for 7 minutes.
10) After transfer, the membrane was cut according to the molecular weight of the target protein, and washed with 1X TBST three times for 5 minutes each at room temperature with shaking.
11) Blocking: The membrane was blocked in blocking buffer (5% skim milk in 1X TBST) for 1 hour at room temperature with shaking.
12) The membrane was washed with 1X TBST 3 times for 5 minutes each at room temperature with shaking.
13) Primary antibody incubation: The primary antibody was added at an appropriate dilution (5% bovine serum albumin in 1X TBST), followed by incubation overnight at 4°C with gentle shaking.
14) The membrane was washed with 1X TBST 3 times for 10 minutes each at room temperature with shaking.
15) Secondary antibody incubation: The secondary antibody was added at an appropriate dilution, followed by incubation for 1 hour at room temperature with gentle shaking.
16) The membrane was washed with 1X TBST 3 times for 10 minutes each at room temperature with shaking.
17) Chemiluminescence: HRP substrate from the West Femto Maximum Sensitivity Chemiluminescence Kit was added to the membrane.
18) Chemiluminescence was detected and photographed using a Bio-Rad ChemiDocTMxrs+ or Tanon 5200 Multi system.
29) Detection indicators: PD-L1 and β-actin.

The results are shown in Table 3 and FIG. 17.

Table 3. Effect of DSYE001-X1 (DAR6) on PD-L1 expression in NCI-H1975 cells

| Experimental group | Blank control | Isotype control antibody | Isotype control ADC | DYSE001-X1 (DAR6) | DYSE003-X1 (DAR6) | DSYE004-X1 (DAR6) |
|---|---|---|---|---|---|---|
| Relative PD-L1 expression | 3 | 2.63 | 2.76 | 2.14 | 2.88 | 2.47 |

The results demonstrated that the bispecific antibody-drug conjugate DSYE001-X1 more significantly induced downregulation of PD-L1 expression compared to both the parental B7H3 single-arm antibody conjugate DSYE003-X1 and the parental PD-L1 antibody conjugate anti-PD-L1-X1 (DSYE004-X1), suggesting that the bispecific antibody-drug conjugate DSYE001-X1 can exert stronger immune suppression relief by reducing target expression levels in addition to exerting an immunomodulatory effect by inhibiting the binding to the corresponding receptors through dual target engagement.

**Examples 7: Evaluation of the efficacy of antibody-drug conjugates in syngeneic mouse model**

To investigate the inhibitory effect of the anti-B7H3 and PD-L1 bispecific antibody-drug conjugate of the present disclosure on tumor growth, a syngeneic mouse model was established in B7H3 and PD-L1 humanized BALB/c mice (BALB/c-hPD-L1/hB7H3) implanted with CT26 cells stably expressing human B7H3 and PD-L1 (CT26-hPD-L1/hB7H3) to evaluate the anti-tumor efficacy of the bispecific antibody-drug conjugate DSYE001-X2.

1. Test drugs and materials

    Isotype control ADC: 10 mg/kg
    G1: Blank control (control group): normal saline
    G2: DSYE002-X2 (reference ADC-4, treatment group): 12 mg/kg
    G3: DSYE001-X2 (treatment group): 5.5 mg/kg
    Note: All test compounds were administered at equimolar doses.

2. Preparation method: All samples were diluted with normal saline.
3. Experimental animals: BALB/c-hPD-L1/hB7-H3 mice, female, 6 to 8 weeks old, weighing approximately 18 to 22 g, purchased from GemPharmatech Co., Ltd. (Jiangsu, China).
4. Experimental method:

CT26-hPD-L1/hB7H3 cells were thawed and cultured, and cryopreserved batches were recorded. CT26-hPD-L1/hB7H3 cells in the logarithmic growth phase (passages 3 to 4 after thawing) were harvested. The culture medium was removed, and the cells were washed twice with DPBS before inoculation (cell viability was measured before and after inoculation). Mice were subcutaneously inoculated with the cells in the right flank at $1 \times 10^6$ cells/100 μL/mouse (without Matrigel). When the tumor volume reached 80 to 120 $mm^3$, the mice were grouped with the day of grouping defined as D0, and dosing was initiated on D0. The coefficient of variation (CV) of tumor volume did not exceed 1/3. The isotype control ADC, DSYE001-X2, and DSYE002-X2 were administered via intravenous (i.v.) injection once weekly for a total of 2 doses at 10 mg/kg, 12 mg/kg, and 5.5 mg/kg, respectively. The experiment was terminated 19 days after dosing. Tumor volume and body weight were measured twice weekly, and data were recorded. Tumor growth inhibition rate was calculated based on tumor volume measurements.

At the end of the experiment, the mice were euthanized, and the tumor growth inhibition rate (TGI) was calculated (TGI (%) = $[1 - (T_i - T_0) / (V_i - V_0)] \times 100$). $T_i$: mean tumor volume of the treatment group and positive control group on day i of dosing; $T_0$: mean tumor volume of the treatment group and positive control group on day 0 of dosing; $V_i$: mean tumor volume of the negative control group on day i of dosing; $V_0$: mean tumor volume of the negative control group on day 0 of dosing.

The experimental results are as shown in FIG. 18 and Tables 4 to 6.

Table 4. Tumor volume[1] ($mm^3$) at different time points for each group

| Group | Day | 0[2] | 2 | 5 | 7 | 9 | 12 | 14 | 16 | 19 |
|---|---|---|---|---|---|---|---|---|---|---|
| G1 | Blank control | 81.50 ±1.90 | 126.89 | | | ±12.14 | 267.76 ±45.49 | 438.60 ±73.78 | 724.36 | ±136.67 |
| 1204.-64 | ±212.77 | 1657.-05 | | | | | | | | ±272.24 |
| 2160.-02 | ±347.65 | 2691.-31 | | | | | | | | ±280.36 |
| G2 | DSYE002-X2 | 81.33 ±2.24 | 120.11 ±11.40 | 181.04 ±25.11 | 268.83 | ±53.55 | 293.77 ±24.34 | 490.60 ±61.40 | 689.85 ±91.49 | 1063.95 ±104.31 |
| 1594.-33 | ±196.83 | | | | | | | | | |
| G3 | DSYE001-X2 | 81.42 ±2.11 | 107.80 ±5.80 | 107.72 | | ±13.27 | 93.29 ±15.59 | 91.61 ±27.02 | 122.18 ±50.29 | 180.09 ±89.34 |

(continued)

| Group | Day | $0^2$ | 2 | 5 | 7 | 9 | 12 | 14 | 16 | 19 |
|---|---|---|---|---|---|---|---|---|---|---|
| 275.66 | ±129.79 | 413.22 | | | | | | | | ±154.54 |
| Note:<br>1. Tumor volume is expressed as mean ± standard error;<br>2. Days after initiation of dosing. | | | | | | | | | | |

Table 5. Changes in tumor volume inhibition rate ($TGI_{TV}$) in CT26 (CT26-hPD-L1/hB7H3)-implanted syngeneic mouse model by test compounds

| Group | 0 | 2 | 5 | 7 | 9 | 12 | 14 | 16 | 19 |
|---|---|---|---|---|---|---|---|---|---|
| G2 | 0.00% | 4.93% | 32.03% | 38.87% | 58.42% | 58.26% | 57.24% | 49.47% | 39.88% |
| G3 | 0.00% | 14.79% | 59.92% | 78.89% | 87.33% | 89.91% | 89.21% | 87.33% | 84.92% |
| Note: $TGI_{TV}$ was calculated relative to G1. | | | | | | | | | |

Table 6. Statistical analysis of tumor volume in different groups of CT26 (CT26-hPDL1/hB7H3)-implanted syngeneic mouse model by test compounds

| Between-group comparison | D0 | D2 | D5 | D7 | D9 | D12 | D14 | D16 | D19 |
|---|---|---|---|---|---|---|---|---|---|
| G1 VS G2 | 0.954 | 0.693 | 0.126 | 0.092 | 0.020* | 0.016* | 0.013* | 0.022* | 0.013* |
| G1 VS G3 | 0.979 | 0.198 | 0.016* | 0.005** | 0.005** | 0.001** | 0.001** | 0.001** | <0.001*** |
| G2 VS G3 | 0.976 | 0.359 | 0.027* | 0.010* | <0.001*** | 0.002** | 0.004** | 0.001** | 0.002** |
| Note: Independent samples t-test was used, *: $p < 0.05$; **: $p < 0.01$; ***: $p < 0.001$. | | | | | | | | | |

The experimental results demonstrated that, compared to DSYE002-X2, the bispecific antibody-drug conjugate DSYE001-X2 of the present disclosure exhibited significantly enhanced tumor growth inhibiting activity after dosing.

**Examples 8: Evaluation of the efficacy of antibody-drug conjugates in NCI-H1975 human non-small cell lung cancer xenograft mice**

To investigate the inhibitory effect of DSYE001-X1 (DAR4) on tumor formation *in vivo,* a subcutaneous xenograft model was established in mice co-implanted with NCI-H1975 human lung cancer cells and human PBMCs to evaluate the *in vivo* anti-tumor efficacy of DSYE001-X1 (DAR4).

NCG mice, female, 6 to 8 weeks old, weighing approximately 18 to 22 g, purchased from GemPharmatech Co., Ltd. (Jiangsu, China). NCI-H1975 cells were cultured in RPMI 1640 medium containing 10% fetal bovine serum (FBS) in an incubator at 37°C with 5% $CO_2$. NCI-H1975 cells in the logarithmic growth phase were harvested and resuspended in HBSS to an appropriate concentration for subcutaneous tumor inoculation in NCG mice. Cryopreserved PBMCs were purchased, thawed, and counted. The resuspended PBMCs were co-cultured with NCI-H1975 cells for 5 days in RPMI 1640 medium containing IL-2 and 10% FBS.

After 5 days of co-culture of PBMCs and NCI-H1975 cells, PBMCs ($3 \times 10^5$ cells) and freshly digested NCI-H1975 cells ($2 \times 10^6$ cells) were harvested and subcutaneously inoculated into the right flank of NCG mice at 0.2 mL/mouse (containing 50% Matrigel). When the tumor volume reached approximately 100 to 200 $mm^3$, 20 mice were selected and randomly divided into 4 groups based on tumor volume, with 5 mice per group. On the day of grouping (day 0), isotype control ADC, DSYE001-X1 (DAR4), reference ADC-DSYE002-X1 (DAR4), and DSYE002-X1 (DAR4) in combination with PDL1 monoclonal antibody were administered via intravenous (i.v.) injection twice weekly for 2 weeks for a total of 4 doses. Specifically, the bispecific ADC was administered at a dose of 18 mg/kg, while the isotype control ADC and PDL1 monoclonal antibody were administered at a dose of 15 mg/kg (equimolar dose). The experiment was terminated on day 28. The experimental groups and dosing regimen were as follows. Tumor volume and body weight were measured twice weekly, and data were recorded.

Isotype control ADC: 15 mg/kg

DSYE001-X1 (DAR4) (treatment group): 18 mg/kg
DSYE002-X1 (DAR4) (treatment group): 8 mg/kg
DSYE002-X1 (reference ADC-2, DAR4) + anti-PD-L1 monoclonal antibody DSYE004 (treatment group): 8 mg/kg + 15 mg/kg

All samples were diluted with PBS.

Relative tumor proliferation rate T/C (%) was calculated using the formula as follows: T/C % = TRTV / CRTV × 100% (TRTV: RTV of the treatment group; CRTV: RTV of the negative control group). Relative tumor volume (RTV) was calculated based on tumor measurements using the formula as follows: RTV = Vt - V0, where V0 represents the mean tumor volume measured at the time of grouping (i.e., D0), and Vt represents the mean tumor volume at a specific measurement. TRTV and CRTV were collected on the same day.

Tumor growth inhibition rate TGI (%) = (1 - T/C) × 100%. T/C % represents the relative tumor proliferation rate, i.e., the percentage of the tumor volume for the treatment group relative to the control group at a specific time point. T and C represent the tumor volume (TV) for the treatment group and the control group at a specific time point, respectively.

At the end of the experiment, the mice were euthanized, and the tumor weight was measured.

The experimental results are as shown in FIG. 19 and Tables 7 to 8.

Table 7. Mean tumor volume of mice in each group (Mean ± SEM)

| Group | Experimental group | Tumor volume ($mm^3$) | | TGI % | T/C % | *P*-value |
|---|---|---|---|---|---|---|
| | | Day 0 | Day 28 | | | |
| 1 | Isotype control ADC, 15 mg/kg | 105.77±3.84 | 1519.89±53.97 | -- | -- | -- |
| 2 | DSYE001-X1 (DAR4), 18 mg/kg | 105.34±3.50 | 511.91±133.60** | 71.25 | 28.75 | 0.003 |
| 3 | DSYE002-X1 (DAR4), 8 mg/kg | 105.46±3.36 | 1502.53±296.94 | 1.21 | 98.79 | 0.951 |
| 4 | DSYE002-X1 (DAR4), 8 mg/kg + PDL1 mAb, 15 mg/kg | 105.57±3.29 | 633.87±136.34** | 62.64 | 37.36 | 0.009 |
| Note: Compared to the isotype control ADC, 15 mg/kg control group, ***p* < 0.01 and **p* < 0.05 are considered statistically significant. | | | | | | |

Table 8. Mean tumor weight of mice in each group (Mean ± SEM)

| Group | Experimental group | Number of animals Start/End | Tumor weight (g) | T/C% | *P*-value |
|---|---|---|---|---|---|
| 1 | Isotype control ADC, 15 mg/kg | 5/4 | 1.521±0.053 | -- | -- |
| 2 | DSYE001-X1 (DAR4), 18 mg/kg | 5/5 | 0.505±0.136** | 33.22 | 0.002 |
| 3 | DSYE002-XI (DAR4), 8 mg/kg | 5/5 | 1.494±0.292 | 98.24 | 0.923 |
| 4 | DSYE002-X1 (DAR4), 8 mg/kg + PDL1 mAb, 15 mg/kg | 5/4 | 0.629±0.130** | 41.33 | 0.008 |
| Note: Compared to the isotype control ADC, 15 mg/kg control group, ***p* < 0.01 and **p* < 0.05 are considered statistically significant. | | | | | |

The results demonstrated that DSYE001-X1 significantly inhibited tumor growth, exhibiting stronger anti-tumor activity than the B7H3 monoclonal antibody ADC (DSYE002-X1) and superior tumor suppression efficacy compared to the combination of B7H3 monoclonal antibody ADC and PDL1 monoclonal antibody.

**Examples 9: Evaluation of the efficacy of antibody-drug conjugates in MDA-MB-231 human breast cancer xenograft mice**

To investigate the inhibitory effect of DSYE001-X1 (DAR6) on tumor formation *in vivo,* a subcutaneous xenograft

model was established in mice co-implanted with MDA-MB-231 human breast cancer cells and human PBMCs to evaluate the *in vivo* anti-tumor efficacy of DSYE001-X1 (DAR6).

Female NCG mice aged 6 to 8 weeks (purchased from GemPharmatech Co., Ltd. (Jiangsu, China)) were used as experimental animals. MDA-MB-231 cells (provided by GemPharmatech, with negative mycoplasma test results) were thawed and passaged, with passage N+13 after thawing. MDA-MB-231 cells in the logarithmic growth phase (passage N+17) were harvested. The culture medium was removed, and the cells were washed twice with DPBS before inoculation (cell viability before and after inoculation: 99.27% and 97.00%, respectively). Mice were inoculated with the cells at $5 \times 10^6$ cells/100 μL/mouse (mixed 1:1 with Yeasen Matrigel). One week after tumor cell inoculation, human PBMCs were administered via tail vein injection at a dose of $7.5 \times 10^6$ cells/100 μL/mouse. On day 9 post-inoculation, when the mean tumor volume reached 85.44 $mm^3$, 12 mice were selected and randomly divided into 2 groups based on tumor volume, with 6 mice per group. The day of grouping was defined as D0, and dosing was initiated on D0, with dosing dates on D0 and D7. The experiment was terminated on day 41. Tumor volume and body weight were measured twice weekly, and data were recorded.

At the end of the experiment, the mice were euthanized, and $TGI_{TV}$ (relative tumor inhibition rate) was calculated. $TGI_{TV}$ (relative tumor inhibition rate) was calculated using the formula as follows:

$$TGI_{TV} = \left(1 - \frac{mean\ RTV_{treat}}{mean\ RTV_{vehicle}}\right) \times 100\%$$

where $mean\ RTV_{treat}$ represents the mean RTV of the treatment group;
$mean\ RTV_{vehicle}$ represents the mean RTV of the vehicle group (in this example, the group administered with normal saline alone);
the RTV was calculated using the formula as follows:

$$RTV_n = \frac{V_{nt}}{V_{n0}}$$

where $V_{nt}$ represents the tumor volume of the mouse numbered n on day t;
$V_{n0}$ represents the tumor volume of the mouse numbered n on day 0;
$RTV_n$ represents the relative tumor volume of the mouse numbered n at day t.

The experimental results are as shown in FIG. 20, Table 9, and Table 10.

Table 9. Changes in tumor volume in different groups

| Group | Tumor volume ($mm^3$) | | | | | | | | | | | | |
|---|---|---|---|---|---|---|---|---|---|---|---|---|---|
| | D0 | D3 | D6 | D10 | D13 | D17 | D20 | D24 | D27 | D31 | D34 | D38 | D41 |
| Normal saline | 85.10 ±3.12 | 121.41 ±6.48 | 168.82 ±10.24 | 239.3 9 ±17.15 | 312.8 5 ±20.60 | 363.5 1 ±27.61 | 479.3 7 ±49.62 | 570.4 6 ±97.98 | 646.4 4 ±131.03 | 832.1 5 ±157.72 | 936.6 1 ±167.01 | 1088.8 6 ±177.21 | 1222.9 1 ±201.13 |
| DSYE001-X1 (DAR6) | 85.15 ±3.37 | 136.70 ±4.38 | 138.64 ±4.96 | 87.46 ±3.64 | 69.63 ±3.99 | 51.06 ±6.15 | 36.40 ±3.04 | 31.52 ±2.72 | 28.77 ±2.55 | 32.27 ±4.06 | 39.76 ±6.59 | 58.82 ±15.98 | 78.66 ±21.64 |

Table 10. Relative tumor inhibition rate ($TGI_{TV}$) in different groups

| Group | D0 | D3 | D6 | D10 | D13 | D17 | D20 | D24 | D27 | D31 | D34 | D38 | D41 |
|---|---|---|---|---|---|---|---|---|---|---|---|---|---|
| Normal saline | - | - | - | - | - | - | - | - | - | - | - | - | - |
| DSYE001-X1 (DAR6) | 0.00 % | 13.04 % | 15.82 % | 62.76 % | 77.21 % | 85.63 % | 92.29 % | 94.35 % | 95.45 % | 96.06 % | 95.73 % | 94.60 % | 94.69 % |

The results demonstrated that DSYE001-X1 significantly inhibited the growth of breast cancer cells.

**Examples 10: Evaluation of the efficacy of antibody-drug conjugates in NCI-H1975 human non-small cell lung cancer xenograft mice**

To investigate the inhibitory effect of DSYE001-X1 (DAR6) on tumor formation *in vivo,* a subcutaneous xenograft model was established in mice co-implanted with NCI-H1975 human lung cancer cells and human PBMCs to evaluate the *in vivo* anti-tumor efficacy of DSYE001-X1 (DAR6).

NCG mice, female, 6 to 8 weeks old, weighing approximately 18 to 22 g, purchased from GemPharmatech Co., Ltd. (Jiangsu, China). NCI-H1975 cells were cultured in RPMI 1640 medium containing 10% fetal bovine serum (FBS) in an incubator at 37°C with 5% $CO_2$. NCI-H1975 cells in the logarithmic growth phase were harvested and resuspended in HBSS to an appropriate concentration for subcutaneous tumor inoculation in NCG mice. Cryopreserved PBMCs were purchased, thawed, and counted. The resuspended PBMCs were co-cultured with NCI-H1975 cells for 5 days in RPMI 1640 medium containing IL-2 and 10% FBS.

After 5 days of co-culture of PBMCs and NCI-H1975 cells, PBMCs ($3 \times 10^5$ cells) and freshly digested NCI-H1975 cells ($2 \times 10^6$ cells) were harvested and subcutaneously inoculated into the right flank of NCG mice at 0.2 mL/mouse (containing 50% Matrigel). When the tumor volume reached approximately 100 to 200 $mm^3$, 15 mice were selected and randomly divided into 3 groups based on tumor volume, with 5 mice per group. On the day of grouping (day 0), DSYE001-X1 (DAR6) and DSYE003-X1 (DAR6) in combination with Pembrolizumab were administered via intravenous (i.v.) injection twice weekly for 2 weeks for a total of 4 doses. Specifically, the bispecific ADC was administered at a dose of 18 mg/kg, while the isotype control ADC and Pembrolizumab were administered at a dose of 15 mg/kg (equimolar dose). The experiment was terminated on day 21. The experimental groups and dosing regimen were as follows. Tumor volume and body weight were measured twice weekly, and data were recorded.

Isotype control ADC: 15 mg/kg
DSYE001-X1 (DAR6) (treatment group): 18 mg/kg
DSYE003-X1 (reference ADC-1, DAR6) + Pembrolizumab (treatment group): 18 mg/kg + 15 mg/kg

All samples were diluted with PBS. Pembrolizumab was prepared using conventional methods, with the sequence derived from Recommended INN list R72 (2014).

Relative tumor proliferation rate T/C (%) was calculated using the formula as follows: T/C % = TRTV / CRTV $\times$ 100% (TRTV: RTV of the treatment group; CRTV: RTV of the negative control group). Relative tumor volume (RTV) was calculated based on tumor measurements using the formula as follows: RTV = Vt - V0, where V0 represents the mean tumor volume measured at the time of grouping (i.e., d0), and Vt represents the mean tumor volume at a specific measurement. TRTV and CRTV were collected on the same day.

Tumor growth inhibition rate TGI (%) = (1 - T/C) $\times$ 100%. T/C % represents the relative tumor proliferation rate, i.e., the percentage of the tumor volume for the treatment group relative to the control group at a specific time point. T and C represent the tumor volume (TV) for the treatment group and the control group at a specific time point, respectively.

At the end of the experiment, the mice were euthanized, and the tumor weight was measured.

The experimental results are as shown in FIG. 21 and Tables 11 to 12.

Table 11. Mean tumor volume of mice in each group (Mean $\pm$ SEM)

| Group | Experimental group | Tumor volume ($mm^3$) | | TGI% | T/C% | P-value |
|---|---|---|---|---|---|---|
| | | Day 0 | Day 21 | | | |
| 1 | Isotype control ADC, 15 mg/kg | 128.12±6.30 | 1514.14±225.88 | -- | -- | -- |
| 2 | DSYE001-X1 (DAR6), 18 mg/kg | 127.83±5.50 | 355.00±123.21**** | 83.61 | 16.39 | <0.0001 |

(continued)

| Group | Experimental group | Tumor volume ($mm^3$) | | TGI% | T/C% | P-value |
|---|---|---|---|---|---|---|
| | | Day 0 | Day 21 | | | |
| 3 | DSYE003-X1 (DAR6), 18 mg/kg + Pembrolizumab, 15 mg/kg | 127.91±6.80 | 520.47±111.70**** | 71.68 | 28.32 | <0.0001 |
| Note: Compared to the isotype control ADC, 15 mg/kg control group, ****p < 0.0001 is considered statistically significant. | | | | | | |

Table 12. Mean tumor weight of mice in each group (Mean ± SEM)

| Group | Experimental group | Number of animals Start/End | Tumor weight (g) | T/C% | *P*-value |
|---|---|---|---|---|---|
| 1 | Isotype control ADC, 15 mg/kg | 5/5 | 1.552±0.234 | -- | -- |
| 2 | DSYE001-X1 (DAR6), 18 mg/kg | 5/5 | 0.376±0.132**** | 24.24 | <0.0001 |
| 3 | DSYE003-X1 (DAR6), 18 mg/kg + Pembrolizumab, 15 mg/kg | 5/5 | 0.539±0.117**** | 34.70 | <0.0001 |
| Note: Compared to the isotype control ADC, 15 mg/kg control group, ****$p$ < 0.0001 is considered statistically significant. | | | | | |

The results demonstrated that DSYE001-X1 significantly inhibited tumor growth, exhibiting superior tumor suppression efficacy compared to the combination of B7H3 monoclonal antibody ADC and Pembrolizumab.

**Example 11: Pharmacokinetic and toxicity study of antibody-drug conjugates following multiple dosing**

Test purpose

DSYE001-X1 (DAR6) was intravenously administered to cynomolgus monkeys once every 3 weeks for a total of 2 doses. The nature, extent, dose-effect, and time-effect relationships of toxicity reactions potentially induced by antibody-drug conjugates were observed to identify target organs or tissues of toxicity, providing references for subsequent studies.

Experimental method

A total of 4 cynomolgus monkeys (2 animals per sex) were used in the experiment, randomly divided into 3 groups based on body weight, with 1 animal per sex per group. Animals in group 1 and group 2 were administered 30 mg/kg and 120 mg/kg of the sample (DSYE001-X1 (DAR6)), representing the low-dose and high-dose groups, respectively. The animals were intravenously administered at a dose of 10 mL/kg over approximately 30 minutes, once every 3 weeks for a total of 2 doses over a 3-week dosing period.

During the experiment, clinical observations were conducted on animals, including body weight, food intake, body temperature, electrocardiogram, and clinical pathology (blood cell count, coagulation function, and blood biochemistry). One week after the last dose (D29), the animals were euthanized for gross anatomy and histopathological examinations (gross abnormalities, bone, and bone marrow). Additionally, the concentrations of total antibody protein and DSYE001-X1 (DAR6) in serum, as well as small-molecule concentrations in plasma, were measured in animals dosed on D1 and D22 for toxicokinetic analysis.

Table 13. Toxicokinetic parameters

| Analyte | Test schedule | Group / Dose | | Sex/number of animals | $t_{1/2}$ (h) | $T_{max}$ (h) | $C_{max}$ (μg/mL) | $AUC_{last}$ (h·mg/mL) |
|---|---|---|---|---|---|---|---|---|
| DSYE001-X1 (DAR6) | D1 | Low-dose group 30 mg/kg | | Male/N = 1 | 32.5 | 0.500 | 776 | 49.4 |
| | | | | Female/N = 1 | 23.5 | 0.500 | 605 | 35.7 |
| | | High-dose group 120 mg/kg | | Male/N = 1 | 47.8 | 0.500 | 4360 | 320 |
| | | | | Female/N = 1 | 135 | 0.500 | 3170 | 289 |
| DSYE001 total antibody | D1 | Low-dose group 30 mg/kg | | Male/N = 1 | 31.1 | 0.500 | 791 | 48.3 |
| | | | | Female/N = 1 | 20.8 | 0.500 | 621 | 36.3 |
| | | High-dose group 120 mg/kg | | Male/N = 1 | 53.1 | 0.500 | 4300 | 356 |
| | | | | Female/N = 1 | 153 | 0.500 | 3220 | 329 |
| **Analyte** | **Test schedule** | **Group / Dose** | | **Sex/number of animals** | **$t_{1/2}$ (h)** | **$T_{max}$ (h)** | **$C_{max}$ (ng/mL)** | **$AUC_{last}$ (h·ng/mL)** |
| X1 | D1 | Low-dose group 30 mg/kg | | Male/N = 1 | NA | 48.0 | 1.36 | 204 |
| | | | | Female/N = 1 | 383 | 4.00 | 2.01 | 281 |
| | | High-dose group 120 mg/kg | | Male/N = 1 | 190 | 4.00 | 6.70 | 818 |
| | | | | Female/N = 1 | 124 | 4.00 | 5.79 | 678 |

Experimental conclusion

During the experiment, no mortality or moribundity was observed in the animals across all dose groups, and no abnormalities related to the sample were observed in terms of body weight, food intake, body temperature, electrocardiogram parameters and waveforms, clinical pathology (blood cell count, coagulation function, and blood biochemistry), gross anatomy, and histopathological examinations (bone and bone marrow). These results indicate that the antibody-drug conjugate has a favorable safety profile.

**Examples 12: Evaluation of the efficacy of antibody-drug conjugate DSYE001-X1 (DAR8) in syngeneic mouse model**

To investigate the inhibitory effect of DSYE001-X1 (DAR8) on tumor growth, a pharmacodynamic evaluation was conducted in BALB/c-hPD-L1/hB7H3 mice, with the specific method referring to Example 7.

The antibody-drug conjugate DSYE001-X1 of the present disclosure demonstrated superior anti-tumor efficacy compared to B7H3 monoclonal antibody ADC or the combination of B7H3 monoclonal antibody ADC and PD-L1 antibody.

Various modifications and variations of the methods and systems according to the present disclosure will be apparent to those skilled in the art without departing from the scope and spirit of the present disclosure. Although the present disclosure has been described in connection with specific preferred embodiments, it should be understood that the present disclosure as claimed should not be limited to such specific embodiments. Indeed, various modifications of the described modes for implementing the present disclosure, as will be apparent to those skilled in molecular biology, immunology, or related fields, are intended to fall within the scope of the appended claims.

**Sequence listing**

Amino acid sequence of DSYE001 (underlined portions indicate CDRs)
Light chain variable region of monoclonal antibody:

DIQMTQSPSSLSASVGDRVTISCRASQSINTYLHWYQQKLGQAPRLLIYYAS QSISGVPSRFSGSGSGTDFTLTINSLEAEDAATYYCQNGHSFPLTFGAGTKVEIK (SEQ ID NO.: 4)

LCDR1: **RASQSINTYLH** (SEQ ID NO.: 1)
LCDR2: **YASQSIS** (SEQ ID NO.: 2)
LCDR3: **QNGHSFPLT** (SEQ ID NO.: 3)

Heavy chain variable region of monoclonal antibody:

QVQLVQSGAEVKKPGASVKVSCKASGYTFTTFGVHWVRQAPGKGLEWVGI IWPGGNTNYNSALMSRVTITADESTSTAYMELSSLRSEDTAVYYCARENYGRAMDY WGQGTTVTVSS (SEQ ID NO.: 8)

HCDR1: **TFGVH** (SEQ ID NO.: 5)
HCDR2: **IIWPGGNTNYNSALMS** (SEQ ID NO.: 6)
HCDR3: **ENYGRAMDY** (SEQ ID NO.: 7)

Heavy chain variable region of nanobody:

DVQLQESGGGLVQAGGSLRLSCTASGYTLSTIWIGWFRQAPGKGLEGVAAI YIGSGATYYVDSVKGRFTISQDNAKNTVYLQMNSLKPEDTAMYYCAATGGTVGSM VRFSPVGDFGYWGQGTQVTVSS (SEQ ID NO.: 15)

HCDR1: **TIWIG** (SEQ ID NO.: 12)
HCDR2: **AIYIGSGATYYVDSVKG** (SEQ ID NO.: 13)
HCDR3: **TGGTVGSMVRFSPVGDFGY** (SEQ ID NO.: 14)
Linker peptide between monoclonal antibody and nanobody:
GGGGSGGGGTGGGGS (SEQ ID NO.: 11)
Heavy chain of monoclonal antibody:

QVQLVQSGAEVKKPGASVKVSCKASGYTFTTFGVHWVRQAPGKGLEWVGI IWPGGNTNYNSALMSRVTITADESTSTAYMELSSLRSEDTAVYYCARENYGRAMDY WGQGTTVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEK TISKAKGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSLSPGK (SEQ ID NO.:10)

Light chain of monoclonal antibody:

DIQMTQSPSSLSASVGDRVTISCRASQSINTYLHWYQQKLGQAPRLLIYYASQSISGVPSRFSGSGSGTDFTLTINSLEAEDAATYYCQNGHSFPLTFGAGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO.: 9)

Heavy chain of bispecific antibody:

QVQLVQSGAEVKKPGASVKVSCKASGYTFTTFGVHWVRQAPGKGLEWVGIIWPGGNTNYNSALMSRVTITADESTSTAYMELSSLRSEDTAVYYCARENYGRAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

GGGGSGGGGTGGGGSDVQLQESGGGLVQAGGSLRLSCTASGYTLSTIWIGWFRQAPGKGLEGVAAIYIGSGATYYVDSVKGRFTISQDNAKNTVYLQMNSLKPEDTAMYYCAATGGTVGSMVRFSPVGDFGYWGQGTQVTVSS (SEQ ID NO.: 16)

Light chain of bispecific antibody:

DIQMTQSPSSLSASVGDRVTISCRASQSINTYLHWYQQKLGQAPRLLIYYASQSISGVPSRFSGSGSGTDFTLTINSLEAEDAATYYCQNGHSFPLTFGAGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO.: 9)

Amino acid sequence of DSYE002 (reference antibody)

DVQLQESGGGLVQAGGSLRLSCTASGYTLS**TIWIG**WFRQAPGKGLEGVA**AIYIGSGATYYVDSVKG**RFTISQDNAKNTVYLQMNSLKPEDTAMYYCAA**TGGTVGSMVRFSPVGDFGY**WGQGTQVTVSSGSEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO.: 17)

Heavy chain of DSYE003

QVQLVQSGAEVKKPGASVKVSCQASGYRFSNFVIHWVRQAPGQRFEWMG WINPYNGNKEFSAKFQDRVTFTADTSANTAYMELRSLRSADTAVYYCARVGPYSW DDSPQDNYYMDVWGKGTTVIVSSASTKGPSVFPLAPSSKSTSGGTAALGCLVKDYFP EPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALHNH YTQKSLSLSPGKGGGGSGGGGTGGGGSDVQLQESGGGLVQAGGSLRLSCTASGYTL STIWIGWFRQAPGKGLEGVAAIYIGSGATYYVDSVKGRFTISQDNAKNTVYLQMNSL KPEDTAMYYCAATGGTVGSMVRFSPVGDFGYWGQGTQVTVSS (SEQ ID NO.: 18)

Light chain of DSYE003 (Human Anti-HIV-1 gp120 clone b12 light chain)

EIVLTQSPGTLSLSPGERATFSCRSSHSIRSRRVAWYQHKPGQAPRLVIHGVS

NRASGISDRFSGSGSGTDFTLTITRVEPEDFALYYCQVYGASSYTFGQGTKLERKRTV AAPSVFIFPPSDEQLKSGTASVVCLLNNFYPREAKVQWKVDNALQSGNSQESVTEQD SKDSTYSLSSTLTLSKADYEKHKVYACEVTHQGLSSPVTKSFNRGEC (SEQ ID NO.: 19)

Amino acid sequence of PD-L1 monoclonal antibody (DSYE004)
Heavy chain of PD-L1 monoclonal antibody

QVQLVQSGAEVKKPGASVKVSCKASGYTFTTFGVHWVRQAPGKGLEWVGI IWPGGNTNYNSALMSRVTITADESTSTAYMELSSLRSEDTAVYYCARENYGRAMDY WGQGTTVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEK TISKAKGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSLSPGK (SEQ ID NO.: 20)

Light chain of PD-L1 monoclonal antibody: same light chain as bispecific antibody
Amino acid sequence of Pembrolizumab
Heavy chain

QVQLVQSGVEVKKPGASVKVSCKASGYTFTNYYMYWVRQAPGQGLEWMGGINPSNGGTNFNEKFKNRVTLTTDSSTTTAYMELKSLQFDDTAVYYCARRDYRFDMGFDYWGQGTTVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK (SEQ ID NO.: 21)

Light chain

EIVLTQSPATLSLSPGERATLSCRASKGVSTSGYSYLHWYQQKPGQAPRLLIYLASYLESGVPARFSGSGSGTDFTLTISSLEPEDFAVYYCQHSRDLPLTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO.: 22)

## Claims

1. A bispecific antibody-drug conjugate having a structure comprising the following fragments: an anti-B7H3 and PD-L1 bispecific antibody or an antigen-binding fragment thereof, a linker unit L, and a cytotoxic drug, wherein the bispecific antibody or the antigen-binding fragment thereof comprises:

    a monoclonal antibody unit targeting PD-L1 and comprising two heavy chains and two light chains,
    a nanobody unit targeting B7H3 and comprising two identical nanobodies,
    wherein the N-terminus of each of the two nanobodies is connected to the C-terminus of each of the Fc fragments of the two heavy chains of the monoclonal antibody unit via a linker peptide.

2. The bispecific antibody-drug conjugate according to claim 1, wherein
the light chain variable region of the monoclonal antibody unit comprises a CDR1 with an amino acid sequence of SEQ ID NO.: 1, a CDR2 with an amino acid sequence of SEQ ID NO.: 2, and a CDR3 with an amino acid sequence of SEQ ID NO.: 3; the heavy chain variable region of the monoclonal antibody unit comprises a CDR1 with an amino acid sequence of SEQ ID NO.: 5, a CDR2 with an amino acid sequence of SEQ ID NO.: 6, and a CDR3 with an amino acid sequence of SEQ ID NO.: 7; and the nanobody comprises a CDR1 with an amino acid sequence of SEQ ID NO.: 12, a CDR2 with an amino acid sequence of SEQ ID NO.: 13, and a CDR3 with an amino acid sequence of SEQ ID NO.: 14.

3. The bispecific antibody-drug conjugate according to claim 1 or 2, wherein
the light chain variable region of the monoclonal antibody unit comprises an amino acid sequence as shown in SEQ ID NO.: 4; the heavy chain variable region of the monoclonal antibody unit comprises an amino acid sequence as shown

in SEQ ID NO.: 8; and the nanobody comprises an amino acid sequence as shown in SEQ ID NO.: 15.

**4.** The bispecific antibody-drug conjugate according to any one of claims 1 to 3, wherein the monoclonal antibody comprises an immunoglobulin constant region, wherein the immunoglobulin constant region is a human IgG constant region, such as a human IgG1 constant region.

**5.** The bispecific antibody-drug conjugate according to any one of claims 1 to 4, wherein

the light chain of the monoclonal antibody unit comprises an amino acid sequence as shown in SEQ ID NO.: 9; the heavy chain of the monoclonal antibody unit comprises an amino acid sequence as shown in SEQ ID NO.: 10; and the nanobody comprises an amino acid sequence as shown in SEQ ID NO.: 15; or
the full-length amino acid sequence of the light chain of the monoclonal antibody unit is as shown in SEQ ID NO.: 9; the full-length amino acid sequence of the heavy chain of the monoclonal antibody unit is as shown in SEQ ID NO.: 10; and the amino acid sequence of the nanobody is as shown in SEQ ID NO.: 15.

**6.** The bispecific antibody-drug conjugate according to any one of claims 1 to 5, wherein the linker peptide is a polypeptide comprising glycine and serine with certain elasticity and protease resistance; preferably, the amino acid sequence of the linker peptide is SEQ ID NO.: 11.

**7.** The bispecific antibody-drug conjugate according to any one of claims 1 to 6, wherein the heavy chain amino acid sequence of the bispecific antibody is as shown in SEQ ID NO.: 16, and the light chain amino acid sequence is as shown in SEQ ID NO.: 9.

**8.** The bispecific antibody-drug conjugate according to any one of claims 1 to 7, wherein the cytotoxic drug is a structure represented by formula (A-1), a tautomer, enantiomer, diastereomer, or mixture of isomers thereof, or a pharmaceutically acceptable salt or solvate thereof,

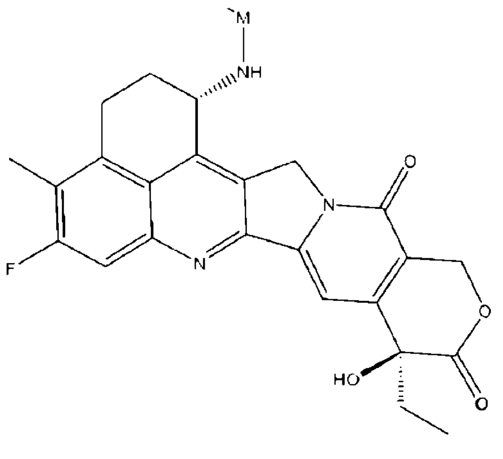

(A-1)

wherein

M is $-L^2-L^1-C(O)-$;
$L^2$ is -O- or -S-, preferably -O-, and $L^2$ is connected to the linker unit L;
$L^1$ is $-(C(R^{1a})(R^{1b}))_m-CH^{2}-$, $C_3$-$C_6$ saturated cycloalkyl, or 3- to 6-membered saturated heterocyclyl, wherein the $C_3$-$C_6$ saturated cycloalkyl and 3- to 6-membered saturated heterocyclyl are each independently and optionally substituted by one or more $R^{2a}$;
m is 1, 2, 3, or 4; heteroatoms in the 3- to 6-membered saturated heterocyclyl are each independently N, O, or S, and the number of heteroatoms is 1, 2, or 3;
$R^{1a}$ and $R^{1b}$ are each independently hydrogen, halogen, hydroxyl, amino, or $C_1$-$C_6$ alkyl, wherein the $C_1$-$C_6$ alkyl is optionally substituted by one or more halogens;
$R^{2a}$ is halogen, hydroxyl, amino, or $C_1$-$C_6$ alkyl, wherein the $C_1$-$C_6$ alkyl is optionally substituted by one or more halogens.

**9.** The bispecific antibody-drug conjugate according to claim 8, wherein

$L^1$ is $-(C(R^{1a})(R^{1b}))_m-CH_2-$; each $R^{1a}$ is independently hydrogen, halogen, or $C_1$-$C_6$ alkyl; each $R^{1b}$ is independently hydrogen, halogen, or $C_1$-$C_6$ alkyl;
preferably, $L^1$ is $-(C(R^{1a})(R^{1b}))_m-CH_2-$; each $R^{1a}$ is independently $C_1$-$C_6$ alkyl, preferably $C_1$-$C_3$ alkyl; each $R^{1b}$ is independently hydrogen or $C_1$-$C_6$ alkyl, preferably hydrogen or $C_1$-$C_3$ alkyl;
preferably, $L^1$ is $-(C(R^{1a})(R^{1b}))_m-CH_2-$; $R^{1a}$ is $-CH_3$; each $R^{1b}$ is independently hydrogen or $-CH_3$;
preferably, $L^1$ is

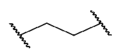, 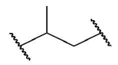, 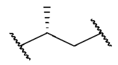, 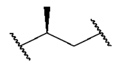, or 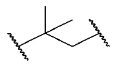;

and/or, the cycloalkyl, heterocyclyl, and alkyl are each independently unsubstituted; and/or, the atoms in the bispecific antibody-drug conjugate are atoms of natural abundance.

**10.** The bispecific antibody-drug conjugate according to claim 8, wherein

$L^1$ is $C_3$-$C_6$ saturated cycloalkyl or 3- to 6-membered saturated heterocyclyl, preferably, $L^1$ is $C_3$-$C_6$ saturated cycloalkyl, wherein the $C_3$-$C_6$ saturated cycloalkyl and 3- to 6-membered saturated heterocyclyl are each independently and optionally substituted by one or more $R^{2a}$, and each $R^{2a}$ is independently halogen or $C_1$-$C_6$ alkyl;
preferably, $L^1$ is cyclopropyl, cyclobutyl, cyclopentyl, or cyclohexyl, wherein the cyclopropyl, cyclobutyl, cyclopentyl, and cyclohexyl are optionally substituted by one or more $R^{2a}$, and each $R^{2a}$ is independently halogen or $C_1$-$C_6$ alkyl;
preferably, $L^1$ is

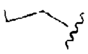, 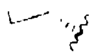, 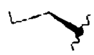, 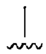, 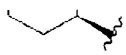, 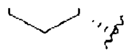,

or

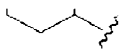.

**11.** The bispecific antibody-drug conjugate according to claim 8, wherein in the structure represented by formula (A-1),

M is $-L^2-L^1-C(O)-$;
$L^2$ is -O-;
$L^1$ is $-(C(R^{1a})(R^{1b}))_m-CH_2-$ or $C_3$-$C_6$ saturated cycloalkyl, wherein the $C_3$-$C_6$ saturated cycloalkyl is optionally substituted by one or more $R^{2a}$;
m is 1 or 2;
$R^{1a}$ and $R^{1b}$ are each independently selected from the group consisting of hydrogen, halogen, and $C_1$-$C_6$ alkyl, wherein the $C_1$-$C_6$ alkyl is optionally substituted by one or more halogens;
each $R^{2a}$ is independently selected from the group consisting of halogen and $C_1$-$C_6$ alkyl, wherein the $C_1$-$C_6$ alkyl is optionally substituted by one or more halogens.

**12.** The bispecific antibody-drug conjugate according to claim 8, wherein the cytotoxic drug is selected from the group consisting of any one of the following structures:

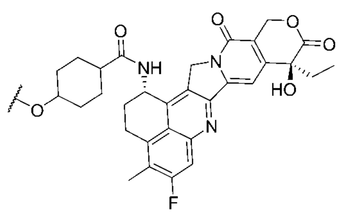, 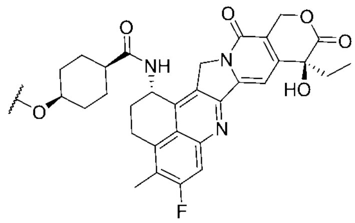, 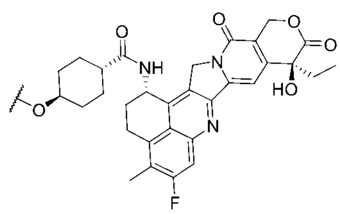,

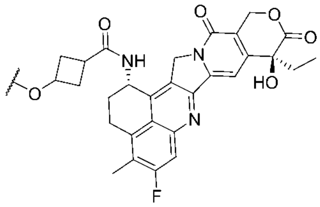, 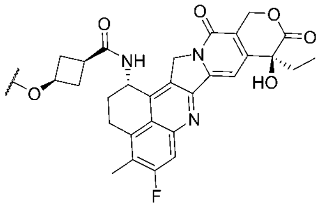, 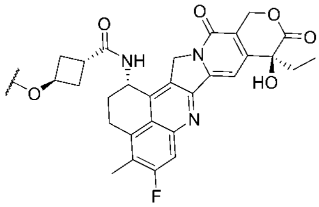,

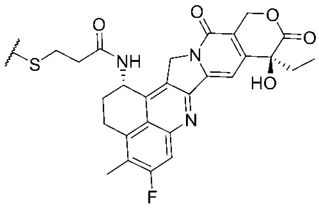, 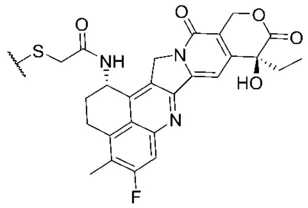, 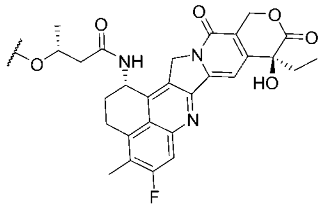,

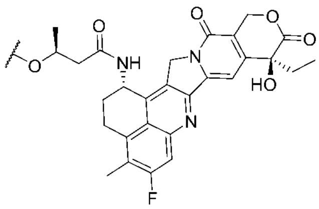, 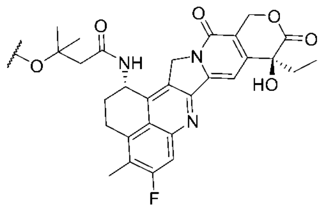, and 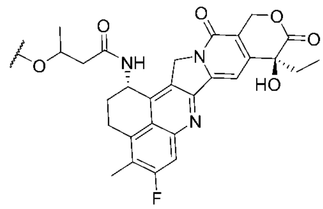.

**13.** The bispecific antibody-drug conjugate according to any one of claims 1 to 12, wherein the linker unit L is -$L_a$-$L_b$-$L_c$-; and the $L_c$ is connected to the cytotoxic drug;

- $L_a$- is

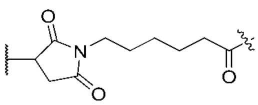 or 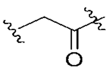;

- $L_b$- is selected from the group consisting of any one of the following structures:

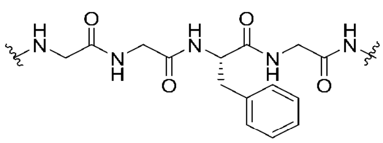, 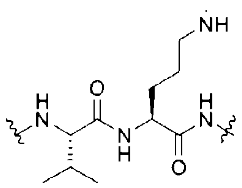, 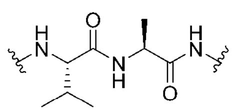,

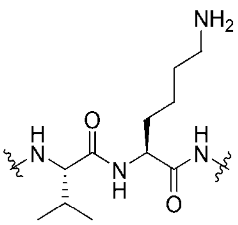, 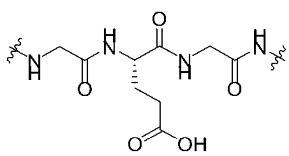, 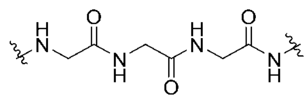,

and

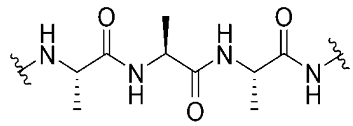

;

preferably

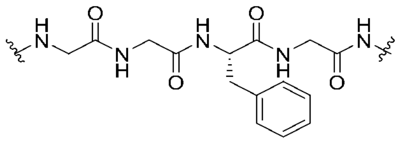

or

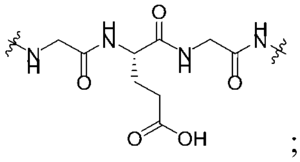

;

- $L_c$- is

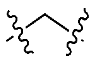

.

**14.** The bispecific antibody-drug conjugate according to claim 13, wherein the linker unit L is

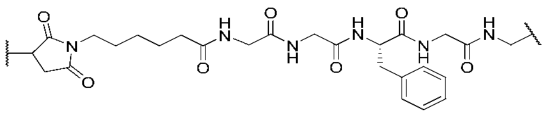

,

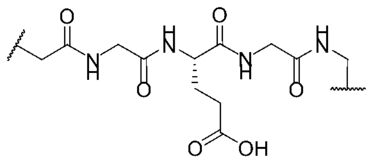

,

or

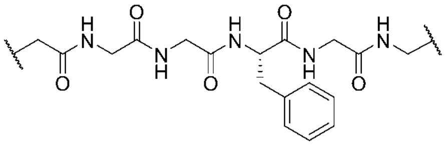

;

preferably

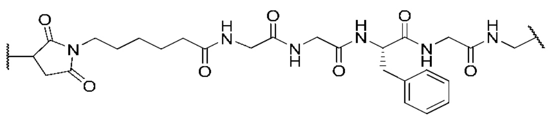

.

**15.** The bispecific antibody-drug conjugate according to any one of claims 1 to 14, wherein the bispecific antibody-drug conjugate has a structure represented by formula (A-2):

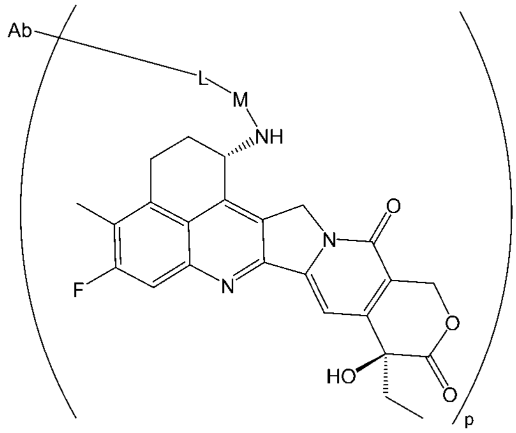

(A-2)

wherein p represents the average number of connections, and p is any integer or decimal from 1 to 10; preferably any integer or decimal from 2 to 8;
Ab is the bispecific antibody or the antigen-binding fragment thereof as defined in any one of claims 1 to 7;
L is the linker unit L as defined in claim 13 or 14.

**16.** The bispecific antibody-drug conjugate according to claim 15, wherein the bispecific antibody-drug conjugate has a structure represented by formula (A-2a) or (A-2b):

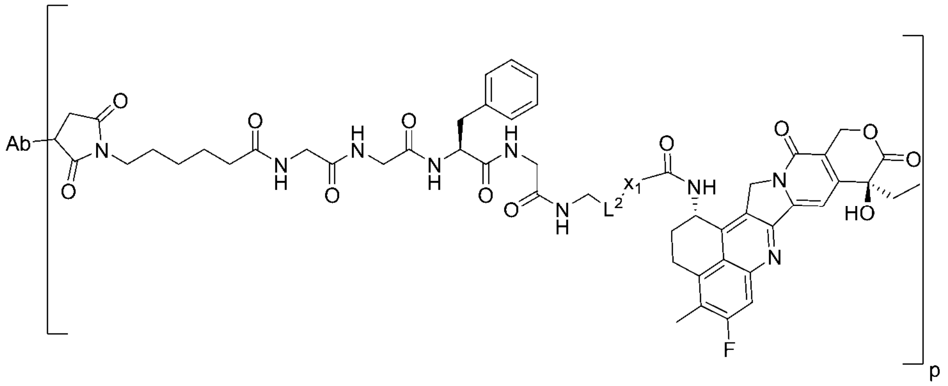

(A-2a)

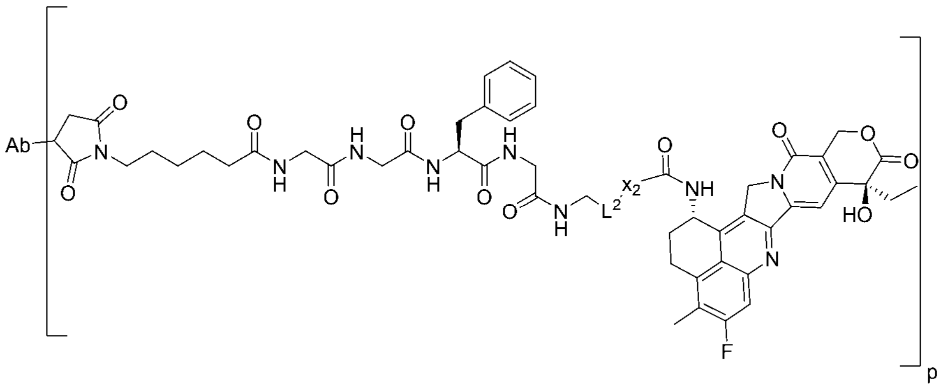

(A-2b),

wherein

p represents the average number of connections, and p is any integer or decimal from 1 to 10, preferably any integer or decimal from 2 to 8;
Ab is the bispecific antibody or the antigen-binding fragment thereof as defined in any one of claims 1 to 7;
$L^2$ is -O- or -S-, preferably -O-;

$X_1$ is $C_3$-$C_6$ saturated cycloalkyl optionally substituted by 1, 2, or 3 $R^{2a}$;
$X_2$ is $-(C(R^{1a})(R^{1b}))_m-CH_2-$;
m is 1 or 2;
$R^{1a}$, $R^{1b}$, and $R^{2a}$ are each independently hydrogen, halogen, or $C_1$-$C_6$ alkyl; the $C_1$-$C_6$ alkyl is optionally substituted by one or more halogens.

**17.** The bispecific antibody-drug conjugate according to any one of claims 1 to 16, wherein the bispecific antibody-drug conjugate is selected from the group consisting of any one of the following structures:

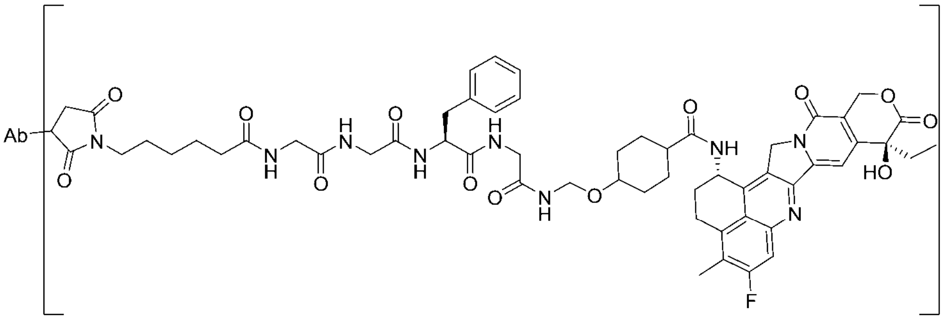
p,

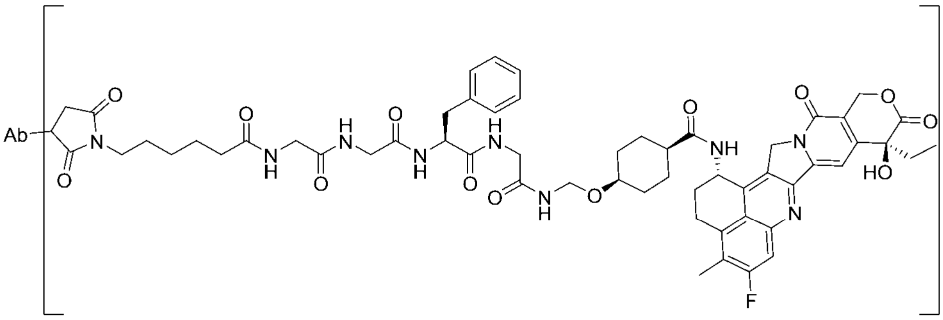
p,

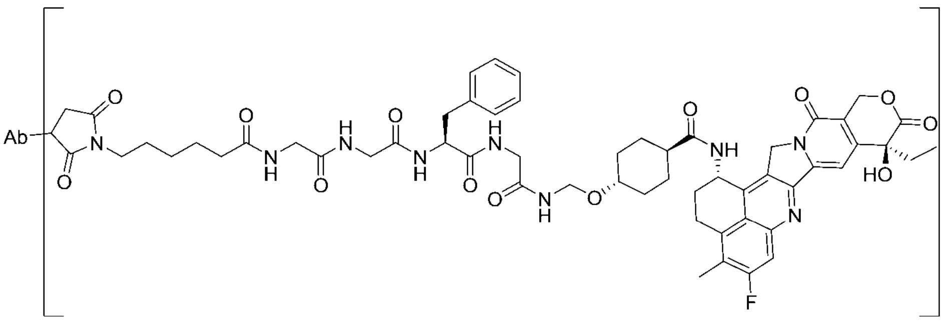
p,

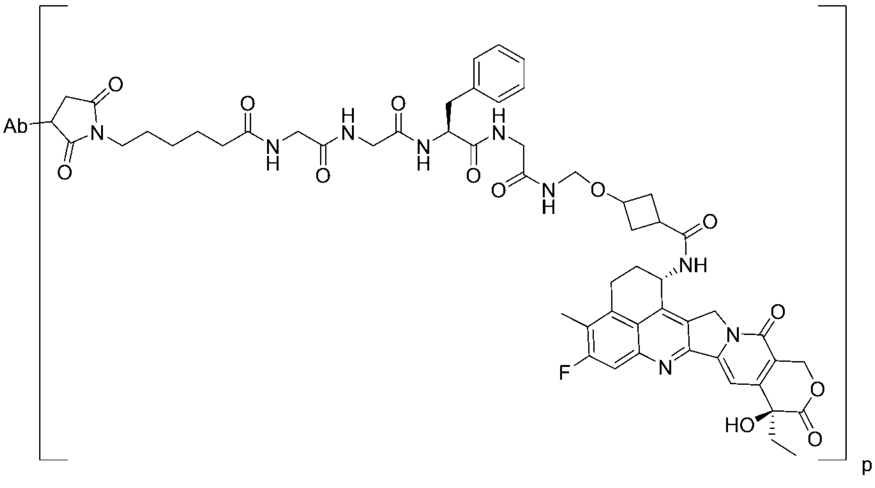

,

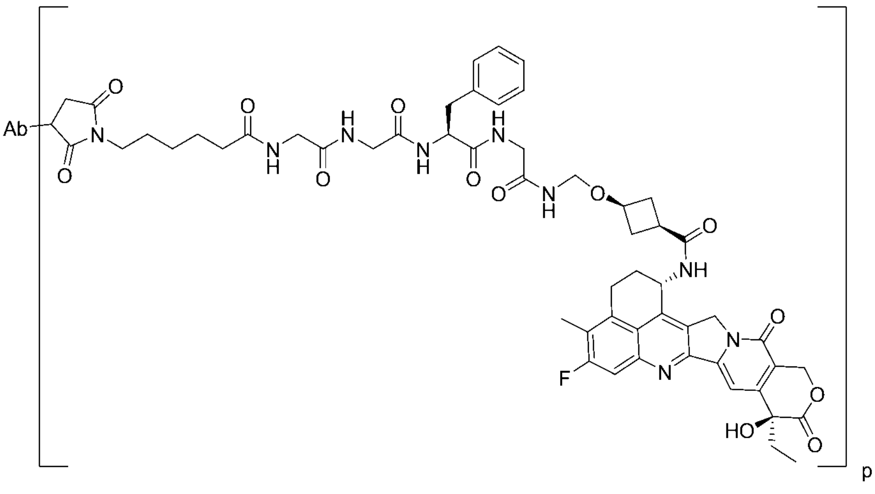

,

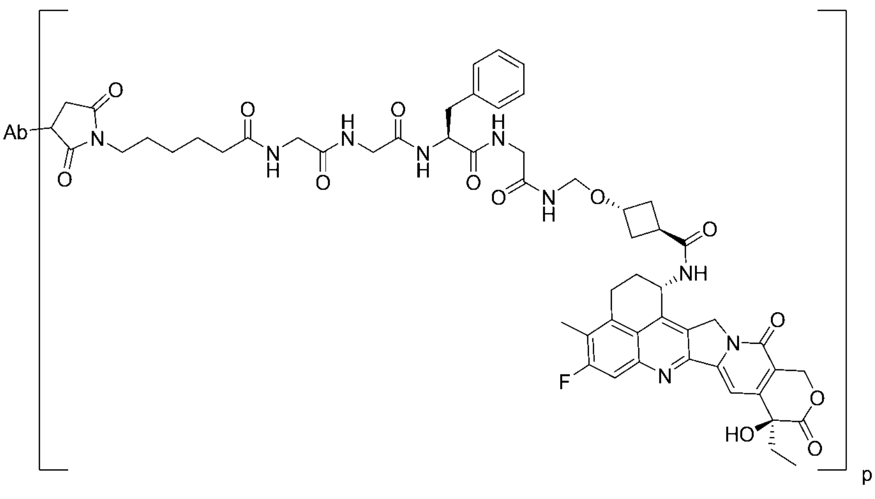

,

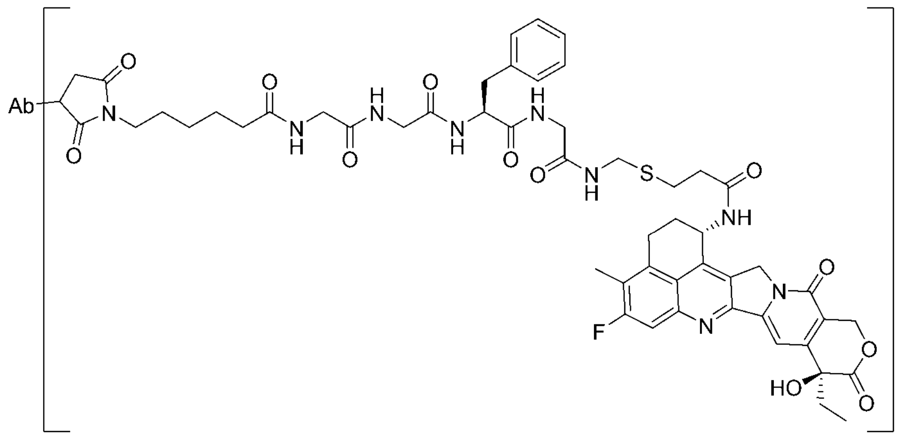

p ,

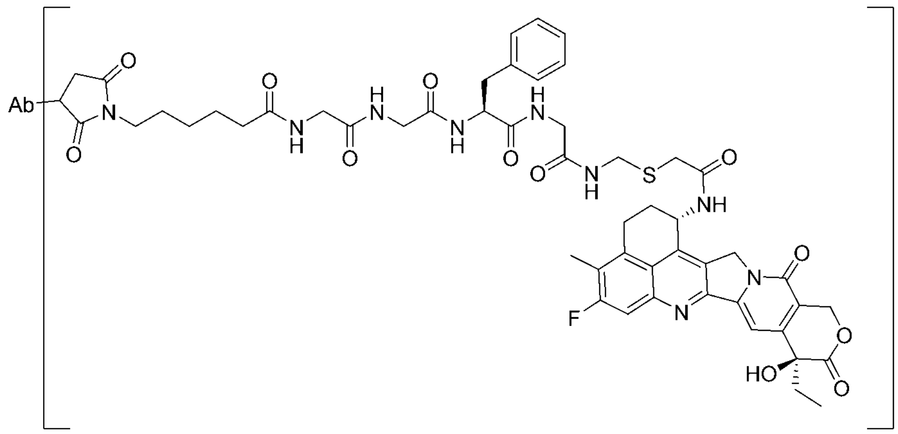

p ,

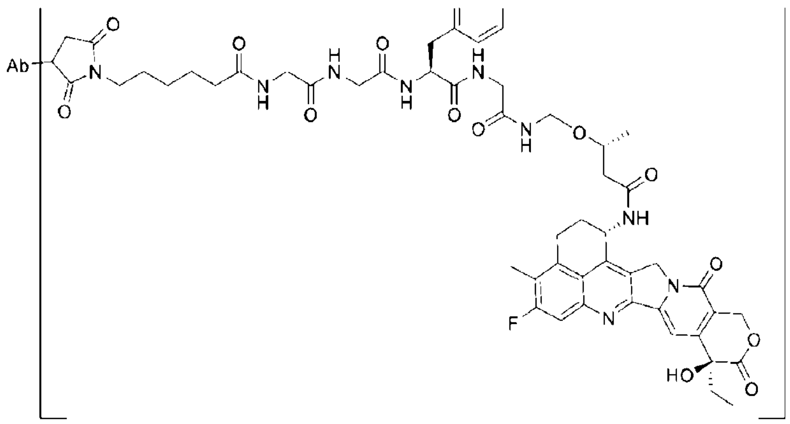

p ,

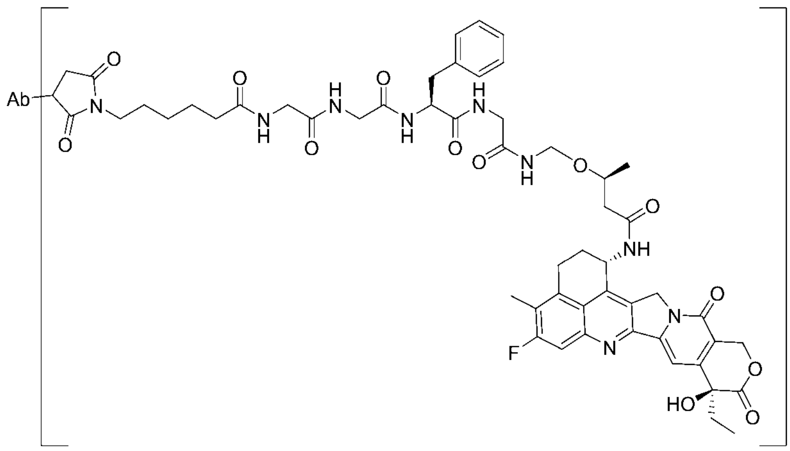

p,

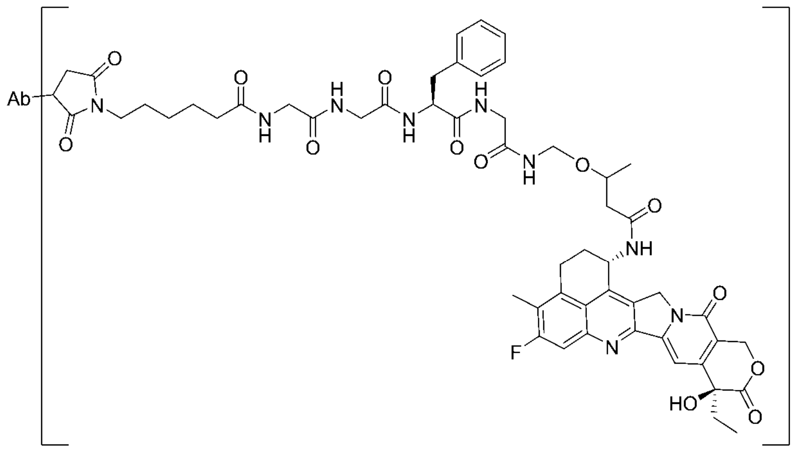

p,

and

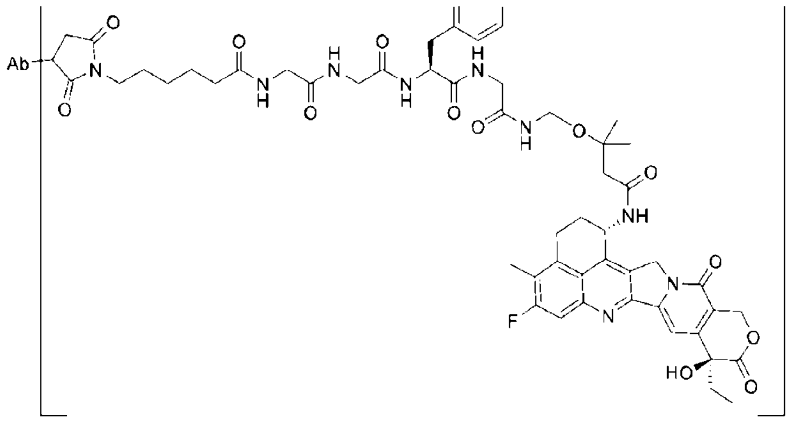

p;

wherein p represents the average number of connections, and p is any integer or decimal from 1 to 10, preferably any integer or decimal from 2 to 8, preferably any integer or decimal from 4 to 8, preferably any integer or decimal from 6 to 8;

Ab is the bispecific antibody or the antigen-binding fragment thereof as defined in any one of claims 1 to 7.

**18.** A bispecific antibody-drug conjugate, wherein the bispecific antibody-drug conjugate is selected from the group consisting of any one of the following structures:

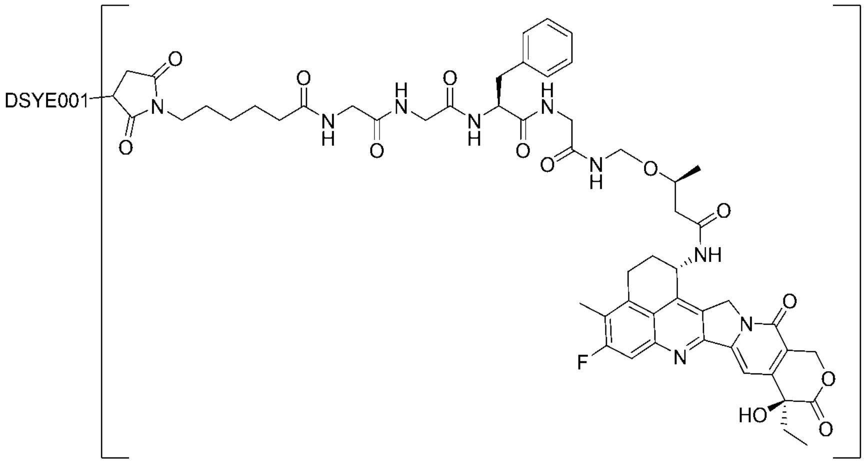

p ,

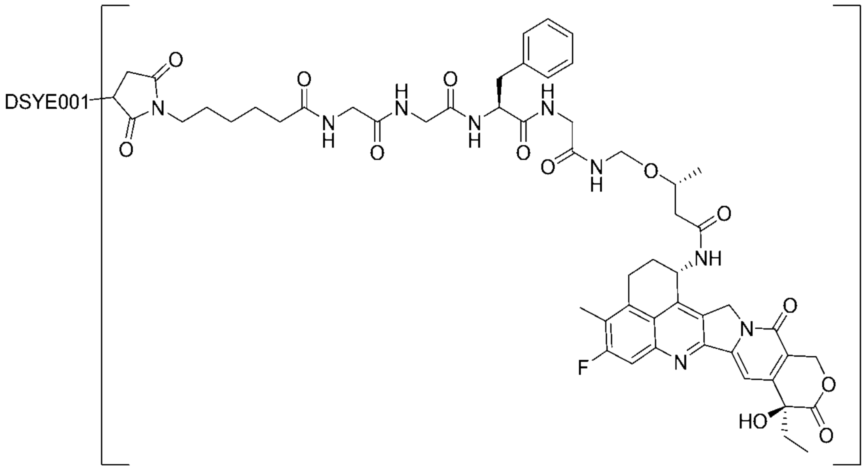

p ,

and

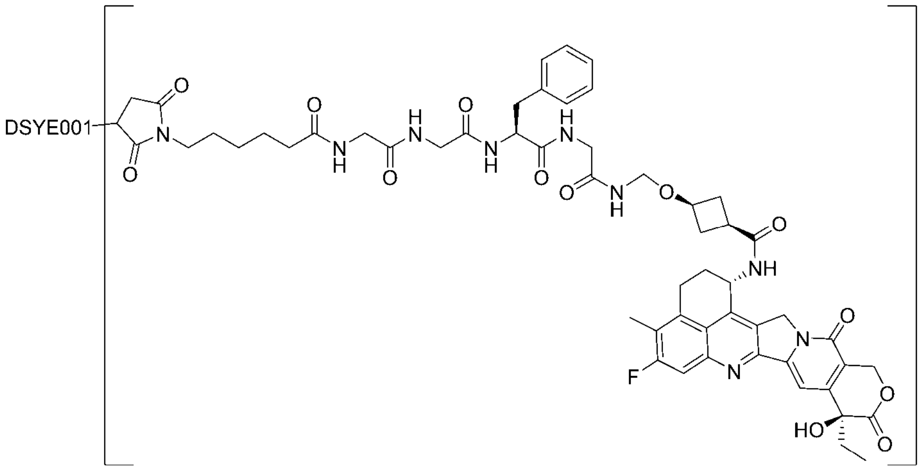

p ;

wherein p represents the average number of connections, and p is any integer or decimal from 1 to 10, preferably any integer or decimal from 3 to 8, preferably any integer or decimal from 4 to 8, preferably any integer or decimal from 6 to 8;

DSYE001 is an anti-B7H3 and PD-L1 bispecific antibody, wherein the heavy chain amino acid sequence of the bispecific antibody is as shown in SEQ ID NO.: 16, and the light chain amino acid sequence of the bispecific

antibody is as shown in SEQ ID NO.: 9.

**19.** A pharmaceutical composition comprising the bispecific antibody-drug conjugate as defined in any one of claims 1 to 18, and a pharmaceutically acceptable carrier or excipient.

**20.** Use of the bispecific antibody-drug conjugate as defined in any one of claims 1 to 19 or the pharmaceutical composition as defined in claim 19 in the manufacture of a medicament for treating and/or preventing cancer; preferably, the cancer is cancer with positive expression of B7H3 and/or PD-L1.

**21.** The use according to claim 20, wherein the cancer is selected from the group consisting of one or more of lung cancer, gastric cancer, liver cancer, colorectal cancer, melanoma, nephroma, ovarian cancer, prostate cancer, bladder cancer, breast cancer, esophageal cancer, carcinoma of large intestine, nasopharyngeal cancer, brain tumor, cervical cancer, blood cancer, bone cancer, lymphoma, pancreatic cancer, and Ewing's sarcoma; preferably, the cancer is lung cancer, prostate cancer, breast cancer, ovarian cancer, or melanoma.

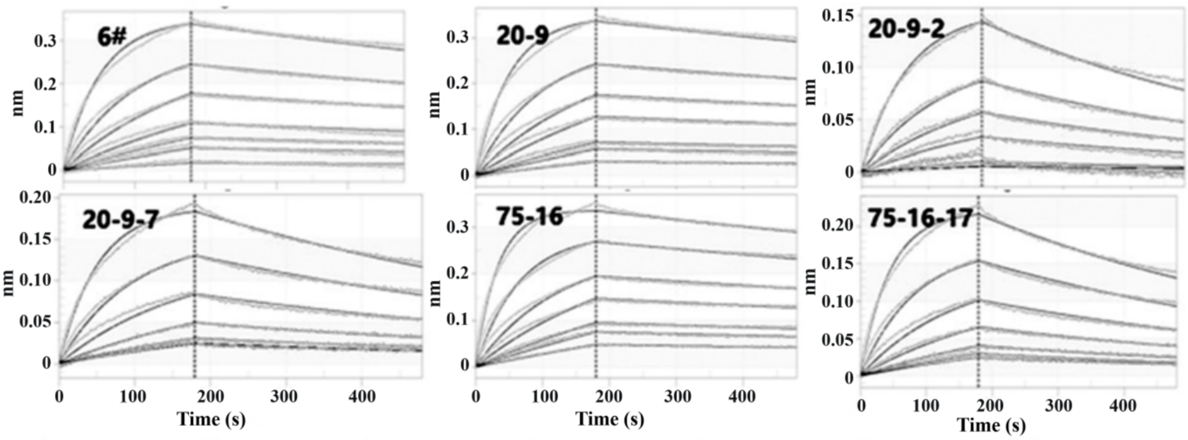


| | 6# | 20-9 | 20-9-2 | 20-9-72 | 75-16 | 75-16-17 |
|---|---|---|---|---|---|---|
| KD(M) | 6.98E-9 | 4.98E-9 | 3.16E-8 | 1.66E-8 | 3.24E-9 | 1.54E-8 |

FIG. 1

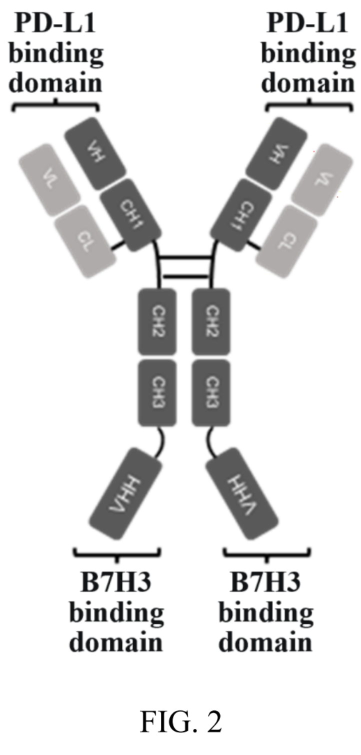


FIG. 2

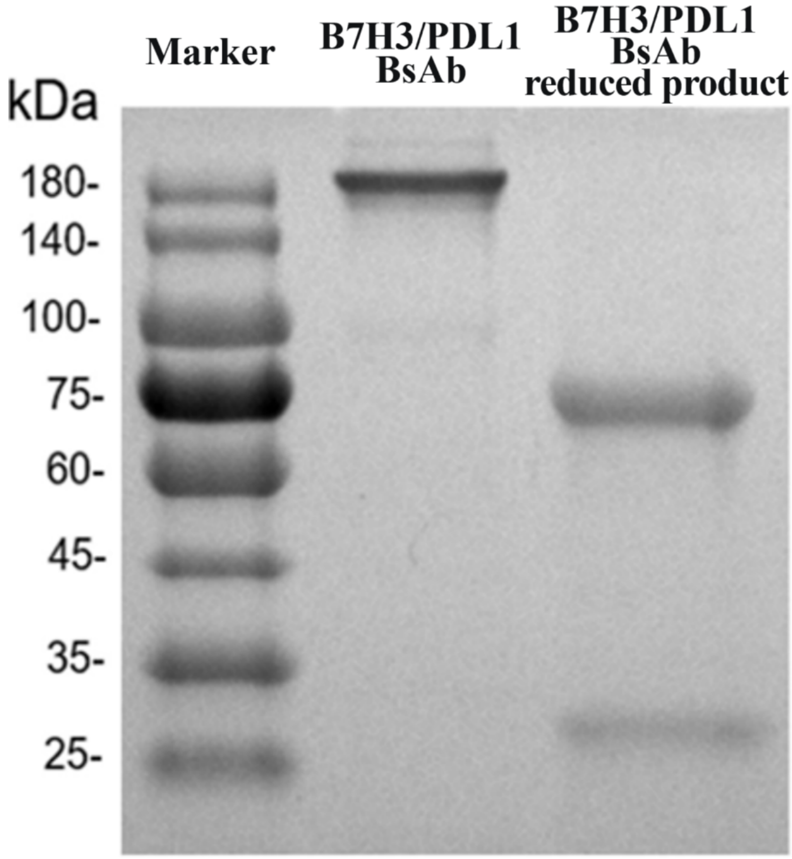
Marker
B7H3/PDL1 BsAb
B7H3/PDL1 BsAb reduced product
kDa
180-
140-
100-
75-
60-
45-
35-
25-

FIG. 3

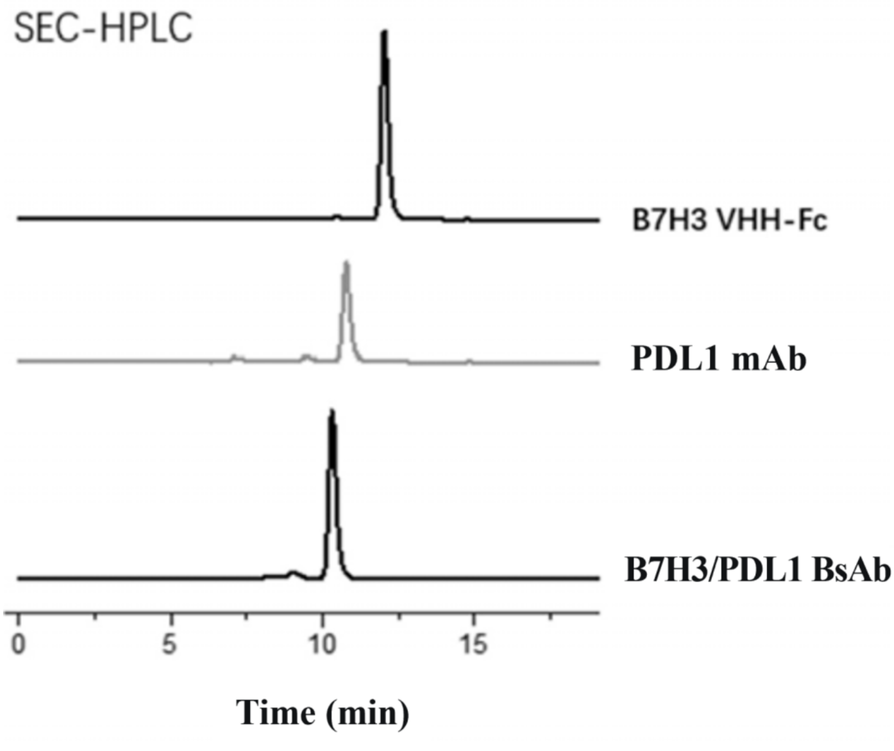
SEC-HPLC
B7H3 VHH-Fc
PDL1 mAb
B7H3/PDL1 BsAb
0
5
10
15
Time (min)

FIG. 4

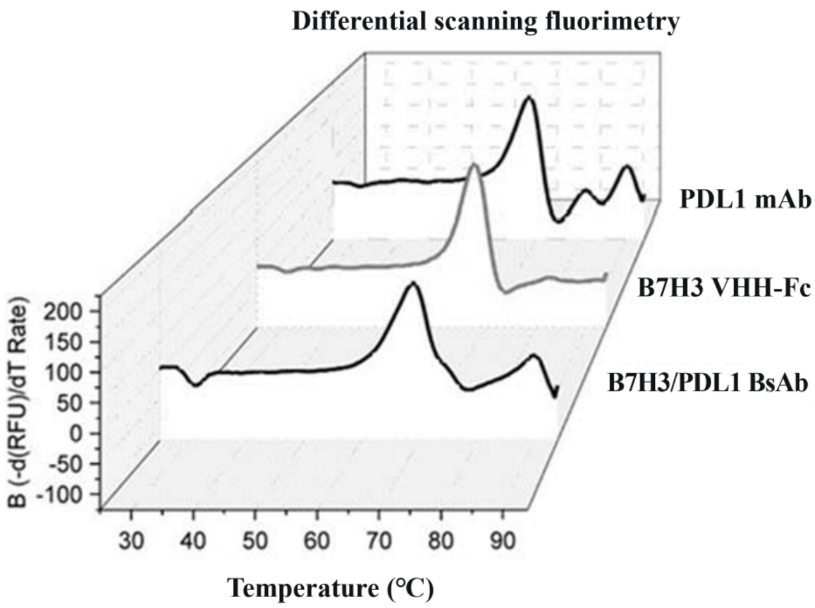


| | Tm (°C) | |
|---|---|---|
| | Fc | Fab |
| B7H3/PDL1 BsAb | 69 | 90 |
| B7H3 VHH-Fc | 68 | - |
| PDL1 mAb | 68.5 | 90 |

FIG. 5

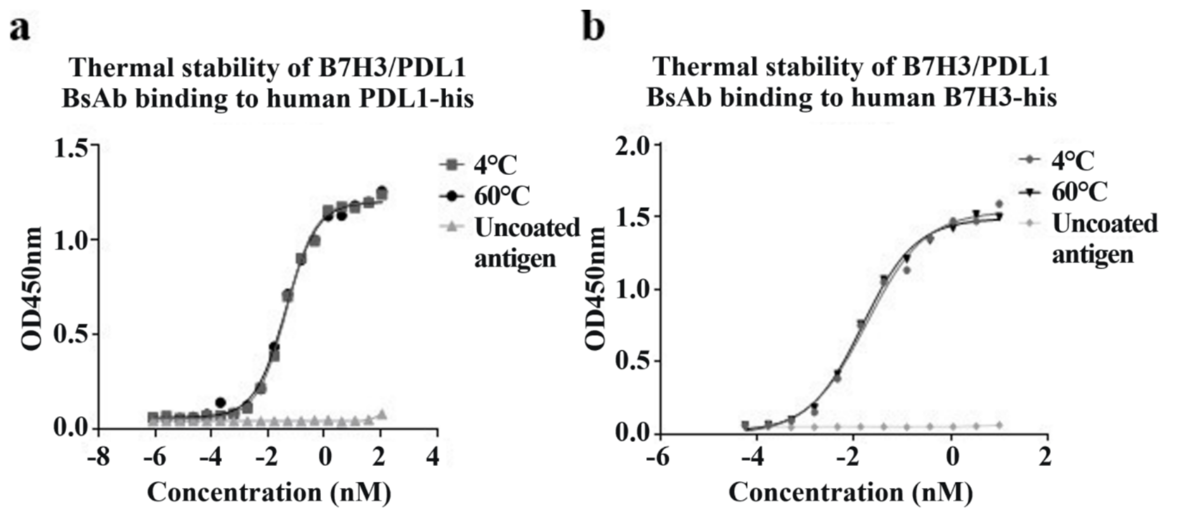


FIG. 6

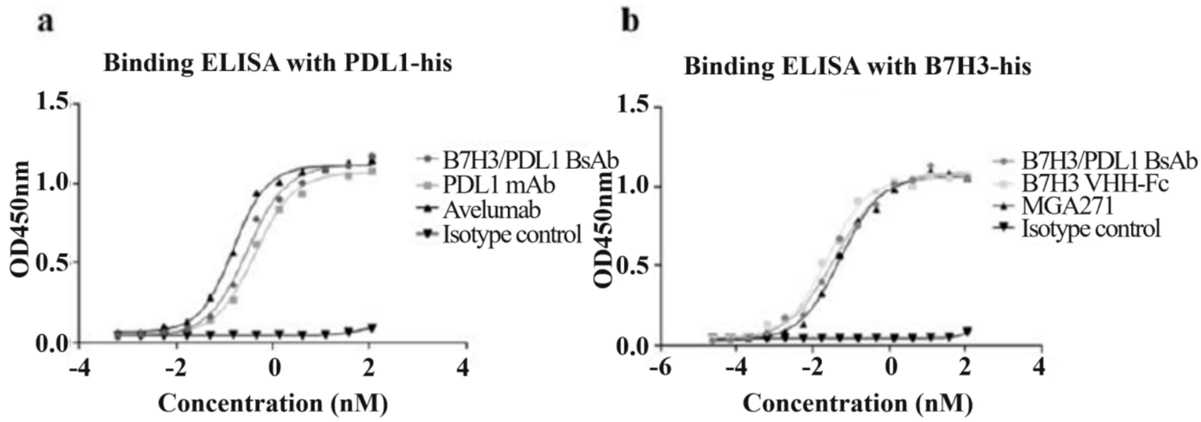


FIG. 7

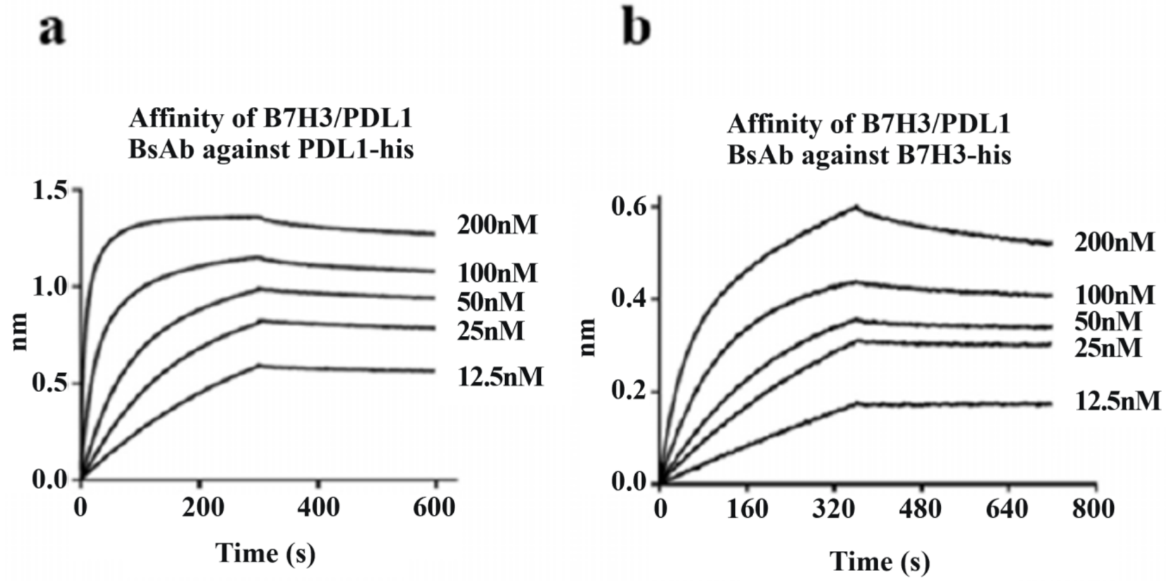


FIG. 8

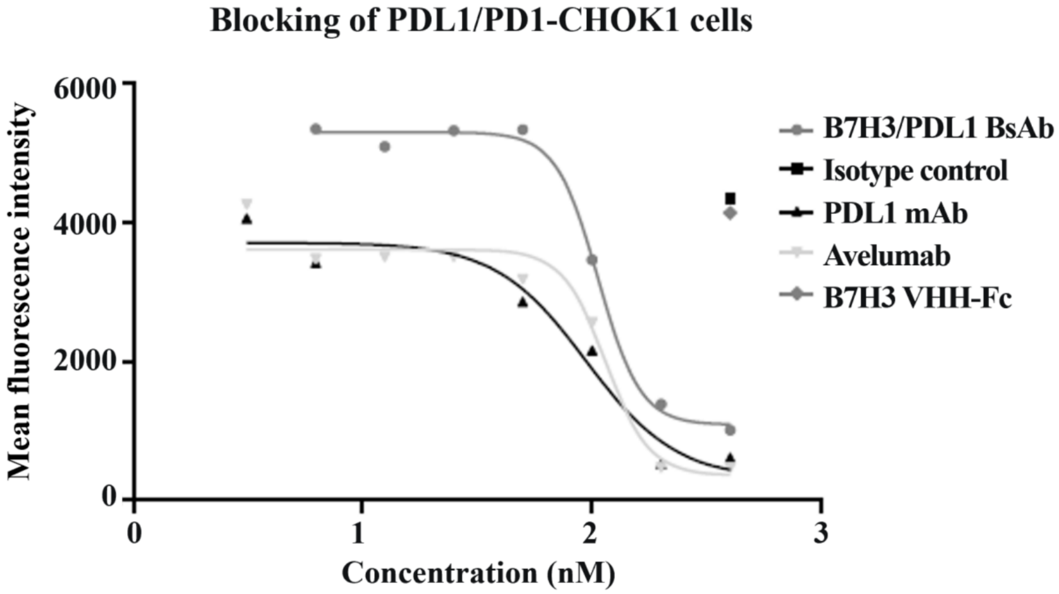
Blocking of PDL1/PD1-CHOK1 cells
Mean fluorescence intensity
6000
4000
2000
0
0
1
2
3
Concentration (nM)
B7H3/PDL1 BsAb
Isotype control
PDL1 mAb
Avelumab
B7H3 VHH-Fc

FIG. 9

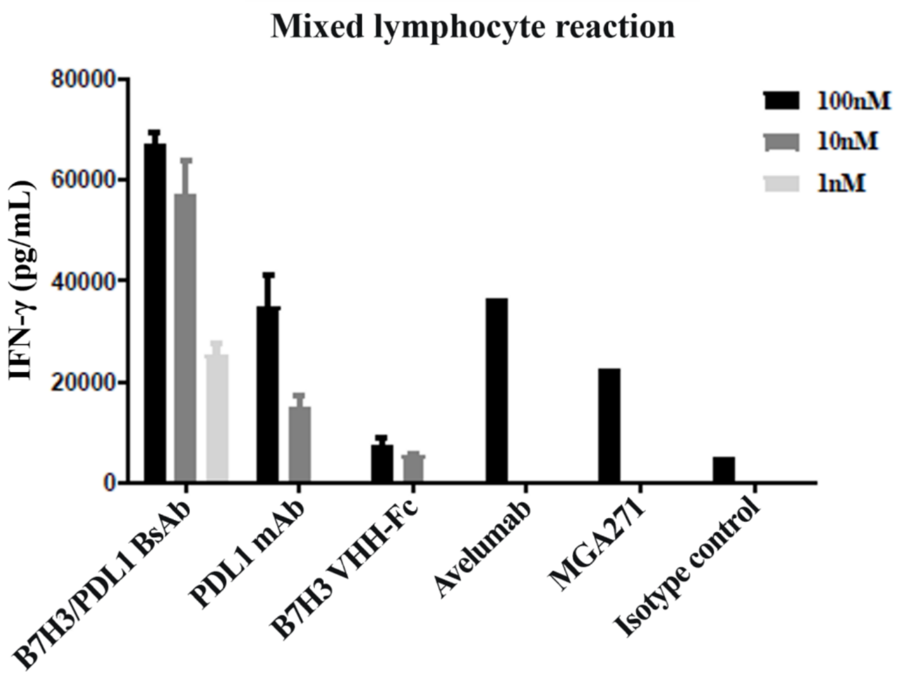
Mixed lymphocyte reaction
IFN-γ (pg/mL)
80000
60000
40000
20000
0
100nM
10nM
1nM
B7H3/PDL1 BsAb
PDL1 mAb
B7H3 VHH-Fc
Avelumab
MGA271
Isotype control

FIG. 10

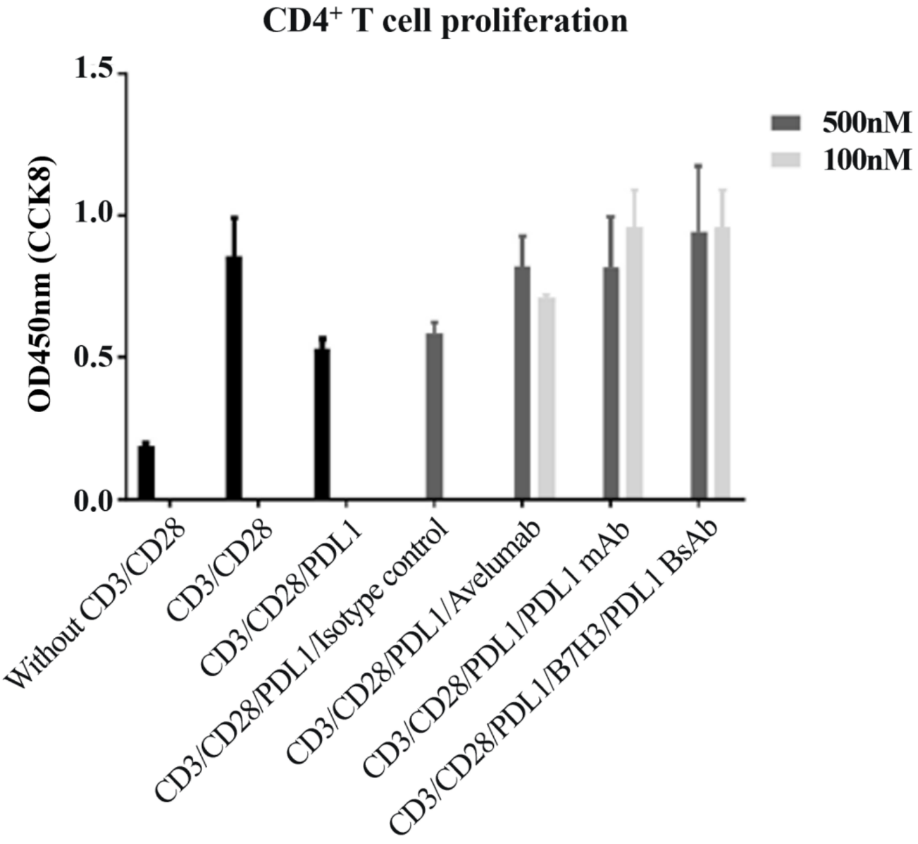
CD4+ T cell proliferation
OD450nm (CCK8)
1.5
1.0
0.5
0.0
500nM
100nM
Without CD3/CD28
CD3/CD28
CD3/CD28/PDL1
CD3/CD28/PDL1/Isotype control
CD3/CD28/PDL1/Avelumab
CD3/CD28/PDL1/PDL1 mAb
CD3/CD28/PDL1/B7H3/PDL1 BsAb

FIG. 11

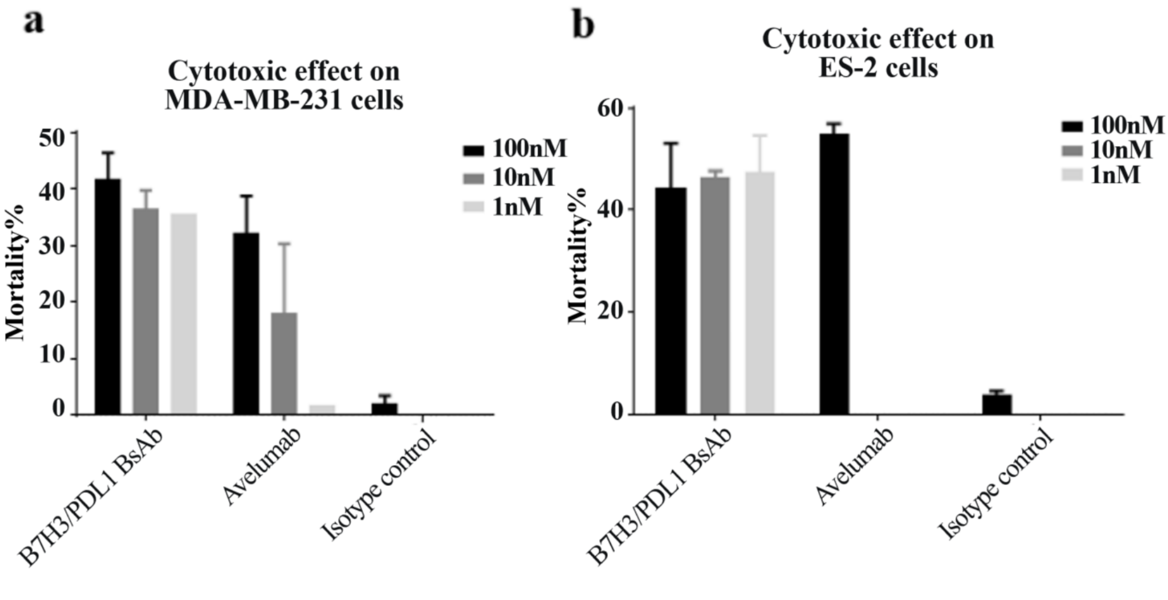
a
Cytotoxic effect on MDA-MB-231 cells
Mortality%
50
40
30
20
10
0
100nM
10nM
1nM
B7H3/PDL1 BsAb
Avelumab
Isotype control
b
Cytotoxic effect on ES-2 cells
Mortality%
60
40
20
0
100nM
10nM
1nM
B7H3/PDL1 BsAb
Avelumab
Isotype control

FIG. 12

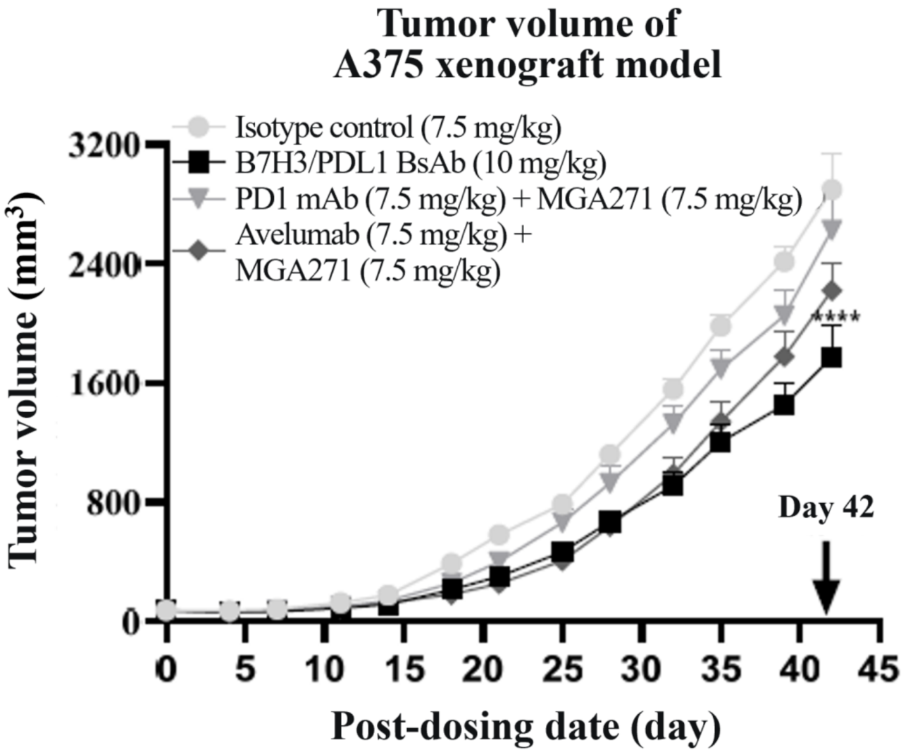
Tumor volume of
A375 xenograft model
Isotype control (7.5 mg/kg)
B7H3/PDL1 BsAb (10 mg/kg)
PD1 mAb (7.5 mg/kg) + MGA271 (7.5 mg/kg)
Avelumab (7.5 mg/kg) +
MGA271 (7.5 mg/kg)
Tumor volume (mm³)
3200
2400
1600
800
0
****
Day 42
0
5
10
15
20
25
30
35
40
45
Post-dosing date (day)

FIG. 13

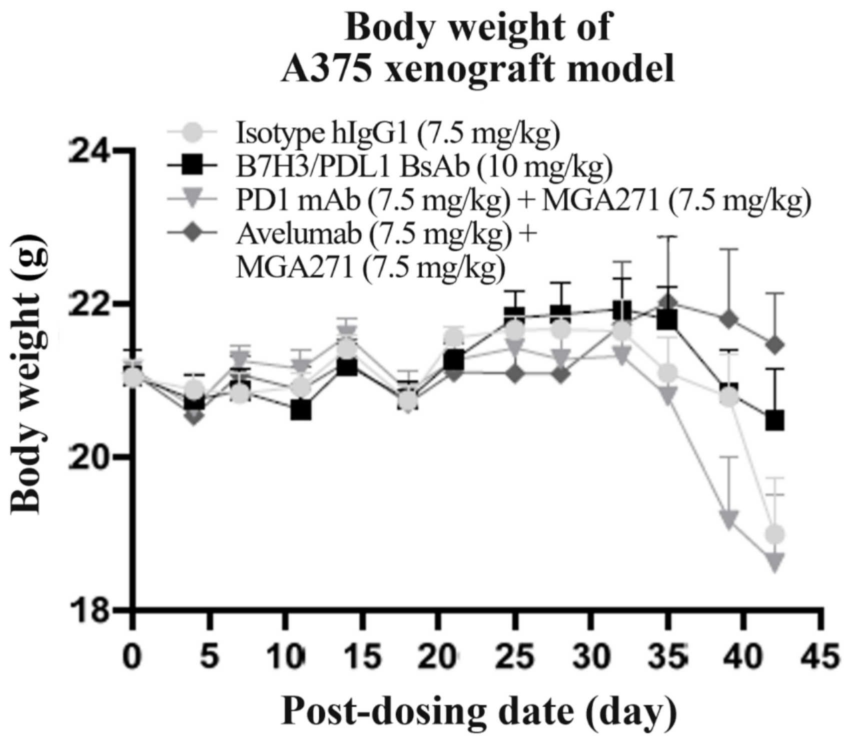
Body weight of
A375 xenograft model
Isotype hIgG1 (7.5 mg/kg)
B7H3/PDL1 BsAb (10 mg/kg)
PD1 mAb (7.5 mg/kg) + MGA271 (7.5 mg/kg)
Avelumab (7.5 mg/kg) +
MGA271 (7.5 mg/kg)
Body weight (g)
24
22
20
18
0
5
10
15
20
25
30
35
40
45
Post-dosing date (day)

FIG. 14

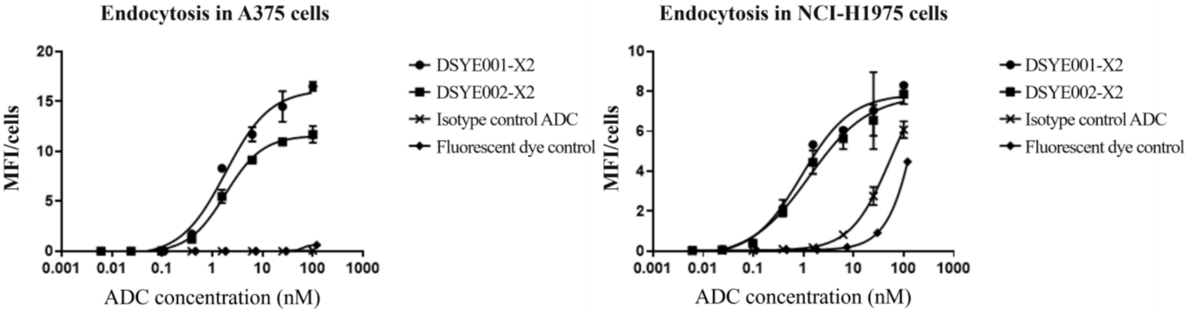


FIG. 15

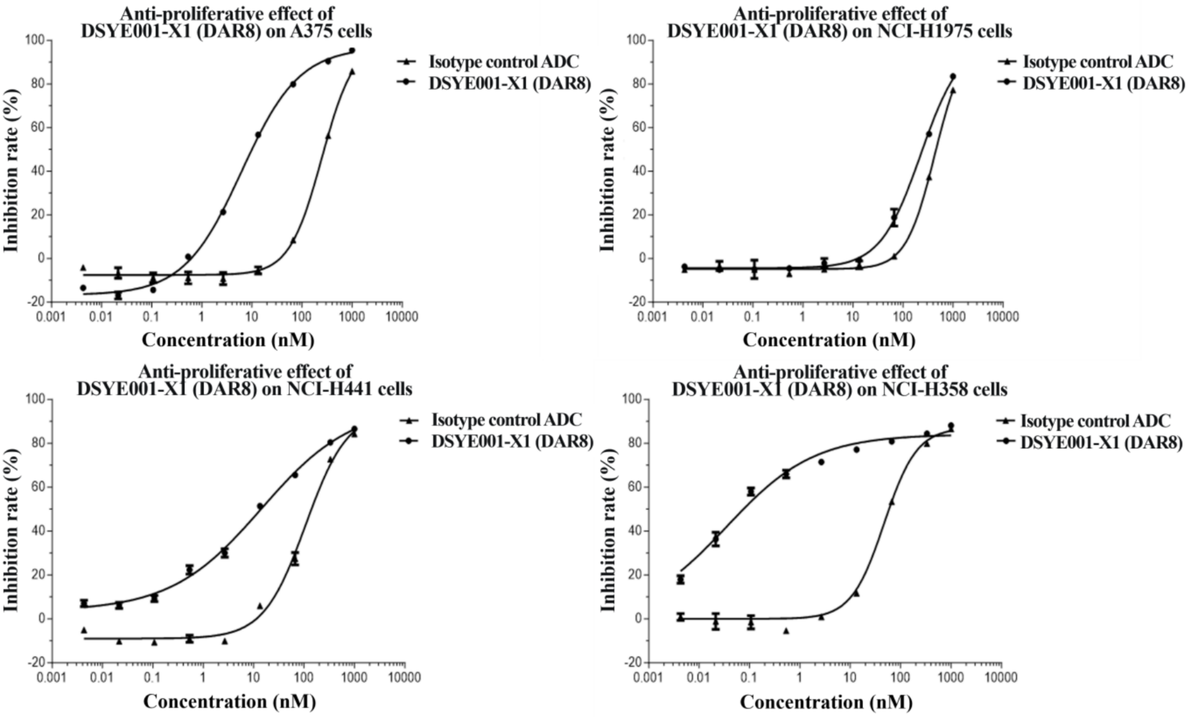


FIG. 16

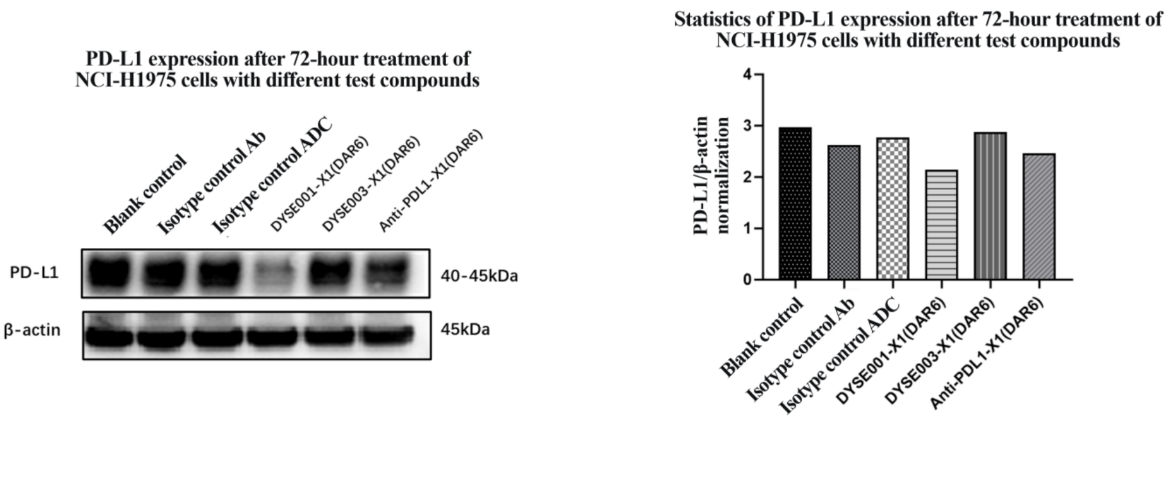


FIG. 17

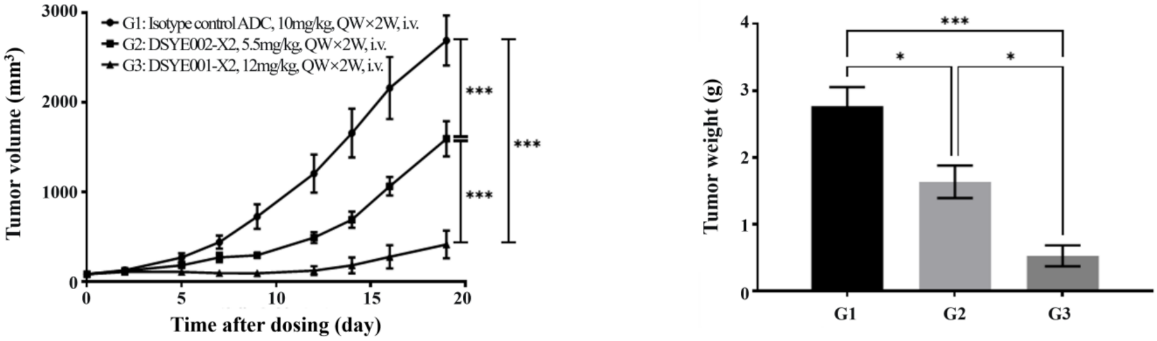


FIG. 18

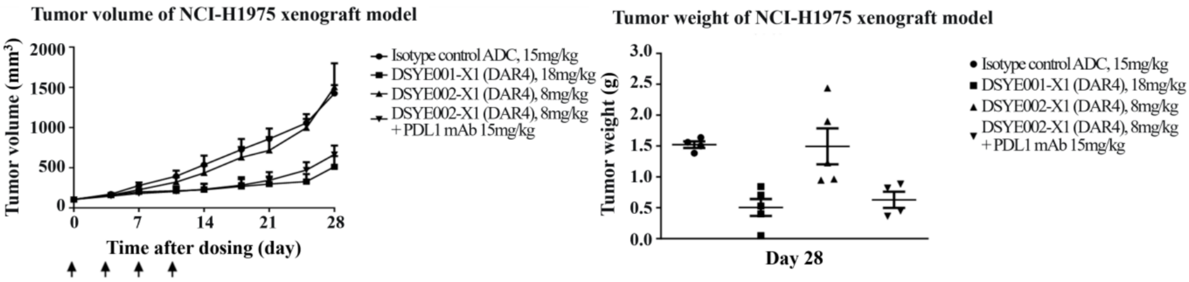


FIG. 19

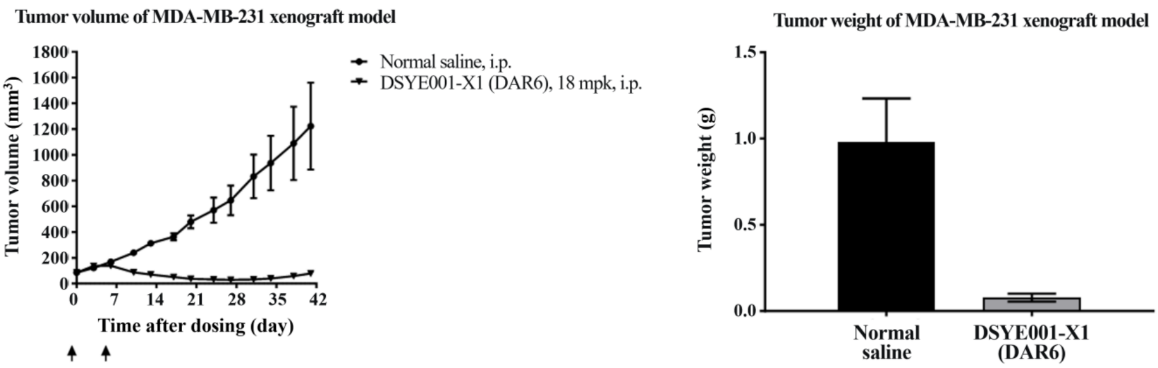


FIG. 20

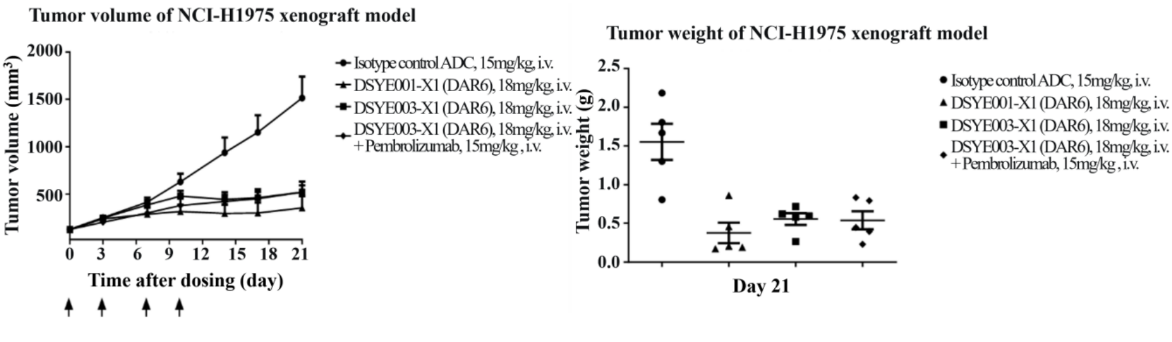


FIG. 21

## INTERNATIONAL SEARCH REPORT

International application No.
**PCT/CN2023/142481**

**A. CLASSIFICATION OF SUBJECT MATTER**

A61K47/68(2017.01)i; C07K16/46(2006.01)i; A61K31/4745(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, A61P, C07K, C12N

Documentation searched other than minimum documentation to the extent that such documents are included in the fields searched

Electronic data base consulted during the international search (name of data base and, where practicable, search terms used)

CJFD, CNTXT, DWPI, ENTXTC, WPABSC, CNABS, VEN, CNKI, PUBMED, STN, GenBank, 中国专利生物序列检索, Chinese Patent Biological Sequence Retrieval System, EBI-EMBL: 偶联, ADC, 双特异性抗体, B7H3, PDL1, 单克隆抗体, 纳米抗体, VHH, 抗肿瘤, 结构检索(权利要求17-18), structural search (claims 17-18), 序列检索(SEQ ID NO: 1-16), sequence search (SEQ ID NOs: 1-16), conjugat+, bispecific antibody, monoclonal antibody, nanobody, anticancer, antitumor

**C. DOCUMENTS CONSIDERED TO BE RELEVANT**

| Category* | Citation of document, with indication, where appropriate, of the relevant passages | Relevant to claim No. |
|---|---|---|
| A | CN 113943371 A (DARTSBIO PHARMACEUTICALS LTD.; SHANGHAI MAISHI BIOTECHNOLOGY CO., LTD.; SHENZHEN CHUANGSHI BIOPHARMACEUTICAL CO., LTD.) 18 January 2022 (2022-01-18) abstract, and claims 1-4 | 1-21 |
| A | CN 114195900 A (BIOTHEUS (ZHUHAI) CO., LTD.) 18 March 2022 (2022-03-18) abstract, and claims 1-8 | 1-21 |
| PA | CN 115894702 A (DARTSBIO PHARMACEUTICALS LTD.; SHANGHAI MAISHI BIOTECHNOLOGY CO., LTD.) 04 April 2023 (2023-04-04) abstract, and claims 1-4 | 1-21 |
| A | WO 2022245186 A1 (LEGOCHEM BIOSCIENCES, INC.; ABL BIO , INC.) 24 November 2022 (2022-11-24) abstract, and claims 1 and 79-81 | 1-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 March 2024** | **24 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/142481**

**C. DOCUMENTS CONSIDERED TO BE RELEVANT**

| Category* | Citation of document, with indication, where appropriate, of the relevant passages | Relevant to claim No. |
|---|---|---|
| A | CN 110642948 A (DASHI PHARMACEUTICAL (GUANGDONG) CO., LTD.; SHANGHAI MAISHI BIOTECHNOLOGY CO., LTD.; SHENZHEN CHUANGSHI BIOLOGY MEDICINE CO., LTD.) 03 January 2020 (2020-01-03)<br>abstract, and claims 1-16 | 1-21 |
| A | WO 2022068878 A1 (DUALITY BIOLOGICS (SUZHOU) CO., LTD.) 07 April 2022 (2022-04-07)<br>abstract, claims 1-103, and description, preparation examples 1 and 38-55 | 1-21 |

Form PCT/ISA/210 (second sheet) (July 2022)

**INTERNATIONAL SEARCH REPORT**

International application No.
**PCT/CN2023/142481**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/142481**

| Patent document cited in search report | | | Publication date (day/month/year) | Patent family member(s) | | | Publication date (day/month/year) |
|---|---|---|---|---|---|---|---|
| CN | 113943371 | A | 18 January 2022 | CN | 113943371 | B | 05 May 2023 |
| CN | 114195900 | A | 18 March 2022 | CN | 114195900 | B | 23 February 2024 |
| CN | 115894702 | A | 04 April 2023 | | None | | |
| WO | 2022245186 | A1 | 24 November 2022 | KR | 20220158181 | A | 30 November 2022 |
| | | | | TW | 202313122 | A | 01 April 2023 |
| CN | 110642948 | A | 03 January 2020 | WO | 2021068871 | A1 | 15 April 2021 |
| | | | | EP | 4043491 | A1 | 17 August 2022 |
| | | | | EP | 4043491 | A4 | 07 December 2022 |
| | | | | JP | 2022551318 | A | 08 December 2022 |
| | | | | CN | 110642948 | B | 29 June 2021 |
| WO | 2022068878 | A1 | 07 April 2022 | US | 2023086097 | A1 | 23 March 2023 |
| | | | | US | 11685742 | B2 | 27 June 2023 |
| | | | | KR | 20230079085 | A | 05 June 2023 |
| | | | | IL | 301793 | A | 01 May 2023 |
| | | | | AU | 2021354823 | A1 | 30 March 2023 |
| | | | | US | 11607459 | B1 | 21 March 2023 |
| | | | | US | 2023331738 | A1 | 19 October 2023 |
| | | | | EP | 4223318 | A1 | 09 August 2023 |
| | | | | JP | 2023551355 | A | 08 December 2023 |
| | | | | CA | 3195515 | A1 | 07 April 2022 |
| | | | | TW | 202214230 | A | 16 April 2022 |
| | | | | US | 2023212182 | A1 | 06 July 2023 |
| | | | | CN | 115925796 | A | 07 April 2023 |
| | | | | CN | 116199739 | A | 02 June 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022116919701
- CN 2023116964970
- CN 2022125089 W


### Non-patent literature cited in the description


- *Remington's Pharmaceutical Sciences*, 2005
- **WARD et al.** Binding Activities of a Repertoire of Single Immunoglobulin Variable Domains Secreted From Escherichia coli''. *Nature*, 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA*, 1988, vol. 85, 5879-5883
- **NGUYEN V.K. et al.** *The EMBO Journal*, 2000, vol. 19, 921-930
- **MUYLDERMANS S.** *J Biotechnol.*, 2001, vol. 74, 277-302
- **VANLANDSCHOOT P. et al.** *Antiviral Research*, 2011, vol. 92, 389-407
- **KABAT et al.** Sequences of Proteins of Immunological Interest. National Institutes of Health, 1991
- **A1-LAZIKANI et al.** *JMol Biol*, 1997, vol. 273, 927-48
- **MATHIEU DONDELINGER et al.** Understanding the Significance and Implications of Antibody Numbering and Antigen-Binding Surface/Residue Definition. *Front. Immunol.*, 16 October 2018
- **RABAT et al.** Sequences of Proteins of Immunological Interest. Public Health Service, National Institutes of Health, 1991